# EUROPEAN PATENT SPECIFICATION

(11) **EP 4 022 047 B1**
(45) Date of publication and mention of the grant of the patent: **22.04.2026**
(21) Application number: 20774885.6
(22) Date of filing: 28.08.2020
(51) Int. Cl.: C12N 9/02, C12N 15/62, A23L 5/20, A23K 10/14, C12N 9/04

(54) **IMPROVED POLYPEPTIDES CAPABLE OF CONVERTING SUBSTRATE 3-KETO- DEOXYNIVALENOL INTO 3-EPI- DEOXYNIVALENOL**
VERBESSERTE, ZUR UMWANDLUNG VON 3-KETO-DEOXYNIVALENOL IN 3-EPI-DEOXYNIVALENOL FÄHIGE POLYPEPTIDE
POLYPEPTIDES AMÉLIORÉS CAPABLES DE CONVERTIR 3-KETO-DÉSOXYNIVALÉNOL 3-EPI-DÉSOXYNIVALÉNOL DANS UN SUBSTRAT

(30) Priority: 30.08.2019 EP 19194632
(43) Date of publication of application: 06.07.2022
(73) Proprietor: DSM Austria GmbH, 3131 Getzersdorf bei Traismauer (AT)
(72) Inventor: NEUMAYER, Bernhard, 3131 Getzersdorf (AT); STREIT, Elisabeth, 3131 Getzersdorf (AT); WEBER, Barbara, 3131 Getzersdorf (AT); VOGTENTANZ, Gudrun, 3131 Getzersdorf (AT)
(74) Representative: Schiweck Weinzierl Koch Patentanwälte Partnerschaft mbB
(86) International application number: PCT/EP2020/074056
(87) International publication number: WO 2021/038026

(56) References cited:
- WO-A1-2019/046954
- "SubName: Full=Aldo/keto reductase {ECO:0000313|EMBL:KQV08982.1}", UNIPROT,, 20 January 2016 (2016-01-20), XP002797459
- "SubName: Full=Aldo/keto reductase {ECO:0000313|EMBL:ODS85017.1}", UNIPROT,, 18 January 2017 (2017-01-18), XP002797458
- "SubName: Full=Aldo/keto reductase {ECO:0000313|EMBL:KKB08280.1}", UNIPROT,, 24 June 2015 (2015-06-24), XP002797455
- "SubName: Full=Aldo/keto reductase {ECO:0000313|EMBL:ODT51284.1}", UNIPROT,, 18 January 2017 (2017-01-18), XP002797454
- "SubName: Full=Aldo/keto reductase {ECO:0000313|EMBL:KKB11079.1}", UNIPROT,, 24 June 2015 (2015-06-24), XP002797457
- "SubName: Full=Aldo/keto reductase {ECO:0000313|EMBL:KFL24809.1}", UNIPROT,, 29 October 2014 (2014-10-29), XP002797456
- "SubName: Full=Aldo/keto reductase {ECO:0000313|EMBL:KKC35898.1}", UNIPROT,, 24 June 2015 (2015-06-24), XP002797451
- "SubName: Full=Putative oxidoreductase, aldo/keto reductase family {ECO:0000313|EMBL:KFC71784.1}; EC=1.1.1.- {ECO:0000313|EMBL:KFC71784.1}", UNIPROT,, 29 October 2014 (2014-10-29), XP002797450
- "SubName: Full=Aryl-alcohol dehydrogenase-like predicted oxidoreductase {ECO:0000313|EMBL:PTR35064.1}", UNIPROT,, 18 July 2018 (2018-07-18), XP002797461
- "SubName: Full=Aldo/keto reductase {ECO:0000313|EMBL:ODT68868.1}", UNIPROT,, 18 January 2017 (2017-01-18), XP002797453
- "SubName: Full=Aldo/keto reductase {ECO:0000313|EMBL:KKB84356.1}; SubName: Full=Predicted oxidoreductase {ECO:0000313|EMBL:SHF63179.1}", UNIPROT,, 24 June 2015 (2015-06-24), XP002797452
- "SubName: Full=Aldo/keto reductase {ECO:0000313|EMBL:APG03273.1}", UNIPROT,, 16 September 2015 (2015-09-16), XP002797460
- DATABASE UniProt [online] 29 October 2014 (2014-10-29), "SubName: Full=Putative oxidoreductase, aldo/keto reductase family {ECO:0000313|EMBL:KFC71784.1}; EC=1.1.1.- {ECO:0000313|EMBL:KFC71784.1};", XP002797450, retrieved from EBI accession no. UNIPROT:A0A085FJY9 Database accession no. A0A085FJY9
- DATABASE UniProt [online] 24 June 2015 (2015-06-24), "SubName: Full=Aldo/keto reductase {ECO:0000313|EMBL:KKC35898.1};", XP002797451, retrieved from EBI accession no. UNIPROT:A0A0F5Q4N8 Database accession no. A0A0F5Q4N8
- DATABASE UniProt [online] 24 June 2015 (2015-06-24), "SubName: Full=Aldo/keto reductase {ECO:0000313|EMBL:KKB84356.1}; SubName: Full=Predicted oxidoreductase {ECO:0000313|EMBL:SHF63179.1};", XP002797452, retrieved from EBI accession no. UNIPROT:A0A0F5LPU3 Database accession no. A0A0F5LPU3
- DATABASE UniProt [online] 18 January 2017 (2017-01-18), "SubName: Full=Aldo/keto reductase {ECO:0000313|EMBL:ODT68868.1};", XP002797453, retrieved from EBI accession no. UNIPROT:A0A1E4FC77 Database accession no. A0A1E4FC77
- DATABASE UniProt [online] 18 January 2017 (2017-01-18), "SubName: Full=Aldo/keto reductase {ECO:0000313|EMBL:ODT51284.1};", XP002797454, retrieved from EBI accession no. UNIPROT:A0A1E4E4C2 Database accession no. A0A1E4E4C2
- DATABASE UniProt [online] 24 June 2015 (2015-06-24), "SubName: Full=Aldo/keto reductase {ECO:0000313|EMBL:KKB08280.1};", XP002797455, retrieved from EBI accession no. UNIPROT:A0A0F5FI82 Database accession no. A0A0F5FI82
- DATABASE UniProt [online] 29 October 2014 (2014-10-29), "SubName: Full=Aldo/keto reductase {ECO:0000313|EMBL:KFL24809.1};", XP002797456, retrieved from EBI accession no. UNIPROT:A0A087LJK6 Database accession no. A0A087LJK6
- DATABASE UniProt [online] 24 June 2015 (2015-06-24), "SubName: Full=Aldo/keto reductase {ECO:0000313|EMBL:KKB11079.1};", XP002797457, retrieved from EBI accession no. UNIPROT:A0A0F5FQD0 Database accession no. A0A0F5FQD0
- DATABASE UniProt [online] 18 January 2017 (2017-01-18), "SubName: Full=Aldo/keto reductase {ECO:0000313|EMBL:ODS85017.1};", XP002797458, retrieved from EBI accession no. UNIPROT:A0A1E4ABE6 Database accession no. A0A1E4ABE6
- DATABASE UniProt [online] 20 January 2016 (2016-01-20), "SubName: Full=Aldo/keto reductase {ECO:0000313|EMBL:KQV08982.1};", XP002797459, retrieved from EBI accession no. UNIPROT:A0A0Q6Q8V6 Database accession no. A0A0Q6Q8V6
- DATABASE UniProt [online] 16 September 2015 (2015-09-16), "SubName: Full=Aldo/keto reductase {ECO:0000313|EMBL:APG03273.1};", XP002797460, retrieved from EBI accession no. UNIPROT:A0A0G9HEV9 Database accession no. A0A0G9HEV9
- DATABASE UniProt [online] 18 July 2018 (2018-07-18), "SubName: Full=Aryl-alcohol dehydrogenase-like predicted oxidoreductase {ECO:0000313|EMBL:PTR35064.1};", XP002797461, retrieved from EBI accession no. UNIPROT:A0A2T5LJ77 Database accession no. A0A2T5LJ77
- YOUSEF I. HASSAN ET AL: "The enzymatic epimerization of deoxynivalenol by Devosia mutans proceeds through the formation of 3-keto-DON intermediate", SCIENTIFIC REPORTS, vol. 7, no. 1, 31 July 2017 (2017-07-31), pages 1 - 11, XP055581678, DOI: 10.1038/s41598-017-07319-0

## Description

### TECHNICAL FIELD OF THE INVENTION

This application contains a Sequence Listing in computer readable form, which is incorporated herein by reference.

The present invention relates to compositions comprising one or more polypeptide(s) comprising or consisting of a sequence of SEQ ID NO. 1-14 or a sequence having a sequence identity of at least 75 % to any one of SEQ ID NO. 1-14 and a carrier as well as uses and methods of these polypeptides.

### DESCRIPTION

The invention is set out in the appended set of claims. Mycotoxins are secondary metabolites produced by filamentous fungi. One representative of mycotoxins is deoxynivalenol (DON), is a type-B trichotecene or vomitoxin, which is produced by a variety of *Fusarium* fungi and can be found throughout the world. These fungi infest cultivated plants, among others, such as various types of grain, wherein the fungal infestation usually occurs before the harvest when the growth of the fungi and/or the mycotoxin production may take place before storage or may even take place after harvest, either prior to storage or under improper storage conditions. The Food and Agriculture Organization of the United Nations (FAO) has estimated that 25 % of agricultural products throughout the world are contaminated with mycotoxins, thus resulting in substantial economic losses. In an international study spanning 8 years, a total of 19,757 samples was analyzed from January 2004 to December 2011; 72 % of them testing positive for at least one mycotoxin, 39 % were found to be co-contaminated, and 56 % testing positive for DON (Schatzmayr and Streit (2013)). Trichothecenes and thus also DON have been found in all regions of the world and in all types of grain and feed crops tested, such as corn, soy flour, wheat, wheat bran, DDGS (dried distillers grains with solubles) as well as in finished animal feed mixtures with an incidence of up to 100 %.

The primary strategy for reducing trichothecene contamination of foods and animal feed products is to restrict the growth of fungi, for example by maintaining "good agricultural practice". This includes, among other things, ensuring that the seed is free of pests and fungal infestation or that agricultural waste products are removed from the field promptly. In addition, fungal growth in the field can be reduced by the use of fungicides. After the harvest, the harvested material should be stored at a residual moisture level of less than 15 % and at a low temperature to prevent the growth of fungi. Likewise, material contaminated by fungal infestation should be removed before further processing. Despite this long list of preventive measures, even in regions with the highest agricultural standards such as North America and Central Europe, up to 68 % of the tested samples were found contaminated with DON as a representative of trichothecenes in the years 2004 to 2011 (Schatzmayr and Streit (2013)).

It is known that toxicity of trichothecenes is (at least) partly due to hydroxyl (-OH) group present at the C-3 atom. One of the most abundant trichothecenes having such a 3-hydroxy group is deoxynivalenol (DON).

Yet, the hydroxyl group of DON and other trichothecenes can be present in two isomeric states, namely in S conformation (DON) or the R conformation (3-epi-DON). Several publications He et al. (2015), Payros et al. (2016) and Pierron et al. (2016).

Hassan et al. (2017), claims that the epimerization of DON to 3-epi-DON proceeds via a two-step process through the formation of 3-keto DON. 3-keto DON comprises a 3-oxo group instead of a 3-hydroxy group as present in the isomers. Finally, Carere et al. (2018) identified an enzyme, namely DmDepB from D. *mutans* 17-2-E-8 that performs the reduction from 3-keto-DON into 3-epi-DON and DON. WO2019/046954 describes this very same enzyme as Hassan et al. (2017) and a further DepB enzyme obtained from *Rhizobium leguminosarum* (RIDepB).

In this context, some sequences are known in the art (UniProt: A0A085FJY9; A0A0F5Q4N8; A0A0F5LPU3; A0A1E4FC77; A0A1E4E4C2; A0A0F5FI82; A0A087LJK6; A0A0F5FQD0; A0A1E4ABE6; A0A0Q6Q8V6; A0A0G9HEV9; A0A2T5LJ77; WO2019/046954 A1). However, there is still a need to provide enzymes which have a better efficiency in converting 3-keto-DON into the non-toxic isomer 3-epi-DON or to convert trichothecenes comprising a 3-oxo group into trichothecenes comprising a 3-hydroxy group in S configuration.

The solution of the present invention is described in the following, exemplified in the examples, illustrated in the Figures and reflected in the claims.

The present invention relates to an agrarian composition comprising
a) one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1; and
b) a carrier.

The present invention also relates to an agrarian composition comprising
a) one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON, and
b) a carrier.

The present invention further relates to a polypeptide comprising a sequence of SEQ ID NO. 1 or a sequence having a sequence identity of at least 92 % to SEQ ID NO. 1, wherein the polypeptide is capable of converting the 3-keto-DON into 3-epi-DON. The present disclosure also relates to a polypeptide comprising a sequence of any one of SEQ ID NO. 63-101.

In addition, the present invention relates to a use of one or more polypeptide(s) comprising or consisting of a sequence of SEQ ID NO. 1-14 or a sequence having a sequence identity of at least 75 % to any one of SEQ ID NO. 1-14 for converting 3-keto-DON into 3-epi-DON.

Also the present invention relates to a use of one or more polypeptide(s) comprising or consisting of a sequence of SEQ ID NO. 1-14 or a sequence having a sequence identity of at least 75 % to any one of SEQ ID NO. 1-14 , wherein the polypeptide is capable of converting the substrate 3-keto-DON into the products 3-epi-DON and DON with a 3-epi-DON:DON ratio of at least 25:1, in the manufacture of a feed additive or feed composition.

Further, the present invention relates to a use of one or more polypeptide(s) comprising or consisting of a sequence of SEQ ID NO. 1-14 or a sequence having a sequence identity of at least 75 % to any one of SEQ ID NO. 1-14 , wherein the polypeptide is capable of converting the substrate 3-keto-DON into the product 3-epi-DON and optionally additionally into the product DON amounting to 100 % of total product, wherein at least 96 % of the total product is 3-epi-DON in the manufacture of a feed additive or feed composition.

The present invention concerns a use of one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a ratio of at least 25:1 (3-epi-DON:DON), in the manufacture of biogas, bioethanol, or sugar, preferably from sugar cane or sugar beets.

Further, the present invention relates to a use of one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON in the manufacture of biogas, bioethanol, or sugar, preferably from sugar cane or sugar beets.

Also encompassed by the present invention are feed additives or food additives as well as feed or food comprising one or more polypeptide(s) comprising or consisting of a sequence of SEQ ID NO. 1-14 or a sequence having a sequence identity of at least 75 % to any one of SEQ ID NO. 1-14 , wherein the polypeptide is capable of converting the substrate 3-keto-DON into the products 3-epi-DON and DON with a 3-epi-DON:DON ratio of at least 25:1.

Further, the present invention concerns feed additives or food additives or feed or food comprising one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON.

Also encompassed by the present invention is an additive for use in agrarian compositions, comprising 3-keto-DON or 3-DON, the additive comprising one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1.

The present invention also relates to an additive for use in agrarian compositions, comprising 3-keto-DON or DON, the additive comprising one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON.

Further, the present invention relates to a method of converting a trichothecene comprising a 3-oxo group into a trichothecene comprising a 3-hydroxy group, the method comprising contacting one or more polypeptide(s) comprising or consisting of a sequence of SEQ ID NO. 1-14 or a sequence having a sequence identity of at least 75 % to any one of SEQ ID NO. 1-14 with a trichothecene comprising a 3-oxo group.

In addition, the present invention relates to a method of converting 3-keto-DON into 3-epi-DON, the method comprising contacting one or more polypeptide(s) comprising or consisting of a sequence of SEQ ID NO. 1-14 or a sequence having a sequence identity of at least 75 % to any one of SEQ ID NO. 1-14 with 3-keto-DON.

Further, the present invention relates to a method of reducing the content of DON in a composition comprising DON or of reducing the toxicity of a composition comprising DON by converting DON into 3-epi-DON, the method comprising
a) contacting the composition with an enzyme capable of converting DON into 3-keto DON; and
b) subsequently or concurrently contacting the composition with one or more polypeptide(s) comprising or consisting of a sequence of SEQ ID NO. 1-14 or a sequence having a sequence identity of at least 75 % to any one of SEQ ID NO. 1-14.

Further, the present invention relates to a host cell comprising one or more polypeptide(s) comprising or consisting of a sequence of SEQ ID NO. 1-14 or a sequence having a sequence identity of at least 75 % to any one of SEQ ID NO. 1-14 wherein the polypeptide is capable of converting the substrate 3-keto-DON into the products 3-epi-DON and DON with a 3-epi-DON: DON ratio of at least 25:1, wherein said recombinant host cell is transferred with an expression vector capable of producing said one or more polypeptide(s).

Also disclosed is a plant genetically modified to express one or more polypeptide(s) comprising or consisting of a sequence of SEQ ID NO. 1-14 or a sequence having a sequence identity of at least 75 % to any one of SEQ ID NO. 1-14 and/or a sequence of SEQ ID NO. 63-101.

The present disclosure concerns a seed of an inventive plant.

The present invention further concerns a polypeptide comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a ratio of at least 25:1 (3-epi-DON/DON) for use in the prevention and/or treatment of mycotoxicosis.

In addition the present invention relates to a polypeptide comprising or consisting of a sequence of SEQ ID NO. 1-14 or a sequence having a sequence identity of at least 75 % to any one of SEQ ID NO. 1-14, the polypeptide is capable of converting the substrate 3-keto-DON into the product 3-epi-DON and optionally additionally into the product DON amounting to 100 % of total product, wherein at least 96 % of the total product is 3-epi-DON for use in the prevention and/or treatment of mycotoxicosis.

The Figures show:

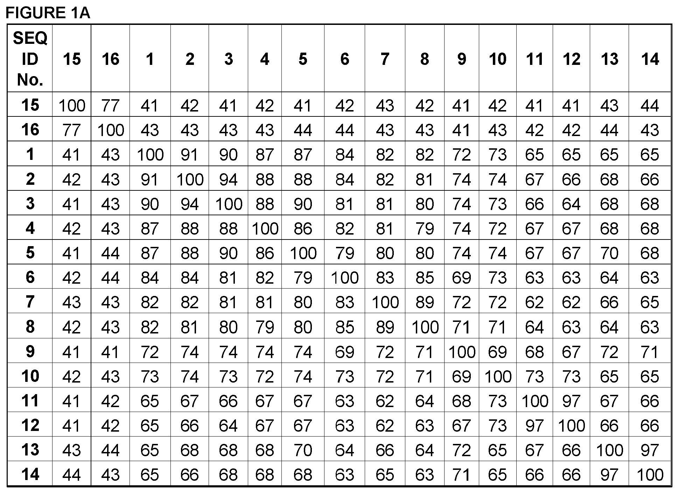

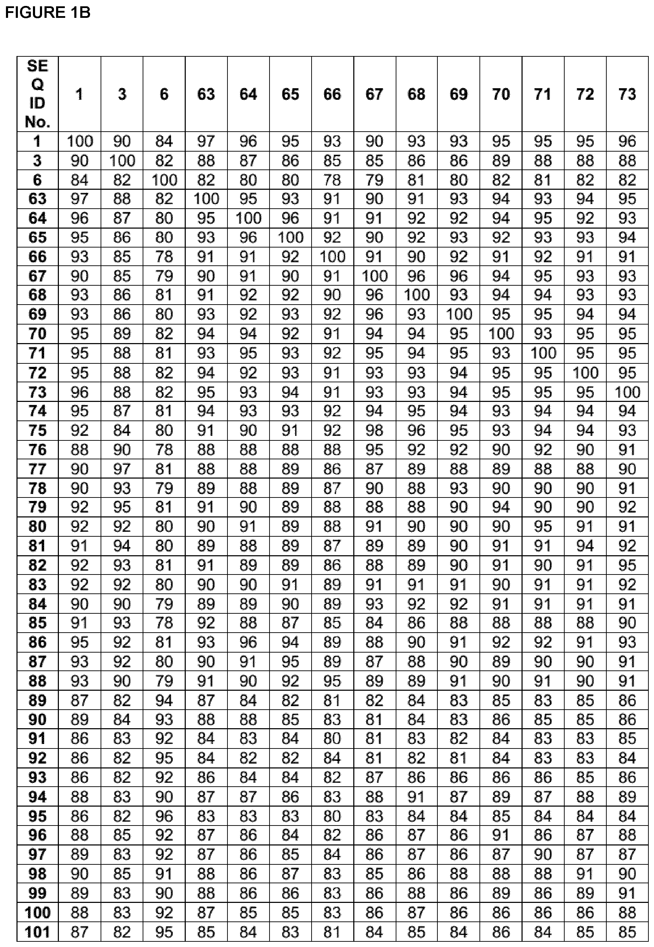

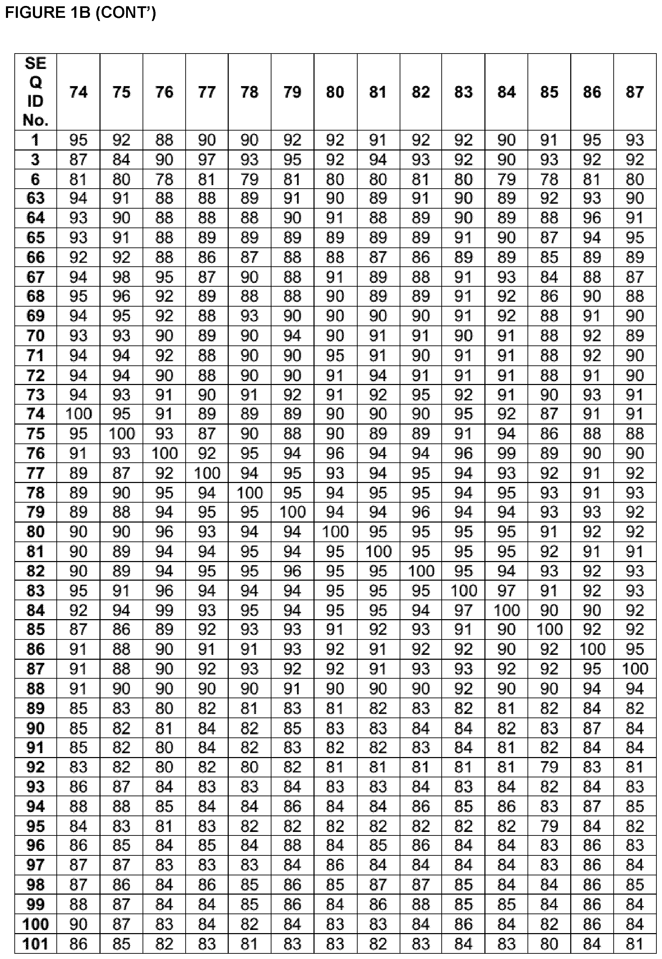

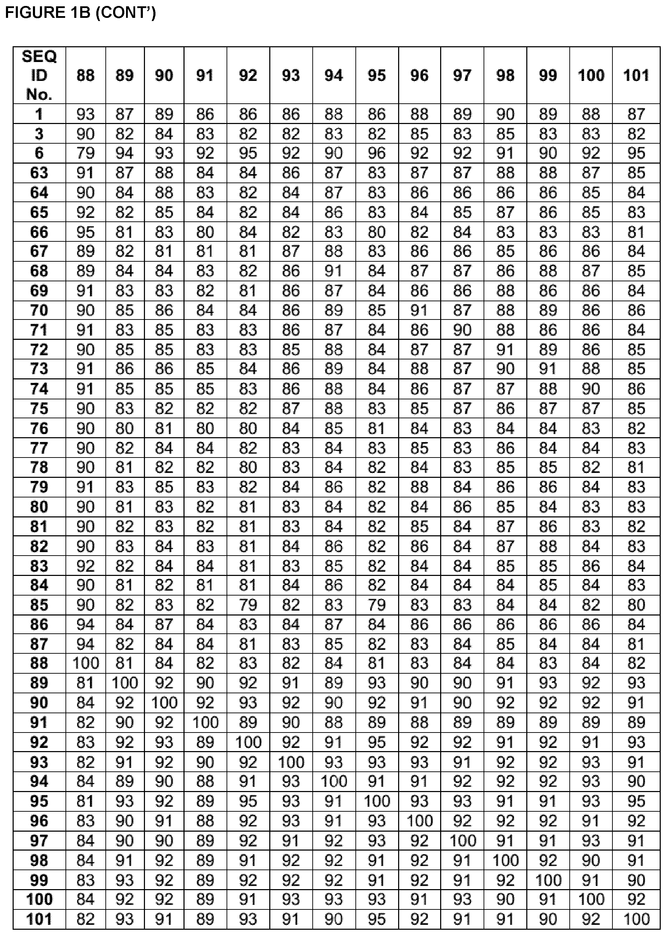

Fig. 1A shows sequence identity over the entire protein length depicted as percent identity (100% coverage of the protein sequence) of SEQ ID NO. 1-16. SEQ ID NO. 1-14 are sequences of 3-keto DON reductases described herein, while SEQ ID NO. 15 and SEQ ID NO. 16 relate to prior art DepB enzyme sequences. Fig. 1B shows sequence identities over the entire protein length depicted as percent identity (100% coverage of the protein sequence) of SEQ ID NO. 1, 3, 6, 63-101.

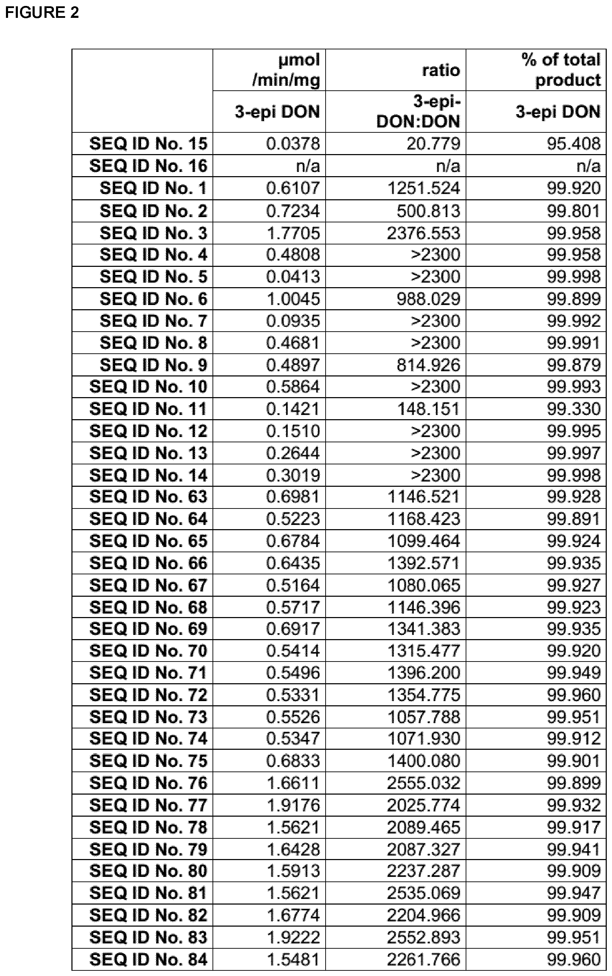

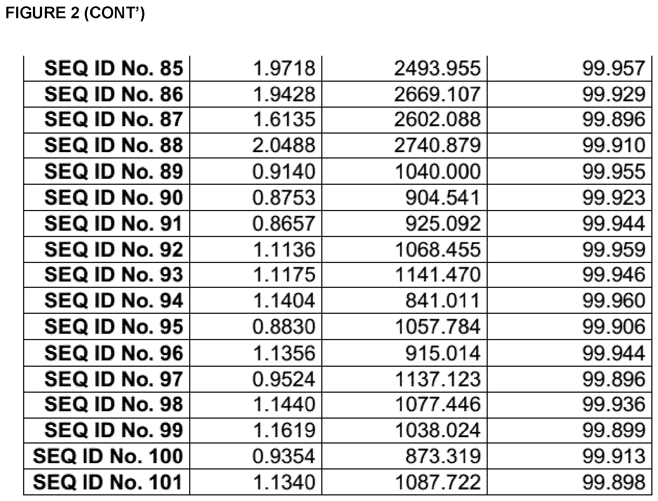

Fig. 2 shows data for different enzymes with regard to the yield of 3-epi-DON. Notably, no data for SEQ ID NO. 16 has been shown, because SEQ ID NO. 16 could not be produced in sufficient amounts to perform the required analytical assays. However, it is - without wishing to be bound to theory - be expected that SEQ ID NO. 16 will perform similar to SEQ ID NO. 15. The reason for this is that SEQ ID NO. 15 and 16 share highly similar sequences especially in the regions of loop A and loop B.

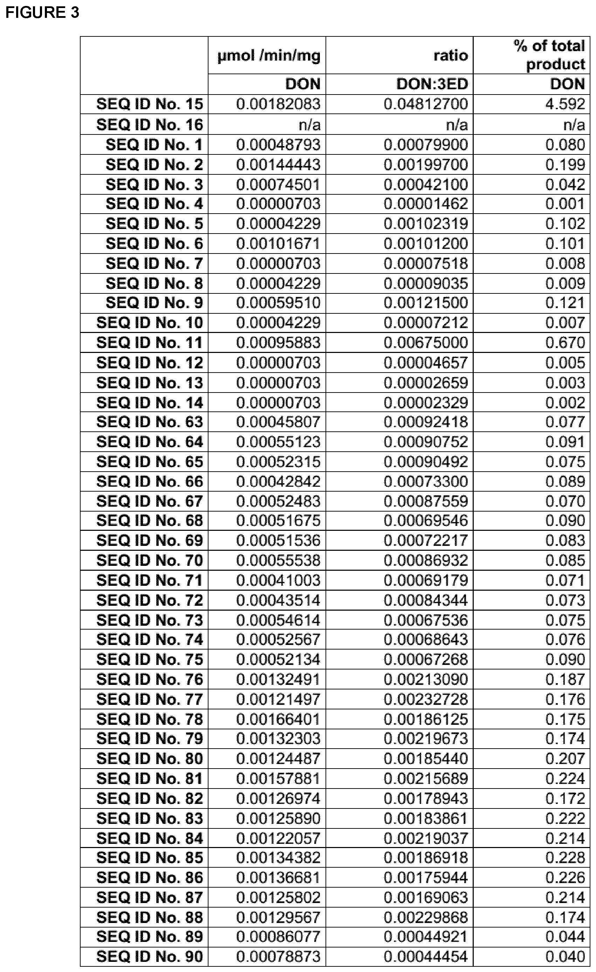

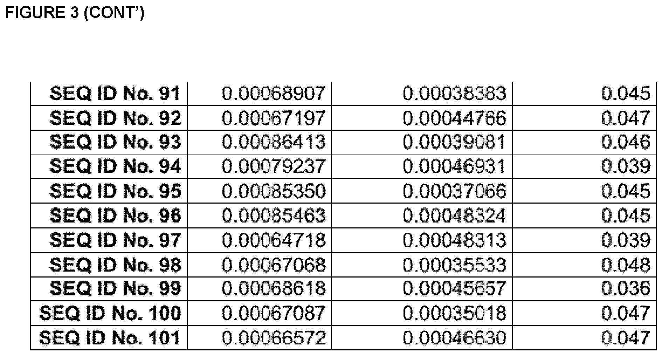

Fig. 3 shows data for different enzymes with regard to the yield of DON. Notably, no data for SEQ ID NO. 16 has been shown, because SEQ ID NO. 16 could not be produced in sufficient amounts to perform the required analytical assays. However, it is - without wishing to be bound to theory - be expected that SEQ ID NO. 16 will perform similar to SEQ ID NO. 15. The reason for this is that SQ ID NO. 15 and 16 share highly similar sequences especially in the regions of loop A and loop B.

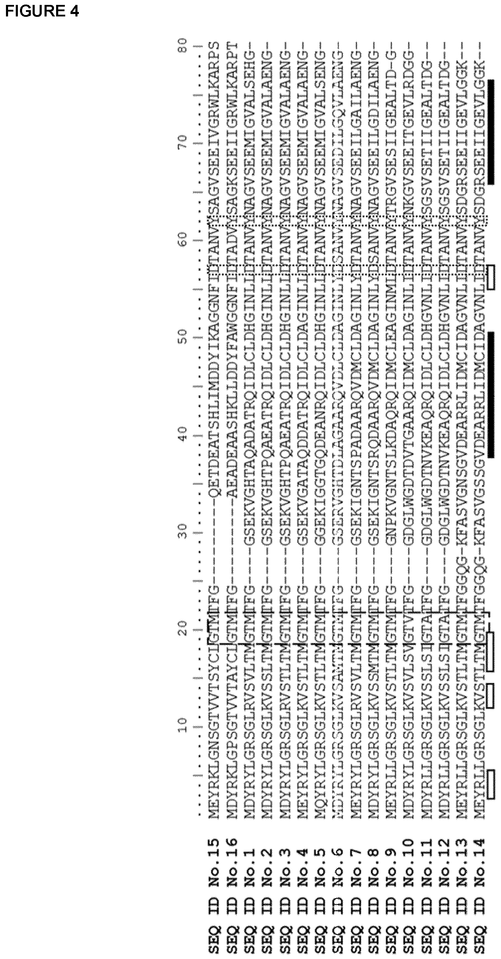

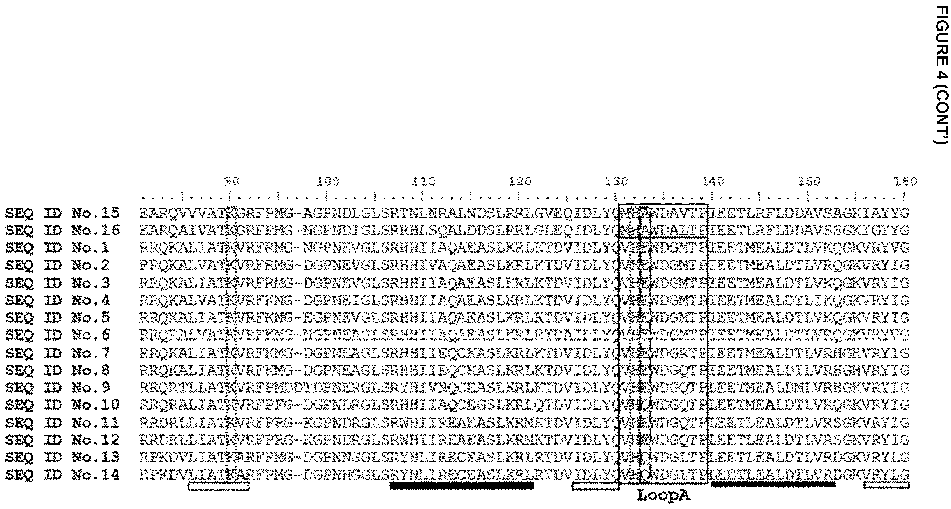

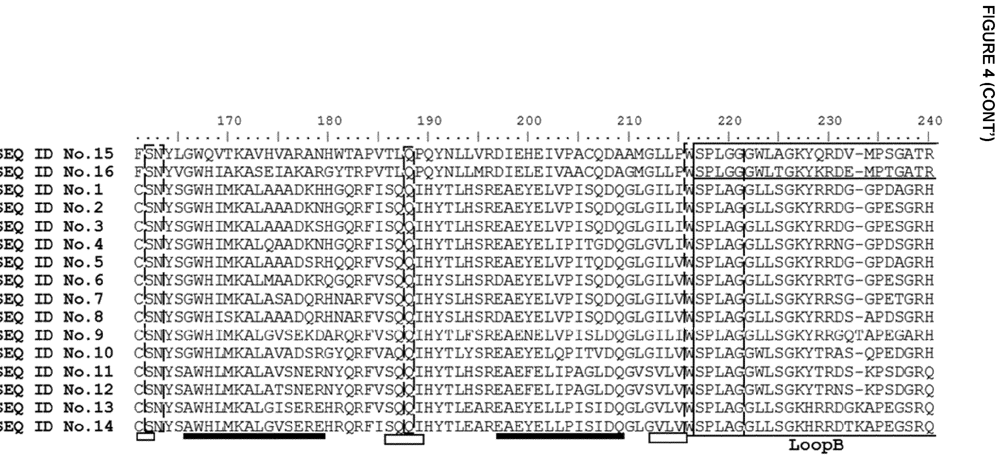

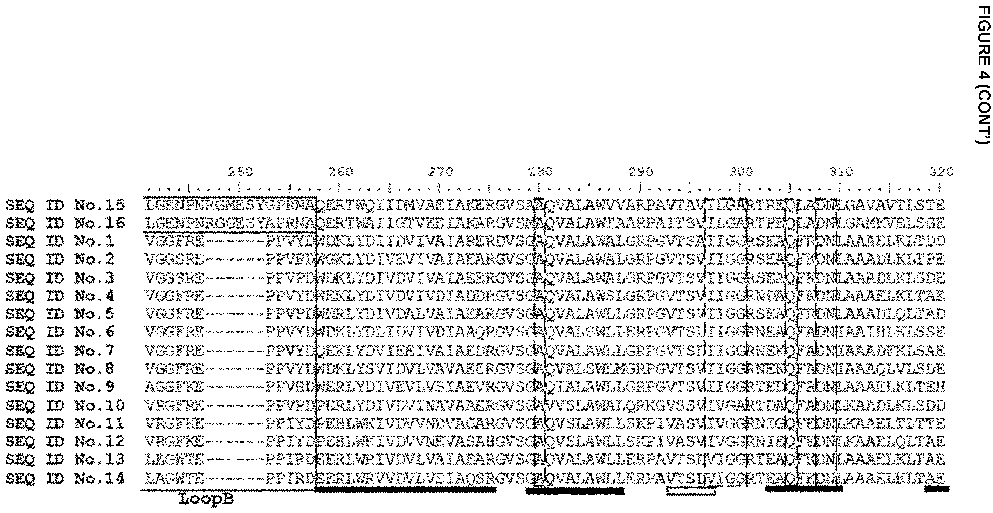

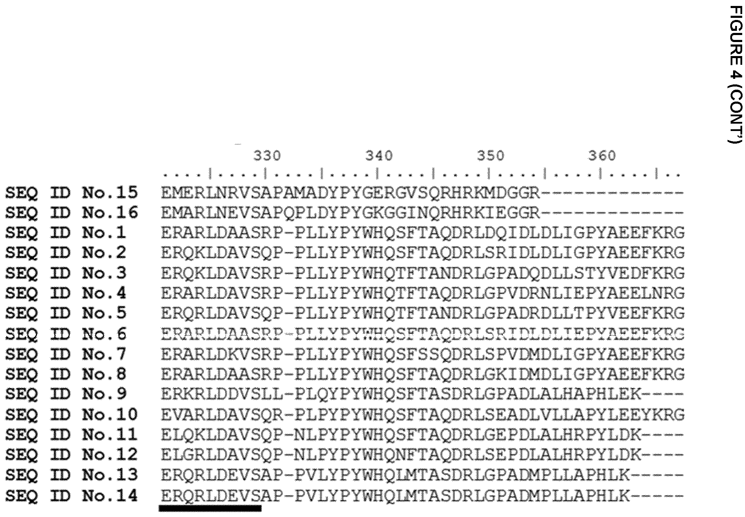

Fig. 4 Substrate specificity loops A and B depicted in the whole sequence of different enzymes described herein. Black bars represent alpha helices and white bars represent beta sheets. Dashed frames indicate the location of residues involved in the predicted active site and the residues making up the catalytic tetrade are framed with dotted lines
   These are also summarized in the sequence table shown herein.

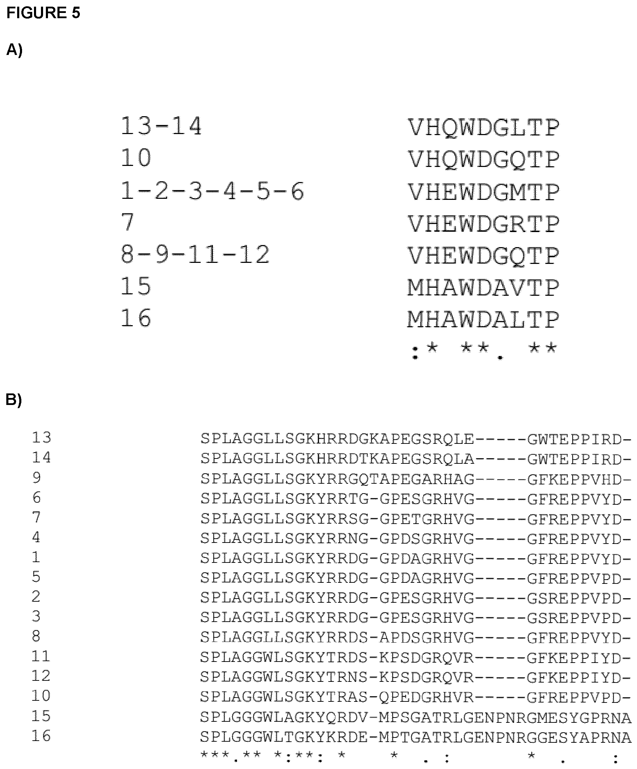

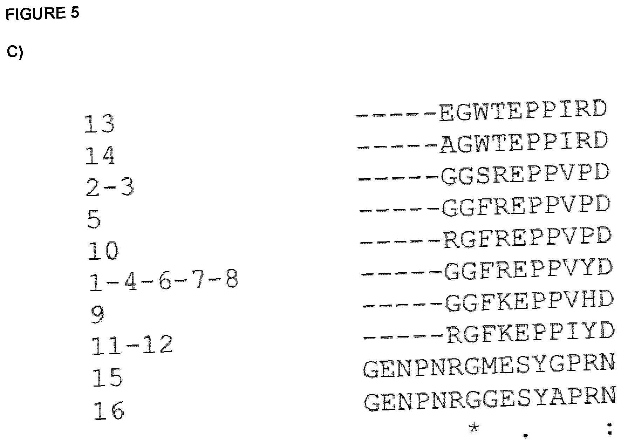

Fig. 5 Sequence Alignment performed with the Clustal W program. A) is the sequence alignment of loop A of SEQ ID NO. 1-14 with SEQ ID NO. 15 and 16. B) is the sequence alignment of loop B of SEQ ID NO. 1-14 with SEQ ID NO. 15 and 16. C) is the sequence alignment of a part of loop B of SEQ ID NO. 1-14 with SEQ ID NO. 15 and 16, which part is - without wishing to be bound to theory - believed to mediate contact to the substrate.

It was surprisingly found that reductases of any one of SEQ ID NO. 1-14 and 63-101 more efficiently convert 3-keto-DON into 3-epi-DON than prior art enzymes. In this conversion a 3-oxo group of trichothecenes (e.g. DON) is converted into a 3-hydroxy group more efficiently compared to prior art enzymes SEQ ID NO. 15 and 16. This higher efficiency is shown in the examples. Since most of the trichothecenes share the feature that they comprise a 3-hydroxy group at position 3 (as will also be discussed later herein) this group can be converted into a 3-oxo group, similarly to the reaction seen for DON. The enzymes of any one of SEQ ID NO. 1-14 and 63-101 are thus capable to convert any trichothecenes comprising a 3-oxo group into trichothecenes comprising a hydroxyl group.

Notably, the enzymes of the present invention are capable of highly stereoselective conversion. This means that the 3-oxo group is converted into a 3-hydroxy group of a specific isomer with higher efficiency than the other 3-hydroxy isomer. Specifically, as shown in the examples the polypeptides used in the invention can convert the 3-oxo group with higher efficiency into the hydroxyl group in S conformation (3-epi-DON) than into the hydroxyl group in R conformation (DON).

It was further surprisingly found that the enzymes of any one of SEQ ID NO. 1-14, 63-101 share sequence identities in two loops in the following called loop A and loop B which are different from known prior art enzymes as shown in the sequence table herein. Further, the polypeptides used in the invention share a common motif that interacts with the substrate as also shown in the sequence table herein.

The present invention relates to an agrarian composition comprising
a) one or more polypeptide(s) comprising or consisting of a sequence of SEQ ID NO. 1-14 or a sequence having a sequence identity of at least 75 % to any one of SEQ ID NO. 1-14 , wherein the polypeptide is capable of converting the substrate 3-keto-DON into the products 3-epi-DON and DON with a 3-epi-DON:DON ratio of at least 25:1; and
b) a carrier.

Alternatively or additionally, the present invention relates to an agrarian composition comprising
a) one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON, and
b) a carrier.

The agrarian composition can be a composition for reducing the amount of 3-keto-DON. The composition can also be a composition for reducing the amount of trichothecenes comprising a 3-oxo group. The composition can be an additive for e.g. feed or food. The composition can also be feed or food. The composition can also be an additive for use in agrarian compositions or an agrarian composition. The agrarian composition can also be a composition comprising trichothecenes such as DON.

The term "polypeptide" when used herein means a peptide, a protein, or a polypeptide, which is used interchangeably and which encompasses amino acid chains of a given length, wherein the amino acid residues are linked by covalent peptide bonds. Also encompassed by the invention are amino acids other than the 20 proteinogenic amino acids of the standard genetic code known to a person skilled in the art, such as selenocysteine. Such polypeptides include any of SEQ ID NO. 1-14 and/or SEQ ID NO. 63-101.

The term polypeptide also refers to, and does not exclude, modifications of the polypeptide. Modifications include glycosylation, acetylation, acylation, phosphorylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cysteine, formation of pyroglutamate, formulation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination; see, for instance, PROTEINS - STRUCTURE AND MOLECULAR PROPERTIES, 2nd Ed., T. E. Creighton, W. H. Freeman and Company, New York (1993); POST-TRANSLATIONAL COVALENT MODIFICATION OF PROTEINS, B. C. Johnson, Ed., Academic Press, New York (1983), pgs. 1-12; Seifter, Meth. Enzymol. 182 (1990); 626-646, Rattan, Ann. NY Acad. Sci. 663 (1992); 48-62.

It is envisioned the one or more polypeptide(s) described herein comprise or consist of a sequence having a sequence identity of at least 80 %, at least 83 %, at least 85 %, at least 87 %, at least 90 %, at least 91 %, at least 92 %, at least 93 %, at least 94 %, at least 95 %, at least 96 %, at least 97 %, at least, 98 %, at least 99 % or more sequence identity to any one of SEQ ID NO. 1-14. It is further envisioned that the one or more polypeptide(s) described herein comprise or consist of a sequence having a sequence identity of at least 80 %, at least 83 %, at least 85 %, at least 87 %, at least 90 %, at least 91 %, at least 92 %, at least 93 %, at least 94 %, at least 95 %, at least 96 %, at least 97 %, at least, 98 %, at least 99 % or more sequence identity to any one of SEQ ID NO. 1. For example, the one or more polypeptide(s) described herein comprise or consist of a sequence having a sequence of SEQ ID NO. 63-75.

It is further envisioned that the one or more polypeptide(s) described herein comprise or consist of a sequence having a sequence identity of at least 80 %, at least 83 %, at least 85 %, at least 87 %, at least 90 %, at least 91 %, at least 92 %, at least 93 %, at least 94 %, at least 95 %, at least 96 %, at least 97 %, at least, 98 %, at least 99 % or more sequence identity to any one of SEQ ID NO. 3. For example, the one or more polypeptide(s) described herein comprise or consist of a sequence having a sequence of SEQ ID NO. 76-88.

It is further envisioned that the one or more polypeptide(s) described herein comprise or consist of a sequence having a sequence identity of at least 80 %, at least 83 %, at least 85 %, at least 87 %, at least 90 %, at least 91 %, at least 92 %, at least 93 %, at least 94 %, at least 95 %, at least 96 %, at least 97 %, at least, 98 %, at least 99 % or more sequence identity to any one of SEQ ID NO. 6. For example, the one or more polypeptide(s) described herein comprise or consist of a sequence having a sequence of SEQ ID NO. 89-101.

It is further encompassed by the present invention that the one or more polypeptide(s) described herein comprise or consist of a sequence having a sequence identity of at least 92 % to any one of SEQ ID NO. 1-14 comprises one or more amino acid substitutions compared to a sequence of any one of SEQ ID NO. 1-14. To determine the sequence identity of two sequences, for example, the sequence of interest is compared to any one of SEQ ID NO 1-14. For example, the one or more polypeptide(s) described herein can comprise or consist of a sequence having a sequence of SEQ ID NO. 63-101 or a sequence having at least 75 %, at least 80 %, at least 83 %, at least 85 %, at least 87 %, at least 90 %, at least 91 %, at least 92 %, at least 93 %, at least 94 %, at least 95 %, at least 96 %, at least 97 %, at least, 98 %, at least 99 % or more sequence identity to any one of SEQ ID NO. 63-101.

In accordance with the present invention, the term "identical" or "percent identity" in the context of two or more polypeptide sequences such as SEQ ID NO. 1-14 and/or 63-101 refers to two or more sequences or subsequences that are the same, or that have a specified percentage of nucleotides that are the same (e.g., at least 85 %, 90 %, 95 %, 96 %, 97 %, 98 % or 99 % identity), when compared and aligned for maximum correspondence over a window of comparison, or over a designated region as measured using a sequence comparison algorithm as known in the art, or by manual alignment and visual inspection. Sequences having, for example, 80 % to 95 % or greater sequence identity are considered to be substantially identical. Such a definition also applies to the complement of a test sequence. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Also available to those having skills in this art are the BLAST and BLAST 2.6 algorithms (Altschul Nucl. Acids Res. 25 (1977), 3389-3402). The BLASTP program for amino acid sequences uses as defaults a word size (W) of 6, an expect threshold of 10, and a comparison of both strands. Furthermore, the BLOSUM62 scoring matrix (Henikoff Proc. Natl. Acad. Sci., USA, 89, (1989), 10915; Henikoff and Henikoff (1992) 'Amino acid substitution matrices from protein blocks.' Proc Natl Acad Sci U S A. 1992 Nov 15;89(22):10915-9) can be used.

For example, BLAST2.6, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments.

As described herein the one or more polypeptides may have a certain sequence identity to any one of SEQ ID NO. 1-14. This implicates that these polypeptides can also be fragments and thus comprise amino acid deletions with regard to any one of SEQ ID NO. 1-14.

As described herein the one or more polypeptides may comprise amino acid substitutions with regard to any one of SEQ ID NO. 1-14. For example, the polypeptide may comprise one or more conservative amino acid substitutions or only comprise of one or more conservative amino acid substitutions compared to any one of SEQ ID NO. 1-14. It is further envisioned that the polypeptide may comprise one or more highly conservative amino acid substitutions or only comprise of one or more highly conservative amino acid substitutions compared to any one of SEQ ID NO. 1-14.

As used herein, "conservative" substitutions mean substitutions as listed as "Exemplary Substitutions" in Table 1 below. "Highly conservative" substitutions as used herein mean substitutions as shown under the heading "Preferred Substitutions" in Table 1 below.

**Table 1. Conservative amino acid substitutions.**

| Original | Exemplary substitution(s) - conservative | Preferred substitution(s) - conservative |
|---|---|---|
| Ala (A) (neutral and small) | Val (V) (non-polar and small) | Val (V) (nonpolar relatively small) |
| | Leu (L) (nonpolar and relatively small) | Gly (G) (neutral and small) |
| | Ile (I) (nonpolar and relatively small) | |
| | Gly (G) (neutral and small) | |
| Arg (R) (polar and relatively large) | Lys (K) (polar and relatively large) | Lys (K) (polar and relatively large) |
| | Gln (Q) (polar and relatively small) | |
| | Asn (N) (polar and relatively small) | |
| Asn (N) (polar and relatively small) | Gln (Q) (polar and relatively small) | Gln (Q) (polar and relatively small) |
| | His (H) (polar and relatively large) | |
| | Asp (D) (polar and relatively small) | |
| | Lys (K) (polar and relatively large) | |
| | Arg (R) (polar and relatively large) | |
| Asp (D) (negative charged side chain) | Glu (E) (polar and relatively small) | Glu (E) (polar and relatively small) |
| | Asn (N) (polar and relatively small) | |
| Cys (C) (special) | Ser (S) (neutral and small) | Ser (S) (neutral and small) |
| | Ala (A) (neutral and small) | |
| Gln (Q) (polar and relatively small) | Asn (N) (polar and relatively small) | Asn (N) (polar and relatively small) |
| | Glu (E) (polar and relatively small) | |
| Glu (E) (polar and relatively small) | Asp (D) (polar and relatively small) | Asp (D) (polar and relatively small) |
| | Gln (Q) (polar and relatively small) | |
| Gly (G) (neutral and small) | Ala (A) (neutral and small) | Ala (A) (neutral and small) |
| His (H) (polar and relatively large) | Asn (N) (polar and relatively small) | Arg (R) (polar and relatively large) |
| | Gln (Q) (polar and relatively small) | |
| | Lys (K) (polar and relatively large) | |
| | Arg (R) (polar and relatively large) | |
| Ile (I) (nonpolar relatively small) | Leu (L) (nonpolar relatively small) | Leu (L) (nonpolar relatively small) |
| | Val (V) (nonpolar relatively small) | |
| | Met (M) (nonpolar relatively small) | |
| | Ala (A) (neutral and small) | |
| | Phe (F) (nonpolar and relatively large) | |
| Leu (L) (nonpolar and relatively small) | Norl | Ile (I) (nonpolar relatively small) |
| | Ile (I) (nonpolar relatively small) | |
| | Val (V) (nonpolar relatively small) | |
| | Met (M) (nonpolar relatively small) | |
| | Ala (A) (neutral and small) | |
| Lys (K) (polar and relatively large) | Arg (R) (polar and relatively large) | Arg (R) (polar and relatively large) |
| | Gln (Q) (polar and relatively small) | |
| | Asn (N) (polar and relatively small) | |
| Met (M) (nonpolar and relatively small) | Leu (L) (nonpolar and relatively small) | Leu (L) (nonpolar and relatively small) |
| | Phe (F) (nonpolar and relatively large) | |
| | Ile (I) (nonpolar relatively small) | |
| Phe (F) (nonpolar and relatively large) | Leu (L) (nonpolar and relatively small) | Tyr (Y) (nonpolar and relatively large) |
| | Val (V) (nonpolar relatively small) | |
| | Ile (I) (nonpolar relatively small) | |
| | Ala (A) (neutral and small) | |
| | Tyr (Y) (nonpolar and relatively large) | |
| Pro (P) (neutral and small) | Ala (A) (neutral and small) | Ala (A) (neutral and small) |
| Ser (S) (neutral and small) | Thr (T) (neutral and small) | Thr (T) (neutral and small) |
| Thr (T) (neutral and small) | Ser (S) (neutral and small) | Ser (S) (neutral and small) |
| Trp (W) (nonpolar and relatively large) | Tyr (Y) (nonpolar and relatively large) | Tyr (Y) (nonpolar and relatively large) |
| | Phe (F) (nonpolar and relatively large) | |
| Tyr (Y) (nonpolar and relatively large) | Trp (W) (nonpolar and relatively large) | Phe (F) (nonpolar and relatively large) |
| | Phe (F) (nonpolar and relatively large) | |
| | Thr (T) (neutral and small) | |
| | Ser (S) (neutral and small) | |
| Val (V) (non-polar and small) | Ile (I) (nonpolar relatively small) | Leu (L) (nonpolar and relatively small) |
| | Leu (L) (nonpolar and relatively small) | |
| | Met (M) (nonpolar relatively small) | |
| | Phe (F) (nonpolar and relatively large) | |
| | Ala (A) (neutral and small) | |

A polypeptide comprising conservative amino acid substitutions may be any polypeptide of SEQ ID NO. 63-101.

The polypeptides of the present invention are capable of converting or convert the substrate 3-keto-DON into the products 3-epi-DON (abbreviated as 3-epi-DON herein) and DON (abbreviated as DON herein) with a ratio of at least 25:1 (3-epi-DON/DON). The reaction described herein is depicted in the reaction scheme below obtained from Hassan et al. (2017) "The enzymatic epimerization of DON by Devosia mutans proceeds through the formation of 3-keto-DON intermediate." Scientific Reports 7, article number 6929.

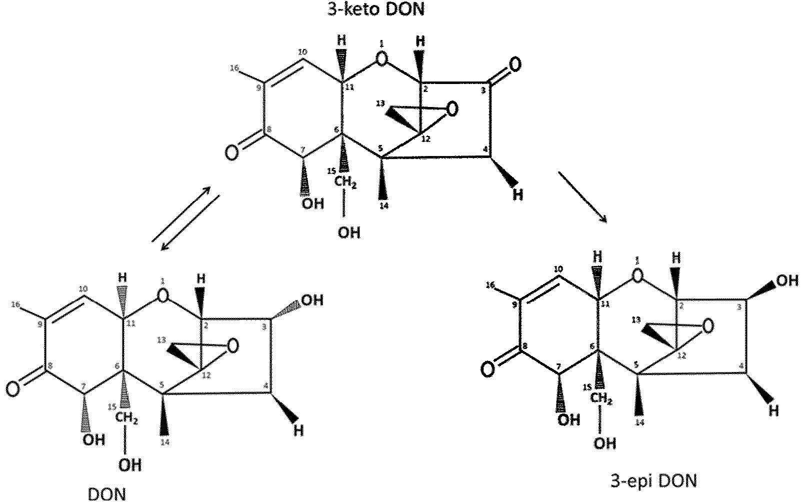

Reaction scheme: Conversion of 3-keto DON into two isomers 3-epi-DON and DON adapted from Hassan et al. (2017).

As can be seen from the above reaction scheme 3-keto DON can be converted into two isomers, namely DON (R configuration) and 3-epi-DON (S configuration). The ratio of at least 25:1 indicates that the isomer 3-epi-DON is obtained in a higher amount than the isomer DON from 3-keto DON by the polypeptides of the present invention. Therefore, the ratio indicates that the isomer 3-epi-DON is obtained 50 times more than the isomer DON. The ratio is calculated by dividing the obtained amount of the isomer 3-epi-DON by the obtained amount of the isomer DON.

It is encompassed by the present disclosure that the polypeptide(s) described herein is/are capable of converting or convert the substrate 3-keto-DON into the products 3-epi-DON and DON with a 3-epi-DON:DON ratio of at least 25:1, at least 30:1, at least 40:1, at least 50:1, at least 60:1, at least 70:1, at least 80:1, at least 90:1, at least 100:1, at least 150:1, at least 200:1, 250:1, 300:1, 350:1, 400:1, 500:1, 600:1, 700:1, 800:1, 900:1, 1000:1 or more.

It is clear that the ratio is not calculable in the case that no DON and only 3-epi-DON is obtained. This is because mathematically a division trough the number 0 is not possible. Yet, the present invention also relates to polypeptides that are 100 % stereo selective for 3-epi-DON. Thus, in case the division is a division through the number 0, such a polypeptide is still embraced by the present invention. Yet, if the division is 0, because only DON is obtained, such polypeptides are not encompassed by the present invention.

One way to measure whether a certain polypeptide is capable of converting or converts the substrate 3-keto-DON into the products 3-epi-DON and DON with a 3-epi-DON:DON ratio of at least 25:1 is explained in detail in the Examples.

It is further envisioned by the present invention that the polypeptide(s) used in the present invention is/are capable of converting or convert the substrate 3-keto-DON into the product 3-epi-DON and optionally additionally into the product DON amounting to 100 % of total product, wherein at least 96 % of the total product is 3-epi-DON. As can be seen from the above reaction scheme, 3-keto DON can be converted into two isomers, namely 3-epi-DON and DON. These two isomers thus represent the total product. The total product per definition equals 100 %. Since the polypeptides of the present invention are highly stereoselective, one isomer (here 3-epi-DON) is obtained with higher efficiency than the other (here DON). Specifically, of the total product obtained (equaling 100 %) at least 96 % is 3-epi-DON.

It is also encompassed by the present invention that at least 96.5 %, 97 %, 97.5 %, 98 %, 98.5 %, 99 %, 99.5 %, 99,9 % or 100 % of the total product (equaling 100 %) is 3-epi-DON.

The polypeptides of any one of SEQ ID NO. 1-14 and/or of SEQ ID NO. 63-101 are thus highly efficient in converting 3-keto-DON into 3-epi-DON. This can also be seen by the fact that only a small amount of polypeptide is necessary to convert 1 µmol 3-epi-DON per minute. This measurement indicates that the polypeptides described herein have a high activity.

The present disclosure thus encompasses that the amount of polypeptide is at most 7 mg, at most 6 mg, at most 5 mg, at most 3 mg, at most 2 mg, at most 1 mg or less, when the 3-epi-DON is obtained at a rate of 1 µmol 3-epi-DON per minute from 3-keto-DON. Similarly, the polypeptides of the present invention are capable of converting 3-keto-DON into 3-epi-DON with a specific activity of at least 0.1 µmol/min/mg, at least 0.2 µmol/min/mg, at least 0.3 µmol/min/mg, at least 0.4 µmol/min/mg, at least 0.5 µmol/min/mg, at least 0.6 µmol/min/mg at least 0.8 µmol/min/mg, at least 1.0 µmol/min/mg, at least 1.2 µmol/min/mg, at least 1.4 µmol/min/mg or at least 1.6 µmol/min/mg.

On the other hand, the activity of the polypeptides described herein for converting 3-epi-DON into DON is low. Thus, a high amount of polypeptide is necessary to obtain DON. Specifically, the amount of polypeptide is at least 600 mg, at least 700 mg, at least 800 mg, at least 900 mg, at least 1000 mg, at least 1100 mg, at least 1300 mg, at least 1500mg, at least 1600 mg, at least 1700 mg, at least 1800 mg, at least 1900 mg, at least 2000 mg or more, when the DON is obtained at a rate of 1 µmol DON per minute from 3-keto-DON.

It is further encompassed that the polypeptide(s) described herein are capable of converting or convert 3-keto-DON into DON with a specific activity of at most 0.0015 µmol/min/mg, at most 0.0010 µmol/min/mg, at most 0.0009 µmol/min/mg, at most 0.0008 µmol/min/mg, at most 0.0007 µmol/min/mg, at most 0.0006 µmol/min/mg or less.

The polypeptide of any one of SEQ ID NO. 1-14 and/or of SEQ ID NO. 63-101, which is capable to convert or converts 3-keto-DON into 3-epi-DON and optionally additionally DON can be a reductase. For example, the polypeptide(s) can be an aldo-keto reductase.

The polypeptides described herein not only have the same functional features but also share structural features. Also these structural features are different from the structural features of the enzymes known from the prior art as depicted in the sequence table herein. Without being bound to theory the inventors expect that these structural features (loop A and/or loop B) are important for the substrate specificity and activity of the enzymes due to the fact that loops A and B comprise parts of the active site. Since inter alia the amino acid Histidine (H) of the catalytic tetrade is located in loop A this loop most likely also contributes more to the activity of the inventive enzymes. Further, without wishing to be bound to theory it is believed that loop B has a greater role than loop A with substrate specificity. In particular, the motif X8-G-X9-X10-X11-X12-X13-X14-X15-X16 in loop B as described herein interacts with the substrate. The substrate can inter alia be a 3-oxo-trichothecene such as 3-keto DON or a redox factor.

It is thus plausible that these common structural features seen in all the polypeptides used in the invention are responsible for the high stereo selectivity as well as for the high specific activity of conversion e.g. from 3-keto DON into 3-epi-DON.

For example, as encompassed by the present disclosure the polypeptide(s) used in the present invention comprise or consist of more than 353, 350, 348 or 351 amino acids. Contrary thereto prior art enzymes of SEQ ID NO. 15 and 16 have 343 amino acids. It is further envisioned that the polypeptide(s) comprise or consist of more than 345, 346, 347, 348, 349, 350, 351, 352, 353 or more amino acids. The polypeptide(s) can comprise or consist of 348, 350, 351, 353 amino acids. The polypeptide(s) can comprise or consist of 344-353 amino acids, preferably 348-353 amino acids.

The polypeptide(s) used in the present invention can comprise one or more loops within the amino acids sequence. A "loop" as used herein is an irregular structure in proteins/polypeptides responsible for direction changes and connection of main secondary structures alpha helices, beta strands. Loops can be unstructured and build random coils, turns, and strands. The difference to the main secondary structures is the lack of a regular geometric repetition of psi and phi angles in residues along the structure). In enzymes they are part of or build substrate binding sites, and active sites. Often the sequence of these structures is not conserved. The structural features of loops are also described in Kundert K, Kortemme T. (2019).

It is further encompassed that the polypeptide(s) described herein comprise a loop within the amino acid positions corresponding to amino acid positions
125 to 133 of SEQ ID NO. 1-10 or 63-101;
127 to 135 of SEQ ID NO. 13 or 14;
124 to 132 of SEQ ID NO. 11 or 12. This loop is also described as loop A herein. Loop A as disclosed herein encompasses the sequence located downstream of the motif I-D/E-LYQ contained in beta sheet 4 and upstream of the motif I/M/L-E/D-E/D-T-L/M contained in alpha helix 4 e.g. of SEQ ID NO. 1-14, 63-101.

It is further envisioned that the polypeptide within the amino acid positions corresponding to the amino acid positions
125 to 133 of SEQ ID NO. 1-10 or 63-101;
127 to 135 of SEQ ID NO. 13 or 14;
124 to 132 of SEQ ID NO. 11 or 12 comprises or consists of a sequence which does not comprise an alanine (Ala/A). Thus, the polypeptides described herein do not comprise an alanine in the loop A region.

It is further encompassed by the present disclosure that the polypeptide(s) used in the present invention within the amino acid positions corresponding to the amino acid positions
125 to 133 of SEQ ID NO. 1-10 or 63-101;
127 to 135 of SEQ ID NO. 13 or 14;
124 to 132 of SEQ ID NO. 11 or 12 , can comprise a sequence comprising the sequence X1-H-X2-W-D-X3-X4-T-P, wherein
X1 is not M;
X2 is not A;
X3 is not A;
X4 is not V.

Thus, the polypeptide(s) described herein can comprise a sequence comprising the sequence X1-H-X2-W-D-X3-X4-T-P, wherein
X1 is not M;
X2 is not A;
X3 is not A;
X4 is not V.

For example, the polypeptide(s) described herein within the amino acid positions corresponding to the amino acid positions
125 to 133 of SEQ ID NO. 1-10 or 63-101;
127 to 135 of SEQ ID NO. 13 or 14;
124 to 132 of SEQ ID NO. 11 or 12 , can comprise or consist of a sequence comprising the sequence X1-H-X2-W-D-X3-X4-T-P, wherein
X1 is V, I, L, M, F or A, preferably, V;
X2 is E, D, Q or N, preferably E or Q;
X3 is G or A, preferably G;
X4 is M, L, **F,** I, R, K, Q, N, E, V, A, or Norleucin, preferably M, R, Q or L.

Thus, the polypeptide(s) described herein can comprise a sequence comprising the sequence X1-H-X2-W-D-X3-X4-T-P, wherein
X1 is V, I, L, M, F or A, preferably, V;
X2 is E, D, Q or N, preferably E or Q;
X3 is G or A preferably G;
X4 is M, L, **F,** I, R, K, Q, N, E, V, A, or Norleucin, preferably M, R, Q or L.

It is further envisioned that the polypeptide(s) described herein can comprise or consist of a sequence of any one of SEQ ID NO. 20-24 or a sequence having at least 80 %, 85 %, 90 %, 95 %, 98 %, 99% or more sequence identity to any one of SEQ ID NO. 20-24.

Additionally or alternatively, the polypeptide(s) described herein can comprise a loop within the amino acid positions corresponding to amino acid positions
211 to 244 of SEQ ID NO. 1-8, 10 or 63-101;
211 to 245 of SEQ ID NO. 9;
210 to 243 of SEQ ID NO. 11 or 12;
213 to 247 of SEQ ID NO. 13 or 14. This loop is also described as loop B herein. Loop B as disclosed herein starts with the serine located in the common motif WSPL-X-G and ends in front of the alpha helix with the sequence X-X-R/K/H-T/L-W/Y-X-I/V/L-I/V/L and consists of 35 or fewer residues e.g. of SEQ ID NO. 1-14, 63-101.

It is further envisioned that the polypeptide(s) described herein within the amino acid positions corresponding to the amino acid positions
211 to 244 of SEQ ID NO. 1-8, 10 or 63-101;
211 to 245 of SEQ ID NO. 9;
210 to 243 of SEQ ID NO. 11 or 12;
213 to 247 of SEQ ID NO. 13 or 14, can comprise a sequence, which does not comprise a methionine (Met/M).

For example, the polypeptide(s) described herein within the amino acid positions corresponding to the amino acid positions
211 to 244 of SEQ ID NO. 1-8, 10 or 63-101;
211 to 245 of SEQ ID NO. 9;
210 to 243 of SEQ ID NO. 11 or 12;
213 to 247 of SEQ ID NO. 13 or 14, can comprise a sequence comprising the sequence S-P-L-X5-G-G-X6-L-X7-G-K, wherein
X5 is not G;
X6 is L, I, V, M, A, Y, **F,** Norleucin or W;
X7 is not A or T.

Thus, the polypeptide(s) described herein can comprise a sequence comprising the sequence S-P-L-X5-G-G-X6-L-X7-G-K, wherein
X5 is not G;
X6 is L, I, V, M, A, Y, **F,** Norleucin or W;
X7 is not A or T.

The polypeptide(s) described herein within the amino acid positions corresponding to the amino acid positions
211 to 244 of SEQ ID NO. 1-8, 10 or 63-101;
211 to 245 of SEQ ID NO. 9;
210 to 243 of SEQ ID NO. 11 or 12;
213 to 247 of SEQ ID NO. 13 or 14, can comprise a sequence comprising the sequence S-P-L-X5-G-G-X6-L-X7-G-K, wherein
X5 is A, V, L, G or I, preferably A;
X6 is L, I, V, M, A, Y, **F,** Norleucin or W, preferably L or W;
X7 is S or **T,** preferably S.

Thus, the polypeptide(s) described herein can comprise a sequence comprising the sequence S-P-L-X5-G-G-X6-L-X7-G-K, wherein
X5 is A, V, L or I, preferably A;
X6 is L, I, V, M, A, Y, **F,** Norleucin or W, preferably L or W;
X7 is S or **T,** preferably S.

It is further envisioned that the polypeptide(s) described herein can comprise or consist of a sequence of any one of SEQ ID NO. 33-46 or a sequence having at least 80 %, 85 %, 90 %, 95 %, 98 %, 99% or more sequence identity to any one of SEQ ID NO. 33-46.

For example, the polypeptide(s) described herein within the amino acid positions corresponding to the amino acid positions
211 to 244 of SEQ ID NO. 1-8, 10 or 63-101;
211 to 245 of SEQ ID NO. 9;
210 to 243 of SEQ ID NO. 11 or 12;
213 to 247 of SEQ ID NO. 13 or 14, can comprise a sequence comprising the sequence X8-G-X9-X10-X11-X12-X13-X14-X15-X16, wherein
X8 is G, R, K, Q, N, A, V, L, I, S, **T,** E, D;
X9 is not M or G;
X10 is not E;
X11 is not S;
X12 is not Y;
X13 is not G or A;
X14 is not P;
X15 is R, K, Q, N, P, A, Y, **W, F, T, S,** H,
X16 is not N.

Thus, the polypeptide(s) described herein can comprise a sequence comprising the sequence X8-G-X9-X10-X11-X12-X13-X14-X15-X16, wherein
X8 is G, R, K, Q, N, A, V, L, I, S, **T,** E, D;
X9 is not E;
X10 is not E;
X11 is not S;
X12 is not Y;
X13 is not G or A;
X14 is not P;
X15 is R, K, Q, N, P, A, Y, W, **F, T,** S, H; and
X16 is not N.

It is also envisioned that the polypeptide(s) described herein within the amino acid positions corresponding to the amino acid positions
211 to 244 of SEQ ID NO. 1-8, 10 or 63-101;
211 to 245 of SEQ ID NO. 9;
210 to 243 of SEQ ID NO. 11 or 12;
213 to 247 of SEQ ID NO. 13 or 14, can comprise a sequence comprising the sequence X8-G-X9-X10-X11-X12-X13-X14-X15-X16, wherein
X8 is G, R, K, Q, N, A, V, L, I, S, **T, E,** D preferably G, R, A, E, more preferably, G;
X9 is W, S, **T, F,** L, V, I, A or Y preferably W, S, **F,** more preferably F;
X10 is **T,** S, R, Q, N or K preferably **T,** R or K, more preferably R;
X11 is E, D or Q preferably E;
X12 is P or A preferably P;
X13 is P or A, preferably P;
X14 is I, L, M, A, F or V, preferably I or V, more preferably V;
X15 is R, K, Q, N, P, A, H, **W, F, T,** S or Y, preferably R, P, H or Y, more preferably P;
X16 is D, E or N, preferably D.

Thus, the polypeptide(s) described herein can comprise a sequence comprising the sequence X8-G-X9-X10-X11-X12-X13-X14-X15-X16, wherein
X8 is G, R, K, Q, N, A, V, L, I, S, **T,** E, D preferably G, R, A, E, more preferably, G;
X9 is W, S, T, **F,** L, V, I, A or Y preferably W, S, **F,** more preferably F;
X10 is **T,** S, R, Q, Nor K preferably **T,** R or K, more preferably R;
X11 is E, D or Q, preferably E;
X12 is P or A, preferably P;
X13 is P or A, preferably P;
X14 is I, L, M, A, F or V, preferably I or V, more preferably V;
X15 is R, K, Q, N, P, A, H, **W, F, T,** S or Y, preferably R, P, H or Y, more preferably P;
X16 is D, E or N, preferably D.

It is further envisioned that the polypeptide(s) described herein can comprise or consist of a sequence of any one of SEQ ID NO. 25-32 or a sequence having at least 80 %, 85 %, 90 %, 95 %, 98 %, 99% or more sequence identity to any one of SEQ ID NO. 25-32.These sequences represent the sequences that can be in contact with the substrate.

It is also envisioned that the polypeptide(s) described herein can comprise a sequence comprising the sequence of any one of SEQ ID NO. 25-32 and/or a sequence as shown in any one of SEQ ID NO. 33-46.

It is also encompassed that the polypeptide(s) described herein can comprise a sequence comprising the sequence of any one of SEQ ID NO. 25-32 and/or 33-46 and optionally additionally a sequence as shown in any one of SEQ ID NO. 20-24.

It is also envisioned that the polypeptide(s) described herein comprise or consist of a sequence as shown in any one of SEQ ID NO. 1-14, 63-101.

The polypeptide(s) described herein can be present in the composition with a carrier. The carrier can be any suitable carrier. Further, the composition may comprise 1, 2, 3, 4, 5, 6, or more carriers. The carrier may be an eatable component, preferably a non-toxic component and/or a component providing for a texture.

For example, the carrier can be a solid or a liquid. Exemplary solids include food/feed supplement(s), dietary supplements, nutraceutical(s) and/or a pharmaceutical(s). Exemplary feed/food supplements inter alia include feed/food additives, vitamins, minerals, amino acids, essential fatty acids, fibre, trace elements, minerals, antioxidants, plant extracts and herbal extracts.

The carrier can also be a carrier for an enzyme. Carriers for enzymes can be both of inorganic and organic origin. Potential inorganic materials used for the immobilization of enzymes are silica (sol-gel silica, fumed silica, colloidal silica nanoparticles and silica gels) and different oxides such as titanium oxide, aluminium oxide and zirconium oxide. Furthermore, clay materials such as bentonite, halloysite, kaolinite, montmorillonite, sepiolite and calcium apatite are applied. Additionally, carbon-based materials such as activated carbons and charcoal are known as effective enzyme immobilizers. Organic enzyme carriers can be biopolymers but also synthetic polymers. The biopolymers include carbohydrates and proteins. Typical examples are maltodextrin, trehalose, inulin, collagen, cellulose, keratins, carragenaan, chitin, chitosan and alginate. As examples for synthetic polymers polyaniline, polyamides, polystyrene, polyurethane, polypropylene, polyvinyl alcohol and ion exchange resins can be mentioned. An enzyme carrier can also be a carrier as described in Zdarta et al. (2018).

The carrier can also be a liquid. Exemplary liquids include H2O, aqueous solutions, salt solutions (e.g. buffers), gels, viscous preparations, fats or oils. Preferable aqueous solutions containing H2O and further substances like puffer substances and/or polyalcohols like polyalkylene oxides (PAO), poly-vinyl alcohols (PVA), polyethylene-co-maleic acid anhydrides, polystyrene-co-malic acid anhydrides, dextrans, celluloses, hydrolyzates of chitosan, starches, glycogen, sorbitol, agarose and derivatives thereof, guar gum, pullulan, inulin, xanthan gum, carrageenan, pectin, alginic acid hydrolyzates, biopolymers, sorbitol, glycerol, cellobiose, and mono propylene glycol (MPG).

The composition can be a composition for reducing the amount of DON. In such cases the composition further comprises an enzyme capable of converting DON into 3-keto-DON. The composition can also be a composition for reducing the amount of trichothecenes. In such cases the composition further comprises an enzyme capable of converting trichothecenes into trichothecenes comprising a 3-oxo group. The compositions described herein can further comprise one or more enzyme(s) capable of converting or converting DON into 3-keto-DON and/or trichothecenes comprising a 3-oxo group into trichothecenes comprising a 3-hydroxyl group. Exemplary enzymes, which can be used in the composition, are inter alia described in WO2016/154640 or WO2019/046954. Thus, the enzymes converting DON into 3-keto DON may comprise or consist of a sequence as disclosed in SEQ ID No. 1 of WO2016/154640 (SEQ ID NO: 61 herein or SEQ ID NO. 7 of WO2019/046954 (SEQ ID NO. 62 herein). Thus, the composition(s) as described herein may further comprise an enzyme comprising or consisting of SEQ ID NO. 61 and/or 62.

The present invention also relates to a method of converting a trichothecene comprising a 3-oxo group into a trichothecene comprising a 3-hydroxy group, the method comprising contacting one or more polypeptide(s) comprising or consisting of a sequence of SEQ ID NO. 1-14 or a sequence having a sequence identity of at least 75 % to any one of SEQ ID NO. 1-14 and/or a sequence of SEQ ID NO. 63-101 with a trichothecene comprising a 3-oxo group.

Trichothecenes have the following common structure:

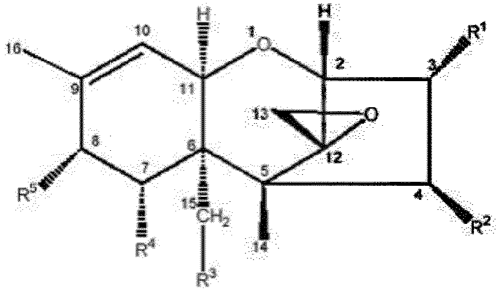

The skilled person knows which substitutions can be present at R1, R2, R3, R4 and R5. It is known that toxicity of trichothecenes is (at least) partly due to hydroxyl (-OH) group present at the C-3 atom. Thus, in some trichothecenes the C-3 is connected to a hydroxyl group (R1 = -OH/-hydroxy group).

Trichothecenes comprising a 3-hydroxy (3-OH) group (at position R1 in the above formula) are known to the skilled person. Non limiting examples of such trichothecenes include DON (DON, CAS No. 51481-10-8), T-2 Toxin (CAS No. 21259-20-1), HT-2 Toxin (CAS No. 26934-87-2), Nivalenol (CAS No. 23282-20-4), Fuseranon X (CAS No. 23255-69-8), Scirpenetriol (CAS No. 2270-41-9), 15-Acetoxyscirpenol (CAS No. 2623-22-5), 4,15-Diacetoxyscirpenol (CAS No. 2270-40-8), Deacetylneosolaniol (CAS No. 74833-39-9), Neosolaniol (CAS No. 36519-25-2), Sporotrichiol (CAS No. 101401-89-2) and Sambucinol (CAS No. 90044-33-0).

These trichothecenes comprising a 3-hydroxy (3-OH) group can be oxidized into trichothecenes comprising a 3-oxo (=O; at position R1). Enzymes capable of such conversion are described herein. The polypeptides can then convert these trichothecenes comprising a 3-oxo group into preferably one of the two isomers of trichothecenes comprising a 3-hydroxy (3-OH) group, namely the R and the S configuration.

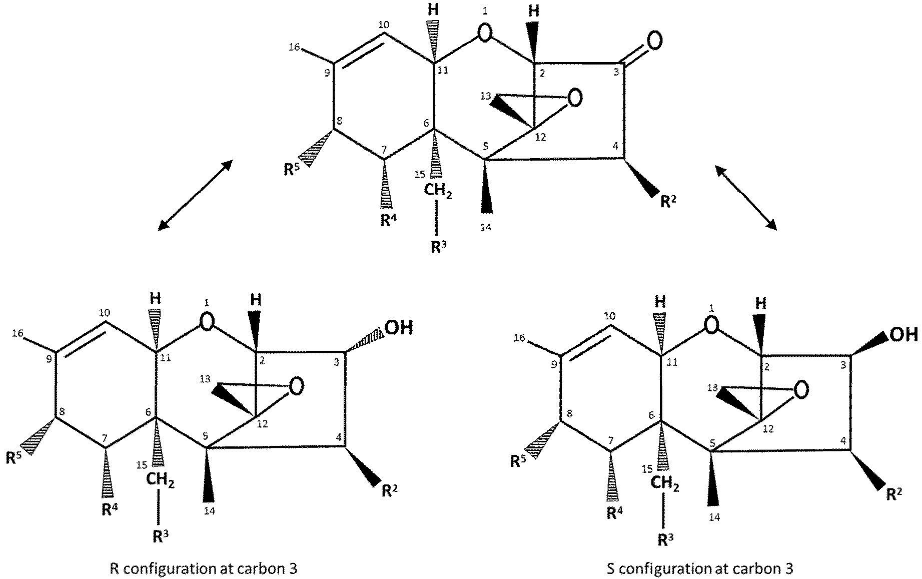

It is encompassed by the present invention that the trichothecene comprising a 3-hydroxy group is present in the S configuration.

Additionally or alternatively, the present invention also relates to a method of converting 3-keto-DON into 3-epi-DON, the method comprising contacting one or more polypeptide(s) comprising or consisting of a sequence of SEQ ID NO. 1-14 or a sequence having a sequence identity of at least 75 % to any one of SEQ ID NO. 1-14 with 3-keto-DON.

The present invention further relates to a method of reducing the content of DON in a composition comprising DON or of reducing the toxicity of a composition comprising DON by converting DON into 3-epi-DON, the method comprising
a) contacting the composition with an enzyme capable of converting DON into 3-keto DON as described inter alia herein; and
b) subsequently or concurrently contacting the composition with one or more polypeptide(s) comprising or consisting of a sequence of SEQ ID NO. 1-14 or a sequence having a sequence identity of at least 75 % to any one of SEQ ID NO. 1-14. This method may further include the step of contacting the composition with at least one quinone cofactor and/or at least one metal ion and/or at least one redox cofactor. The at least one quinone cofactor may be selected from the group of PQQ (pyrroloquinoline quinone (CAS No. 72909-34-3), tryptophan tryptophylquinone (TTQ, CAS No. 134645-25-3), topaquinone (TPQ, CAS No. 64192-68-3), lysine tyrosylquinone (LTQ, CAS No. 178989-72-5) and cysteine tryptophylquinone (CTQ, CAS No. 400616-72-0). The metal ion, enabling a fast and complete binding of the quinone cofactor to the enzyme capable of converting DON into 3-keto DON, may preferably be the alcohol dehydrogenase SEQ ID NO. 1-3 described in WO2016/154640, can be selected from the group of Li⁺, Na⁺, K⁺, Mg²⁺, Ca²⁺, Zn²⁺, Zn³⁺, Mn²⁺, Mn³⁺, Fe²⁺, Fe³⁺, Cu²⁺, Cu³⁺, Co²⁺ and Co³, preferably Ca²⁺ and Mg²⁺. The at least one redox cofactor can be selected from the group of NAD+, NADP+, the phenazine methosulphate group (PMS, CAS No. 299-11-6), PMS derivatives, potassium hexacyanoferrate (III), sodium hexacyanoferrate (III), cytochrome C, coenzyme Q1, coenzyme Q10, methylene blue and N,N,N',N'-tetramethyl-p-phenylenediamine (TMPD). Examples of PMS derivatives are: 1-hydroxyphenazine, 2-(pentaprenyloxy)dihydrophenazine, 5,10-dihydro-9-dimethylallylphenazine-1-carboxylic acid, 5,10-dihydrophenazine-1-carboxylic acid, 5-methylphenazinium methyl sulfate, 6-acetophenazine-1-carboxylic acid, benthophoenin, clofazimine, dihydromethanophenazine, esmeraldic acid, esmeraldin B, izumiphenazine A - C, Janus Green B cation, methanophenazine pelagiomicin A, phenazine, phenazine-1,6-dicarboxylic acid, phenazine-1-carboxamide, phenazine-1-carboxylic acid, phenosafranine, pyocyanin, saphenamycin, or saphenic acid methyl ester.

The present invention also concerns nucleic acid molecules encoding for a polypeptide as described herein. The nucleic acid may be introduced or inserted into an expression vector. The term "expression vector" refers to a nucleic acid molecule construct that is able to express a gene *in vivo* or *in vitro.* In particular, it can encompass DNA constructs suitable for transferring the polypeptide-encoding nucleotide sequence into the host cell so as to be integrated in the genome or freely located in the extrachromosomal space, and to intracellularly express the polypeptide-encoding nucleotide sequence and, optionally, transport the polypeptide out of the cell.

Further, the present invention also relates to a host cell (e.g., recombinant isolated host cell) comprising one or more polypeptide(s) comprising or consisting of a sequence of SEQ ID NO. 1-14 or a sequence having a sequence identity of at least 75 % to any one of SEQ ID NO. 1-14, wherein said recombinant host cell is transferred with an expression vector capable of producing said one or more polypeptide(s). The present invention also relates to a host cell comprising a nucleic acid sequence encoding for a polypeptide as described herein.

The term "host cell" refers to all cells (e.g., recombinant host cell, isolated host cell or isolated recombinant host cell) containing either a nucleotide sequence to be expressed, or an expression vector, and which is able to produce an enzyme or a polypeptide according to the invention. The term also encompasses any progeny of a parent cell that is not identical to the parent cell due to mutations that occur during replication, as well as a recombinant host cell, an isolated host cell (e.g., an isolated recombinant host cell). In particular, this refers to prokaryotic and/or eukaryotic cells, preferably *Pichia pastoris, Escherichia coli, Bacillus subtilis, Streptomyces, Hansenula, Trichoderma, Lactobacillus, Aspergillus,* plant cells and/or spores of *Bacillus, Trichoderma* or *Aspergillus.* The name P. *pastoris* used herein is synonymous with the name *Komagataella pastoris, P. pastoris* being the older and *K. pastoris* the systematically newer name (Yamada et al. (1995)). Notably, species of *Komagataella pastoris* have been recently reassigned to be *Komagataella phaffii* (Kurtzman (2009)). *Komagataella phaffii* as used herein can e.g. relate to strains *Komagataella phaffii* CBS 7435, *Komagataella phaffii* GS115 or *Komagataella phaffii* JC308.

It is further encompassed that the host cell can further express a cofactor for the polypeptide(s) described herein. The co-factor may be any suitable co-factor. For example, the co-factor is NAD/H or NADP/H.

The present disclosure further relates to a plant genetically modified to express one or more polypeptide(s) comprising or consisting of a sequence of SEQ ID NO. 1-14 or a sequence having a sequence identity of at least 75 % to any one of SEQ ID NO. 1-14. Exemplary plants include inter alia corn (maize), wheat, barley, rye and oat.

The present disclosure also relates to a seed of a plant as described herein.

The present disclosure also relates to a preparation comprising
a) one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1; and
b) a carrier.

Additionally or alternatively, the present disclosure also relates to a preparation comprising
a) one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON.
b) a carrier.

A "preparation" in accordance with the present invention is obtainable by using the polypeptide(s) described herein in a composition as described herein. In one aspect, the preparation can therefore comprise the polypeptides or parts of the polypeptide(s) described herein as well as further components such as the carrier, agrarian extracts etc. The preparation can also comprise further molecules and/or proteins and/or substances e.g. a left over from a buffer used in the method of the present invention due to e.g. less efficient purification of the polypeptide(s) described herein.

The present invention further relates to a polypeptide comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a ratio of at least 25:1 (3-epi-DON/DON) for use in the prevention and/or treatment of mycotoxicosis.

The present invention also concerns a polypeptide comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON for use in the prevention and/or treatment of mycotoxicosis.

The present disclosure also relates to a pharmaceutical composition comprising a polypeptide comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the product 3-epi-DON and optionally additionally into the product DON amounting to 100 % of total product, wherein at least 96 % of the total product is 3-epi-DON for use in the prevention and/or treatment of mycotoxicosis.

The pharmaceutical composition may further comprise a pharmaceutically acceptable carrier.

Preferably, a therapeutically effective amount of the one or more polypeptide(s) as described herein are administered. The subject can be afflicted with mycotoxicosis.

In some preferred aspects the method or use relating to the present invention (e.g., comprising SEQ ID NO: 1) is carried out in the pH range from about 4 to about 10 (e.g., pH from about 4.5 to about 10, e.g., pH about 6.5) and/or is carried out in the temperature (T) range from about 14°C to about 60°C (e.g., T from about 14.9°C to about 36°C, e.g., T from about 25°C to about 31°C), preferably as described in the Examples section herein (e.g., Examples 4-5).

It is noted that as used herein, the singular forms "a", "an", and "the", include plural references unless the context clearly indicates otherwise. Thus, for example, reference to "a polypeptide" includes one or more of such different polypeptides and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific aspects relating to the invention described herein.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

The terminology used herein is for the purpose of describing particular aspects only, and is not intended to limit the scope of the present invention, which is defined solely by the claims.

The following sequences are used in the present application.

| **#** | **Synony m** | **Sequence** |
|---|---|---|
| 1 | SEQ ID NO. 1 | MDYRYLGRSGLRVSVLTMGTMTFGGSEKVGHTAQADATRQIDLCLDHGI NLLDTANVYNAGVSEEMIGVALSEHGRRQKALIATKVRFRMGNGPNEVG LSRHHIIAQAEASLKRLKTDVIDLYQVHEWDGMTPIEETMEALDTLVKQGK VRYVGCSNYSGWHIMKALAAADKHHGQRFISQQIHYTLHSREAEYELVPI SQDQGLGILIWSPLAGGLLSGKYRRDGGPDAGRHVGGFREPPVYDWDK LYDIIDVIVAIARERDVSGAQVALAWALGRPGVTSAIIGGRSEAQFRDNLAA AELKLTDDERARLDAASRPPLLYPYWHQSFTAQDRLDQIDLDLIGPYAEEF KRG |
| | | Loop A from amino acid position 125 to 133 |
| | | Loop B from amino acid position 211 to 244 (length 34 amino acids) |
| | | Total amino acids: 353 |
| 2 | SEQ ID NO. 2 | MDYRYLGRSGLKVSSLTMGTMTFGGSEKVGHTPQAEATRQIDLCLDHGI NLLDTANVYNAGVSEEMIGVALAENGRRQKALVATKVRFRMGDGPNEVG LSRHHIVAQAEASLKRLKTDVIDLYQVHEWDGMTPIEETMEALDTLVRQG KVRYIGCSNYSGWHIMKALAAADKNHGQRFISQQIHYTLHSREAEYELVPI SQDQGLGILIWSPLAGGLLSGKYRRDGGPESGRHVGGSREPPVPDWGK LYDIVEVIVAIAEARGVSGAQVALAWALGRPGVTSVIIGGRSEAQFKDNLA AADLKLTPEERQKLDAVSQPPLLYPYWHQSFTAQDRLSRIDLDLIGPYAEE FKRG |
| | | Loop A from amino acid position 125 to 133 |
| | | Loop B from amino acid position 211 to 244 (length 34 amino acids) |
| | | Total amino acids: 353 |
| 3 | SEQ ID NO. 3 | MDYRYLGRSGLRVSTLTMGTMTFGGSEKVGHTPQAEATRQIDLCLDHGI NLLDTANVYNAGVSEEMIGVALAENGRRQKALIATKVRFRMGDGPNEVG LSRHHIIAQAEASLKRLKTDVIDLYQVHEWDGMTPIEETMEALDTLVKQGK VRYIGCSNYSGWHIMKALAAADKSHGQRFISQQIHYTLHSREAEYELVPIS QDQGLGILIWSPLAGGLLSGKYRRDGGPESGRHVGGSREPPVPDWDKL YDIVDVIVAIAEERGVSGAQVALAWALGRPGVTSVIIGGRSEAQFKDNLAA |
| | | ADLKLSDEERQKLDAVSRPPLLYPYWHQTFTANDRLGPADQDLLSTYVE DFKRG Insert A from amino acid position 4[?]5 to 4[?]6 |
| | | Loop A from amino acid position 125 to 133 |
| | | Loop B from amino acid position 211 to 244 (length 34 amino acids) |
| | | Total amino acids: 353 |
| 4 | SEQ ID NO. 4 | MEYRYLGRSGLKVSTLTMGTMTFGGSEKVGATAQDDATRQIDLCLDAGI NLLDTANVYNAGVSEEMIGVALAENGRRQKALVATKVRFKMGDGPNEIGL SRHHIIAQAEASLKRLKTDVIDLYQVHEWDGMTPIEETMEALDTLIKQGKV RYIGCSNYSGWHIMKALQAADKNHGQRFISQQIHYTLHSREAEYELIPITG DQGLGVLIWSPLAGGLLSGKYRRNGGPDSGRHVGGFREPPVYDWEKLY DIVDVIVDIADDRGVSGAQVALAWSLGRPGVTSVIIGGRNDAQFKDNLAAA ELKLTAEERARLDAVSRPPLLYPYWHQTFTAQDRLGPVDRNLIEPYAEEL NRG |
| | | Loop A from amino acid position 125 to 133 |
| | | Loop B from amino acid position 211 to 244 (length 34 amino acids) |
| | | Total amino acids: 353 |
| 5 | SEQ ID NO. 5 | MQYRYLGRSGLKVSTLTMGTMTFGGGEKIGGTGQDEANRQIDLCLDHGI NLLDTANVYNAGVSEEMIGVALSENGRRQKALIATKVRFKMGEGPNEVGL SRHHIIAQAEASLKRLKTDVIDLYQVHEWDGMTPIEETMEALDTLVKQGKV RYIGCSNYSGWHIMKALAAADSRHQQRFVSQQIHYTLHSREAEYELVPIT QDQGLGILVWSPLAGGLLSGKYRRDGGPDAGRHVGGFREPPVPDWNRL YDIVDALVAIAEARGVSGAQVALAWLLGRPGVTSVIIGGRSEAQFRDNLAA ADLQLTADERQRLDAVSQPPLLYPYWHQTFTANDRLGPADRDLLTPYVE EFKRG |
| | | Loop A from amino acid position 125 to 133 |
| | | Loop B from amino acid position 211 to 244 (length 34 amino acids) |
| | | Total amino acids: 353 |
| 6 | SEQ ID NO. 6 | MDYRYLGRSGLKVSAMTMGTMTFGGSERVGHTDLAGAARQVDLCLDAG INLYDSANVYNAGVSEDILGQVLAENGRRQRALVATKVRFKMGNGPNEA GLSRHHIIAQAEASLKRLRTDAIDLYQVHEWDGMTPIEETMEALDTLVRQG KVRYVGCSNYSGWHIMKALMAADKRQGQRFVSQQIHYSLHSRDAEYELV PISQDQGLGILVWSPLAGGLLSGKYRRTGGPESGRHVGGFREPPVYDW DKLYDLIDVIVDIAAQRGVSGAQVALSWLLERPGVTSLIIGGRNEAQFADNI AAIHLKLSSEERARLDAASRPPLLYPYWHQSFTAQDRLSRIDLDLIEPYAE EFKRG |
| | | Loop A from amino acid position 125 to 133 |
| | | Loop B from amino acid position 211 to 244 (length 34 amino acids) |
| | | Total amino acids: 353 |
| 7 | SEQ ID NO. 7 | MEYRYLGRSGLRVSVLTMGTMTFGGSEKIGNTSPADAARQVDMCLDAGI NLYDTANVYNAGVSEEILGAILAENGRRQKALIATKVRFKMGDGPNEAGL SRHHIIEQCKASLKRLKTDVIDLYQVHEWDGRTPIEETMEALDTLVRHGHV RYIGCSNYSGWHIMKALASADQRHNARFVSQQIHYSLHSREAEYELVPIS QDQGLGILVWSPLAGGLLSGKYRRSGGPETGRHVGGFREPPVYDQEKL YDVIEEIVAIAEDRGVSGAQVALAWLLGRPGVTSLIIGGRNEKQFADNIAAA DFKLSAEERARLDKVSRPPLLYPYWHQSFSSQDRLSPVDMDLIGPYAEEF KRG |
| | | Loop A from amino acid position 125 to 133 |
| | | Loop B from amino acid position 211 to 244 (length 34 amino acids) |
| | | Total amino acids: 353 |
| 8 | SEQ ID NO. 8 | MDYRYLGRSGLKVSSMTMGTMTFGGSEKIGNTSRQDAARQVDMCLDAG INLYDSANVYNAGVSEEILGDILAENGRRQKALIATKVRFKMGDGPNEAGL SRHHIIEQCKASLKRLKTDVIDLYQVHEWDGQTPIEETMEALDILVRHGHV RYIGCSNYSGWHISKALAAADQRHNARFVSQQIHYSLHSRDAEYELVPIS QDQGLGILVWSPLAGGLLSGKYRRDSAPDSGRHVGGFREPPVYDWDKL YSVIDVLVAVAEERGVSGAQVALSWLMGRPGVTSVIIGGRNEKQFADNIA |
| | | AAQLVLSDEERARLDAASRPPLLYPYWHQSFTAQDRLGKIDMDLIGPYAE EFKRG |
| | | Loop A from amino acid position 125 to 133 |
| | | Loop B from amino acid position 211 to 244 (length 34 amino acids) |
| | | Total amino acids: 353 |
| 9 | SEQ ID NO. 9 | MEYRLLGRSGLKVSTLTMGTMTFGGNPKVGNTSLKDAQRQIDMCLEAGI NMLDTANVYTRGVSESIIGEALTDGRRQRTLLATKVRFPMDDTDPNERGL SRYHIVNQCEASLKRLKTDVIDLYQVHEWDGQTPLEETMEALDMLVRHG KVRYIGCSNYSGWHIMKALGVSEKDARQRFVSQQIHYTLFSREAENELVP ISLDQGLGILIWSPLAGGLLSGKYRRGQTAPEGARHAGGFKEPPVHDWE RLYDIVEVLVSIAEVRGVSGAQIALAWLLGRPGVTSVIIGGRTEDQFRDNL AAAELKLTEHERKRLDDVSLLPLQYPYWHQSFTASDRLGPADLALHAPHL EK |
| | | Loop A from amino acid position 125 to 133 |
| | | Loop B from amino acid position 211 to 245 (length 35 amino acids) |
| | | Total amino acids: 350 |
| 10 | SEQ ID NO. 10 | MDYRYLGRSGLKVSVLSVGTVTFGGDGLWGDTDVTGAARQIDMCLDAGI NLIDTANVYNKGVSEEITGEVLRDGGRRQRALIATKVRFPFGDGPNDRGL SRHHIIAQCEGSLKRLQTDVIDLYQVHQWDGQTPLEETMEALDTLVRQGK VRYIGCSNYSGWHLMKALAVADSRGYQRFVAQQIHYTLYSREAEYELQPI TVDQGLGILVWSPLAGGWLSGKYTRASQPEDGRHVRGFREPPVPDPER LYDIVDVINAVAAERGVSGAVVSLAWALQRKGVSSVIVGARTDAQFADNL KAADLKLSDDEVARLDAVSQRPLPYPYWHQSFTAQDRLSEADLVLLAPYL EEYKRG |
| | | Loop A from amino acid position 125 to 133 |
| | | Loop B from amino acid position 211 to 244 (length 34 amino acids) |
| | | Total amino acids: 353 |
| 11 | SEQ ID NO. 11 | MDYRLLGRSGLKVSSLSIGTATFGGDGLWGDTNVKEAQRQIDLCLDHGV NLIDTANVYSGSVSETIIGEALTDGRRDRLLIATKVRFPRGKGPNDRGLSR WHIIREAEASLKRMKTDVIDLYQVHEWDGQTPLEETLEALDTLVRSGKVR YIGCSNYSAWHLMKALAVSNERNYQRFVSQQIHYTLHSREAEFELIPAGL DQGVSVLVWSPLAGGWLSGKYTRDSKPSDGRQVRGFKEPPIYDPEHLW KIVDVVNDVAGARGVSGAQVSLAWLLSKPIVASVIVGGRNIGQFEDNLKA AELTLTTEELQKLDAVSQPNLPYPYWHQSFTAQDRLGEPDLALHRPYLDK [?] |
| | | Loop A from amino acid position 124 to 132 |
| | | Loop B from amino acid position 210 to 243 (length 34 amino acids) |
| | | Total amino acids: 348 |
| 12 | SEQ ID NO. 12 | MDYRLLGRSGLKVSSLSIGTATFGGDGLWGDTNVKEAQRQIDLCLDHGV NLIDTANVYSGGSVSETIIGEALTDGRRDRLLIATKVRFPRGKGPNDRGLSR WHIIREAEASLKRMKTDVIDLYQVHEWDGQTPLEETLEALDTLVRSGKVR YIGCSNYSAWHLMKALATSNERNYQRFVSQQIHYTLHSREAEFELIPAGL DQGVSVLVWSPLAGGWLSGKYTRNSKPSDGRQVRGFKEPPIYDPEHLW KIVDVVNEVASAHGVSGAQVSLAWLLSKPIVASVIVGGRNIEQFEDNLKAA ELQLTAEELGRLDAVSQPNLPYPYWHQNFTAQDRLSEPDLALHRPYLDK |
| | | Loop A from amino acid position 124 to 132 |
| | | Loop B from amino acid position 210 to 243 (length 34 amino acids) |
| | | Total amino acids: 348 |
| 13 | SEQ ID NO. 13 | MEYRLLGRSGLKVSTLTMGTMTFGGQGKFASVGNSGVDEARRLIDMCID AGVNLIDTANVYSDGRSEEIIGEVLGGKRPKDVLIATKARFPMGDGPNNG GLSRYHLIRECEASLKRLRTDVIDLYQVHQWDGLTPLEETLEALDTLVRDG KVRYLGCSNYSAWHLMKALGISEREHRQRFVSQQIHYTLEAREAEYELLP ISIDQGLGVLVWSPLAGGLLSGKHRRDGKAPEGSRQLEGWTEPPIRDEE RLWRIVDVLVAIAEARGVSGAQVALAWLLERPAVTSLVIGGRTEAQFKDN LAAAELKLTAEERQRLDEVSAPPVLYPYWHQLMTASDRLGPADMPLLAP HLK |
| | | Loop A from amino acid position 127 to 135 |
| | | Loop B from amino acid position 213 to 247 (length 35 amino acids) |
| | | Total amino acids: 351 |
| 14 | SEQ ID NO. 14 | MEYRLLGRSGLKVSTLTMGTMTFGGQGKFASVGSSGVDEARRLIDMCID AGVNLIDTANVYSDGRSEEIIGEVLGGKRPKDVLIATKARFPMGDGPNHG GLSRYHLIRECEASLKRLRTDVIDLYQVHQWDGLTPLEETLEALDTLVRDG KVRYLGCSNYSAWHLMKALGVSEREHRQRFISQQIHYTLEAREAEYELLP ISIDQGLGVLVWSPLAGGLLSGKHRRDTKAPEGSRQLAGWTEPPIRDEER LWRVVDVLVSIAQSRGVSGAQVALAWLLERPAVTSLVIGGRTEAQFKDNL AAAELKLTAEERQRLDEVSAPPVLYPYWHQLMTASDRLGPADMPLLAPH LK |
| | | Loop A from amino acid position 127 to 135 |
| | | Loop B from amino acid position 213 to 247 (length 35 amino acids) |
| | | Total amino acids: 351 |
| 15 | SEQ ID NO. 15 Describe d by Hassan et al. (2017) cited herein | MEYRKLGNSGTVVTSYCLGTMTFGQETDEATSHLIMDDYIKAGGNFIDTA NVYSAGVSEEIVGRWLKARPSEARQVVVATKGRFPMGAGPNDLGLSRTN LNRALNDSLRRLGVEQIDLYQMHAWDAVTPIEETLRFLDDAVSAGKIAYY GFSNYLGWQVTKAVHVARANHWTAPVTLQPQYNLLVRDIEHEIVPACQD AAMGLLPWSPLGGGWLAGKYQRDVMPSGATRLGENPNRGMESYGPRN AQERTWQIIDMVAEIAKERGVSAAQVALAWVVARPAVTAVILGARTREQL ADNLGAVAVTLSTEEMERLNRVSAPAMADYPYGERGVSQRHRKMDGGR |
| | | Loop A from amino acid position 121 to 129 |
| | | Loop B from amino acid position 207 to 246 (length 40 amino acids) |
| | | Total amino acids: 343 |
| 16 | SEQ ID NO. 16 describe d in WO2019 /046954 | MDYRKLGPSGTVVTAYCLGTMTGAEADEAASHKLLDDYFAWGGNFIDTA DVYSAGKSEEIIGRWLKARPTEARQAIVATKGRFPMGNGPNDIGLSRRHL SQALDDSLRRLGLEQIDLYQMHAWDALTPIEETLRFLDDAVSSGKIGYYGF SNYVGWHIAKASEIAKARGYTRPVTLQPQYNLLMRDIELEIVAACQDAGM GLLPWSPLGGGWLTGKYKRDEMPTGATRLGENPNRGGESYAPRNAQE RTWAIIGTVEEIAKARGVSMAQVALAWTAARPAITSVILGARTPEQLADNL GAMKVELSGEEMARLNEVSAPQPLDYPYGKGGINQRHRKIEGGR |
| | | Loop A from amino acid position 121 to 129 |
| | | Loop B from amino acid position 207 to 246 (length 40 amino acids) |
| | | Total amino acids: 342 |
| 17 | SEQ ID NO. 17 | X1-H-X2-W-D-X3-X4-T-P |
| 18 | SEQ ID NO. 18 | S-P-L-X5-G-G-X6-L-X7-G-K |
| 19 | SEQ ID NO. 19 | X8-G-X9-X10-X11-X12-X13-X14-X15-X16 |
| 20 | SEQ ID NO. 20 | VHEWDGMTP (= Loop A of SEQ ID NO. 1-6) |
| 21 | SEQ ID NO. 21 | VHEWDGRTP (= Loop A of SEQ ID NO. 7) |
| 22 | SEQ ID NO. 22 | VHEWDGQTP (= Loop A of SEQ ID NO. 8, 9, 11, 12) |
| 23 | SEQ ID NO. 23 | VHQWDGLTP (= Loop A of SEQ ID NO. 13 and 14) |
| 24 | SEQ ID NO. 24 | VHQWDGQTP (= Loop A of SEQ ID NO. 10) |
| 25 | SEQ ID NO. 25 | GGFREPPVYD (= part of loop B of SEQ ID NO. 1, 4, 6-8) |
| 26 | SEQ ID NO. 26 | GGSREPPVPD (= part of loop B of SEQ ID NO. 2 and 3) |
| 27 | SEQ ID NO. 27 | GGFREPPVPD (= part of loop B of SEQ ID NO. 5) |
| 28 | SEQ ID NO. 28 | GGFKEPPVHD (= part of loop B of SEQ ID NO. 9) |
| 29 | SEQ ID NO. 29 | RGFREPPVPD (= part of loop B of SEQ ID NO. 10) |
| 30 | SEQ ID NO. 30 | EGWTEPPIRD (= part of loop B of SEQ ID NO. 13) |
| 31 | SEQ ID NO. 31 | AGWTEPPIRD (= part of loop B of SEQ ID NO. 14) |
| 32 | SEQ ID NO. 32 | RGFKEPPIYD (= part of loop B of SEQ ID NO. 11, 12) |
| 33 | SEQ ID NO. 33 | SPLAGGLLSGKYRRDGGPDAGRHVGGFREPPVYD (SEQ ID NO. 1) |
| 34 | SEQ ID NO. 34 | SPLAGGLLSGKYRRDGGPESGRHVGGSREPPVPD (SEQ ID NO. 2) |
| 35 | SEQ ID NO. 35 | SPLAGGLLSGKYRRDGGPESGRHVGGSREPPVPD (SEQ ID NO. 3) |
| 36 | SEQ ID NO. 36 | SPLAGGLLSGKYRRNGGPDSGRHVGGFREPPVYD (SEQ ID NO. 4) |
| 37 | SEQ ID NO. 37 | SPLAGGLLSGKYRRDGGPDAGRHVGGFREPPVPD (SEQ ID NO. 5) |
| 38 | SEQ ID NO. 38 | SPLAGGLLSGKYRRTGGPESGRHVGGFREPPVYD (SEQ ID NO. 6) |
| 39 | SEQ ID NO. 39 | SPLAGGLLSGKYRRSGGPETGRHVGGFREPPVYD (SEQ ID NO. 7) |
| 40 | SEQ ID NO. 40 | SPLAGGLLSGKYRRDSAPDSGRHVGGFREPPVYD (SEQ ID NO. 8) |
| 41 | SEQ ID NO. 41 | SPLAGGLLSGKYRRGQTAPEGARHAGGFKEPPVHD (SEQ ID NO. 9) |
| 42 | SEQ ID NO. 42 | SPLAGGWLSGKYTRASQPEDGRHVRGFREPPVPD (SEQ ID NO. 10) |
| 43 | SEQ ID NO. 43 | SPLAGGWLSGKYTRDSKPSDGRQVRGFKEPPIYD (SEQ ID NO. 11) |
| 44 | SEQ ID NO. 44 | SPLAGGWLSGKYTRNSKPSDGRQVRGFKEPPIYD (SEQ ID NO. 12) |
| 45 | SEQ ID NO. 45 | SPLAGGLLSGKHRRDGKAPEGSRQLEGWTEPPIRD (SEQ ID NO. 13) |
| 46 | SEQ ID NO. 46 | SPLAGGLLSGKHRRDTKAPEGSRQLAGWTEPPIRD (SEQ ID NO. 14) |
| 47 | SEQ ID NO. 47 | Atgacccgatttgcccattatcccagcctgaccgatatgcccgtcgtcatctcaggcggtgcctcgggcat cggcgaaagcctggtgcgaaactttgccgcccagggcgccagggtcggtttcgtcgatatcgccgtcg acgccggtgaacgcctcgccgccgaattgacggggcaggggcaaagcgtagccttcaccgcctgcg acatcaccgatacccaggcctatcaatccgccattgccggcttcgccgccacccatggcgatgccctgg ccctggtcaacaatgccgcccatgaccagcgccacgattgggctgaggtcacccctgcctattgggac gatcgcatggcggtcaacctcaagcatgcctttttcgccatccaggccgtggcgccgggcatggtccgc gccgggcgcggctccatcatcaataccggctcgatcagctggatgatcatgtcctcccgcattcccgtct atgaaaccgccaaggccgccatccatggcctcacccgcggcatggcgcgcgaactggggcagtcgg gcattcgcgtcaattccctggtgcccggctgggtcatgaccgagcggcaattgacccattggctcgatgc cgagggcgagcgggccatcgcacaaaaccaggtgctggccggccgggtctatcccgacgatgtcgc gcgcatggccctgttccttgccgccgacgacagcgccatgatttcggcccagcaatttgtcgtcgatggc ggctgggccaatgcctga |
| | DNA sequenc e encoding SEQ ID NO. 2 | |
| 48 | SEQ ID NO. 48 | Atgagccgcattttcaacgtcgccatcgtcggggtcggcatcggccgcagccacctggtcgagggctat gtgcccaatgccgacaaatacaaggtcgtcgcgctgtgcgatctcaatgtcgagcgcatgacgcccatt gccgacgaattcggcgtcgagcgccgcatcaccaatttcgacgatatcctcgccatggacgatatcgac |
| | DNA sequenc e encoding SEQ ID NO. 3 | atcatcgatatctgcaccccgccgggcctgcattatcccatggtcatggcggcgctgaaggccggcaag catgtaatctgcgaaaagccgctggtcggctcgctcgcccaggtcgatgaagtcattgcccaggaaaa gatctccaagggcaagctgatgccggtgttccagtatcgctttggcgacggcatcgaacaggccaagg ccatcatcgatgccggcattgccggcaagccattcgtcggcacggtggaaaccatgtggcgccgtggc cccgattattatgccgtgccatggcgcggcaaatgggccaccgaactgggcggcgtgctgatgaccca tgccatccaccagcacgatctcttcacctatctcatgggcgatgtgacgcggctcttcggccgggtcgcc acccgcgtcaacgccattgaagtggaagactgcgttaccgccagccttgagctcgaaaatggcgccct gggcagctttaccgccacgctgggcgcagccgaggaaatcacaaggctgttcctgaccttcgagaac gtcaccttcgaaagcgattacgaggcctataatcccggcgccaagccgtggcgcatccagccgcgca atcccgagacggcggcgaaaatcgacgccctgctcaaggactggaagcatgtgccctcgcgtttcga aacgcagatggcccgcttccatgcggccctcttgggtgaagcgccgatgccggtgacctcggccgattc gcgccgggcgctggaaatcgtcaccgccttctaccattcctcgactacccacgaagatgtggccctgcc catcgcgtccgaccatccgcgttatcagagctgggttccgccccagttccgcgctgcctga |
| 49 | SEQ ID NO. 49 | Atggactatcgctatctcggccgctcgggccttagggtttcggtgctgaccatgggcacaatgacctttgg cggctcggaaaaggtggggcatacagcccaggccgatgcgacgcgccagattgatctctgccttgatc acggcatcaacctgctcgacaccgccaatgtctataatgccggggtctccgaggagatgatcggggtg gcgctgagcgaacatggccggcggcaaaaggctttgattgccaccaaggtgcgcttcaggatgggta acgggcccaatgaagtggggctgagccggcatcatatcattgcccaggccgaggcgagcctcaagc ggctcaagaccgatgtcatcgatctctaccaggtgcatgaatgggatgggatgacgcccatcgaggag acgatggaagcgctcgatacgctcgtgaaacagggcaaggtgcgctatgtcggctgctcgaattattcg ggctggcacatcatgaaggcgctggctgcggccgacaaacatcatggccagcgcttcatctcccagca gatccactacacgctgcattcgcgcgaagccgaatatgaactggtgccgatcagccaggaccagggg ctgggtattctgatttggtcgccgctggcgggcggactgctctcgggcaagtatcgtcgcgatggcgggc ccgatgccggccgccatgttggaggcttccgcgagccgccggtttatgactgggacaagctctacgaca ttatcgacgtcatcgtcgccattgcccgggagcgcgatgtgtcgggcgcgcaggtggcgttggcctggg cattggggcggccgggcgtgacctcggccattatcggtggccgtagcgaggcccagttccgcgacaat ctggcggcggccgaactcaagctgaccgatgacgagcgcgcccggctcgatgcggccagccggcc gccattgctttatccctattggcaccagtcttttaccgcccaggaccggctggaccagatcgacctggacc tgatcgggccctatgccgaggagtttaaacgtggctga |
| | DNA sequenc e encoding SEQ ID NO. 1 | |
| 50 | SEQ ID NO. 50 | Atggacttgaatttgcgcgacaaggttgccgtcattaccgggggaacggtggggatcggcctggccatt gccgaaggcctggcggcggagggcgtcaatctggtgctggtggggcgcgacaagctgcgggctgac gatgcggcagcgcttgtggccgacaagttcgggattatcgccacggcgatcagcgcggatgtcgcgac tgccgaagggtgcgatgccgtgatcaagggcacggccaaggcctttggtggcgccgatatcctgatca acaatgccgggaccgggtcgaacgagaccattgccgaggccgatgatgccaaatggcaatattattg ggacctgcatgtgatggcggccgtgcggctggcgcgggggctggtgccgggaatgaaaaagcgggg cggcggcgtcgtgctgcacaatgcctcgatctgcgcggtgcagccgctctggtatgagccgatctacaa caccaccaaggccgccttgatgatgttttccaagacgctggccaatgaggtggtgggcgacaatattcg cgtcaataccatcaatcccgggctggtgctgacgcccgattgggtcaagaccgccaagcagattgccg gcgaagatggctggcaggcgcatctgcaaggggtggccgacgaggcgggcggcatgaagcgcttc gcgacgcccgaggaattggccaatttcttcgtcttcatgtgttcggacagagccagctattccaccggctc gacctattttgtcgatggcggctggctcaagacggtttga |
| | DNA sequenc e encoding SEQ ID NO. 5 | |
| 51 | SEQ ID NO. 51 | Atggaactcggactctattccttcgccgaaaacacgcccgatcccctgaacggccgccgcctgcaaag cccggccgaacgtctcaaggacctgctggaagaaatcgaactggccgaccagctcggcctatcctttta cggcctgggcgaacaccaccgccccgactacaccgcttccgcgcccgtcaccatcctggccgccgcc gccgcgcgcaccaagtcgatccgcctctctaccgcggtcaccgtgctcagctcggaagaccccatccg cgtctatcagcaattcgccacgctcgataatctcagcaatggccgcgccgagatcatggccggccgcg gctccttcatcgagagcttccccctcttcgggctcgacctcaacgattacgacgcgctgttcgaggaaaa gctcgaaatgctgctcaaaatccgcgaaggcggcaaggtcacctggcccggcagcacccataccaa gcccatccccggcattgccatctatcccatcccggtccagcagcccctgccgctctggatcgccgtcggc ggcacgccaaattccgttgcccgcgcggcctattacggcctgcccctgatgatcgccatcatcggcggtc agccgcgccagtttgcgccgctggtcgatttctaccgcgaaaccgctgaaaaggtcggccgcgacccc aaatcgctgcccgtcggcatcagctcgcacggcttcatcgccgccgacagccaggacgcccgcgaca |
| | DNA sequenc e encoding SEQ ID NO. 6 | |
| | | tcgccttccccgcccatagcgaggccatgagccgcatcggcaaggaacgcggttggccgcccaccac ccgcgcccaattcgacgccggcgccacgcccaacggcgcctatttcatggggtctccgcaggaagtca tcgacaagattctgatgcagcacgaatggttcaaacacgaccgcttcggcctgcaattcagcgtcggca ccctgccccacgacaaggtcatgaaagccatcgagctttacggcaccgtggtcaagccggcggtgga caaggcgctaggctaa |
| 52 | SEQ ID NO. 52 | AtgAgcactgaaatcgtcaccgatgtcgttgtgattggcgcgggcccctgcgggctgtttgcggccttcg aactcgggctgctcgacctcaaatgccatttcatcgacattctggaccgccccggcgggcagtgcgccg agctttatcccgaaaagccgatctacgatattcccggcttcccggtggtgaccggccaggatctgaccga caatctgatggcccagatcgcgcccttcgcccccgaattccacttcaaccgcatggtcaattcgatcgag aagcaggaagacggctcgtttaagctcgaaaccgacgccgacgagattttccacgcgaaggtcgtggt gatcgcggccggcggcggctcgttccagcccaagcgcccgccggtcgacaatatcgaagcctatgaa aacaagtcggttttctattcggtccgcaagatggacgatttccgtgaccaggacgtggtgatcgtcggcg gcggcgattccgccctcgactggacgctcaacctgcagcccatcgtgcgctcgctgaccctggtacacc gccgcgatgccttcaaggccgcgcccgcttcggtcaacaagatgaaggaaatggtcgccgagggtaa gatcaatttcctgctcggccaggtggccaagctcgatggcgaaaacggccagatcaaccacgtgcatc tgaccaccgatgccggcgatctctcggtgcccgccacccgcctgctgcccttcttcggcctcaccatgaa gctgggtccggtggccgattggggccttgagctcaatgacaatacgatcaaggtcgataccgaaaagtt cgagacctcggtgcccggcattttcgccattggcgacatcaactggtatccgggcaagctcaagctgatc ctctccggcttccacgaggctgccctcatggcccaggccgccaagaagattgtgtcgcccgacgagcg cgtggtgttccaatacaccaccagctccaccaagctgcagaagaagctgggcgtcgcctag |
| | DNA sequenc e encoding SEQ ID NO. 7 | |
| 53 | SEQ ID NO. 53 | atggactatcgctatctcgggcgctcgggcctgaaagtctcgagcatgaccatgggcaccatgacctttg gcggcagcgaaaagatcggcaataccagccgacaggacgcggcgcggcaggtggatatgtgtctcg atgcgggcatcaacctctatgacagcgccaatgtctataatgcgggggtgtccgaggaaatcctgggcg acattctcgccgaaaacgggcggcggcagaaagcgctgatcgccaccaaggtacgcttcaagatgg gcgatggccccaacgaggccgggctcagccgacaccatatcatcgagcagtgcaaggcgagcctca aacgcctcaagaccgatgtgatcgacctctatcaggtgcatgaatgggatgggcagacccccatcgag gaaacgatggaagcgctcgacattctcgtgcgccacggccatgtgcgctatatcgggtgctccaattatt cgggctggcatatttccaaggcccttgccgccgccgaccagcggcacaatgcgcgctttgtgtcccagc agatccattattcgctgcatagccgcgacgccgaatatgagctggtgccgatttcccaggaccaggggc tgggcattctcgtctggtcgccgctggcgggcgggttgctttcgggcaagtatcgccgggacagcgcgc cggacagcgggcggcatgtcggcgggttccgcgagccgccggtctatgactgggacaagctctattcg gtcatcgacgtgctggtggcggtggccgaggagcgtggcgtttcgggggcgcaggtggcgctgagctg gctgatgggacgcccgggcgtcacttccgtcatcatcggcggacgtaacgaaaagcagtttgccgaca atatcgcagcggcccagctcgtgctcagtgacgaggaacgcgcccggctcgatgcggccagccgac cgccgttgctctatccctactggcaccagtccttcaccgcgcaggatcggctgggcaagatcgacatgg atttgatcggtccttatgccgaggagttcaagcgtggctga |
| | DNA sequenc e encoding SEQ ID NO. 8 | |
| 54 | SEQ ID NO. 54 | atggaataccgcttgctcgggcgctcgggactgaaggtctcgacccttaccatgggcacgatgacgttc ggggggaacccgaaggtcggcaataccagcctcaaggacgcgcagcgccagatcgacatgtgtctg gaggcgggcatcaacatgctcgacacggccaatgtctatacgcggggcgtgtcggaaagcattatcgg ggaggcactgaccgatgggcggcgccagcggacgctgctggcgaccaaggtgcgcttcccgatgga cgacaccgaccccaacgagcggggactgtcgcgctaccacatcgtcaaccagtgcgaggcaagcct caagcggctcaagaccgatgtgatcgacctctaccaggtgcatgaatgggacgggcagaccccgctc gaggagaccatggaagcgctcgacatgctcgtgcgccacggcaaggtccgctatatcggctgctcga actattcgggctggcacatcatgaaggcgctcggcgtttccgagaaggacgcccggcagcgcttcgtct cccagcagatccactacacgctgttttcgcgcgaggcggagaacgagctggtgccgatcagcctcgac caggggctgggcattctcatctggagcccgctggcgggcggcctgctttcgggcaagtatcgccgcggc cagacggcgccggaaggggcgcgccatgccgggggcttcaaggagccgccggtgcatgactggga gcggctctacgatatcgtggaagtgctggtttcgatcgccgaggtgcgcggggtttcgggcgcgcagatc gcgctggcctggctgctggggcgcccgggggtgacctcggtgatcatcggcgggcgcacggaagac cagttcagggataacctggcggcggccgaactcaagctgaccgagcatgagcgcaagcggctagac gacgtgtcgctgctgccgctgcaatatccctactggcaccagagctttacggcctcggaccggctgggg ccggcggacctggcgctgcatgcaccccatctcgaaaaatag |
| | DNA sequenc e encoding SEQ ID NO. 9 | |
| 55 | SEQ ID | atggactaccgctatctcggccggtcgggcctcaaggtttcggtgctgagcgtcgggacggtgacgttcg |
| | NO. 55 | gcggcgacgggctgtggggcgatacggacgtcaccggcgccgcccggcagatcgacatgtgcctcg atgcaggcatcaacctcatcgataccgccaatgtctacaacaagggcgtttcggaagagatcaccggc gaggtgctgcgcgacggcggtcggcggcagcgcgcgctgatcgccaccaaggtgcgctttccgttcgg cgacggtcccaatgaccgcgggctgtcgcgccaccacatcatcgcccagtgcgagggcagcctcaa acgcctccagaccgacgtcatcgatctctaccaggtccatcagtgggacgggcagacgcccctcgag gagacgatggaagccctcgataccctggtgcggcaggggaaggtccgctacatcggctgctcgaact attcgggctggcacctgatgaaggcgctcgccgtcgccgacagccgcggctaccagcggttcgtcgcc cagcagatccactacaccctctattcgcgcgaggccgaatacgagctgcagccgatcaccgtcgacc aggggctcggcatactcgtctggagcccgctcgccggcgggtggctctcgggcaagtacacgcgcgc cagccagcccgaggacggccgacacgtgcgcggtttccgcgagccgccggtgcccgatcccgagcg gctctacgacatcgtcgatgtcatcaatgcggttgccgccgagcggggcgtttcgggcgcggtcgtttcg ctggcttgggctttgcagcgcaagggcgtctcgtcggtgatcgtgggcgcgcgcaccgacgcgcagttc gccgataacctcaaggccgccgatctaaagctcagcgacgacgaggtcgcccggctcgacgccgtc agccagcgtccgcttccctatccctattggcaccagtccttcaccgcccaggaccgcttgagcgaagcc gatctcgtgctgctcgccccttatctcgaggagtacaagcgtggctga |
| | DNA sequenc e encoding SEQ ID NO. 10 | |
| 56 | SEQ ID NO. 56 | atggactatcgattgctcggccgttccggcctcaaggtgtcgtcgctgtcgatcggcacggcgacgttcg gcggcgacgggttgtggggcgacaccaacgtcaaggaggcgcagcgccagatcgacctctgcctcg accacggcgtcaacctgatcgacaccgccaatgtctattcgggcagcgtgtcggagacgatcatcggc gaggcgctgaccgatggccggcgcgaccgcttgctgatcgccaccaaggtgcgcttccccaggggca agggaccgaacgatcgcgggctcagccgatggcacatcatccgtgaggcggaagcctcgctgaagc ggatgaagaccgacgtcatcgacctgtaccaggtgcatgagtgggatgggcagacgccgctcgagg agacgctggaagcactcgacaccctcgtccgctccggcaaggtccgctatatcggctgctcgaactatt cggcctggcacctgatgaaggcgctcgcggtttccaacgagcgcaactaccagcgcttcgtttcccagc agatccactacacgctgcactcgcgcgaagccgagttcgagctgatcccggccggcctcgaccaggg tgtctcggtgctggtgtggtcgccgctcgccggcgggtggctctcgggcaagtacacccgcgattccaag ccgagcgacggccggcaggtgcgcggcttcaaggagccgccgatctacgatcccgagcatctgtgga agatcgtcgacgtggtcaacgacgtggcgggcgcgcgtggcgtttccggcgcgcaggtgtcgcttgcct ggttgctgtcgaagccgatcgtcgcttcggtgatcgtcggcggccgcaatatcggacagttcgaagaca acctcaaagccgcggaactgactctcaccaccgaggagctgcagaagctcgacgcggtcagccagc cgaacctgccctacccgtactggcaccagagctttaccgcgcaggaccggctcggtgagcccgacctg gcgctgcaccgcccctatctcgacaagtga |
| | DNA sequenc e encoding SEQ ID NO. 11 | |
| 57 | SEQ ID NO. 57 | atggactatcgattgctcggccgttccggcctcaaggtctcgtcgctgtcgatcggcacggcgacgttcgg cggcgatgggttgtggggcgataccaacgtcaaggaggcgcagcgccagatcgacctctgcctcgac catggcgtcaacctgatcgacaccgccaacgtctattcggggagcgtctcggaaacgatcatcggcga ggcgctgaccgacgggcggcgtgatcggctgctgatcgccaccaaggtgcgcttcccacgcggcaag gggcccaatgatcgcggcctcagccgctggcacatcatccgcgaggcggaagcgtcgctcaaacgg atgaagaccgacgtcatcgacctctaccaggtgcatgagtgggacgggcagacgccgctcgaggaa acgctggaggcgctcgatacgctcgtccgctcgggcaaggtccgctatatcggctgctcgaactattcgg cctggcacctgatgaaggcgctcgccacctcgaacgagcgcaactaccagcgcttcgtctcccagcag atccactacacgctgcattcgcgtgaggccgagttcgagctgatcccggccgggctcgaccagggcgtt tcggtgctggtgtggtcgccgctggccggcgggtggctgtcgggcaagtacacccgcaactcgaagcc gagcgatggccggcaggtgcgcggcttcaaggagccgccgatctatgatccggagcacctgtggaag atcgtcgatgtggtcaacgaagtggccagcgcgcatggcgtctccggcgcgcaggtgtcgctggcctg gctgttgagtaagccgatcgttgcctcggtgatcgtcggcggccgcaatatcgagcagttcgaggacaa cctgaaagccgccgagctgcagctcaccgccgaggaactgggcagactcgacgcggtcagccagc cgaacctcccctacccctactggcaccagaactttaccgcgcaggatcgcctcagcgagcctgacctg gcgctgcaccggccctatctcgacaaatga |
| | DNA sequenc e encoding SEQ ID NO. 12 | |
| 58 | SEQ ID NO. 58 | atggaatatcgactgcttggccgctccggcctcaaagtctccaccctcacgatgggcacgatgaccttcg gcggccagggcaagttcgcctcggtcggcaactccggcgtcgacgaagcacgccggctgatcgacat gtgcatcgatgccggcgtcaatctcatcgatacggcgaacgtctactccgatggccgttcggaagaaat catcggcgaagtgctcggcggcaagcgtccgaaggacgtactgatcgcgaccaaggcacgctttccg atgggcgacggccccaacaacggcggcctctctcgctatcacctgattcgcgagtgcgaagcgagcct gaagcgcctgcgcacggacgtgatcgatctctaccaggttcaccagtgggatggcctgaccccgctgg aagaaaccctggaagcgctcgacaccctggttcgcgacggcaaggtgcgttacctgggctgctccaac |
| | DNA sequenc e encoding | |
| | SEQ ID NO. 13 | tattccgcgtggcacctgatgaaggcgctcggcatcagtgagcgcgagcatcgccagcggttcgtcag ccaacagatccattacacgctggaggcgcgcgaggccgaatacgagctgctgccgatctcgatcgac cagggcctcggcgtcctcgtctggagtccgcttgccggtggcctgctttcgggcaagcaccgtcgcgatg gcaaggcaccggagggctcacgtcagctggagggctggaccgagccgccgatccgcgacgaaga gcgcctttggcgcatcgtcgacgtgctggtggccatcgccgaagcgcgcggcgtgtcgggtgcacagg tcgcgctggcctggctgctcgagcgtcccgcggtgacctccctggtcatcggcggtcgtaccgaggcgc agttcaaggacaacctcgccgccgccgagctgaagctcacggccgaagagcgtcagcggctggacg aggtcagtgcgccgccggtgctctacccgtactggcaccagctgatgacggcgtcggaccgcctgggt ccggcggacatgccattgctggcgccgcatctgaagtaa |
| 59 | SEQ ID NO. 59 | atggaataccgactgcttggccgctccggcctcaaagtctccaccctcacgatgggcacgatgaccttc ggcggccagggcaaattcgcctcggtcggtagctccggcgtcgacgaagcgcgccggctgatcgaca tgtgcatcgacgccggcgtgaatctcatcgacacggcgaacgtctattccgacggtcgttcggaagaga ttatcggtgaagtgctcggcggcaagcgtccgaaggacgtcctgatcgcgaccaaggcgcgctttccc atgggtgatggcccgaaccatggtggcctgtcgcgctatcacctgattcgcgaatgcgaagccagcctg aagcggctgcgcacggacgtgatcgatctctaccaggttcaccagtgggatggcctgacgcctctgga ggaaaccctggaagcgctcgacaccctcgttcgcgacggcaaggtgcgttacctgggttgctccaacta ttccgcctggcacctgatgaaggcgctcggcgtgagcgagcgtgagcaccgccagcgtttcatcagcc agcagatccattacacgctggaagcgcgcgaagccgaatacgaactgctgccgatttcgatcgacca gggcctcggcgtgctggtgtggagtccgctcgccggcgggctgctttcgggcaagcaccgtcgcgatac caaggccccggaaggctcgcgccagctggccggctggaccgaaccgccgatccgcgacgaagag cgcctgtggcgcgtcgtcgacgtactggtttcaatcgcccaatcgcgtggcgtgtcgggagcgcaggtc gcgctggcctggttgctcgagcgtcctgcggtcacctcgctggtgatcggtggtcgtaccgaggcacagt tcaaggacaacctggcggccgccgaactgaagctcacggccgaagagcgtcagcgtctggatgaag tcagcgcgccgccggtgctctatccgtactggcaccagttgatgaccgcatcggatcgcctgggtccgg ccgacatgcccctgctggcgccgcacctcaaataa |
| | DNA sequenc e encoding SEQ ID NO. 14 | |
| 60 | SEQ ID NO. 60 | atggaataccgctatctcggccgctcaggcttgaaggtgtcgacattgaccatgggcaccatgacctttg gcggctcggaaaaggttggggcgacggcgcaagacgatgcgacgcgccagatcgacctgtgcctcg atgcagggatcaatctgctcgacacggccaatgtctacaatgccggggtctccgaggaaatgattggcg tggcgctggccgagaatggtcgccgccagaaagcactcgtagccaccaaggtccgcttcaagatggg cgatgggcccaacgagatcggcctcagccgtcaccatattattgcccaggccgaagccagccttaagc gcttgaaaacagacgttattgatctctatcaggtccacgaatgggacggcatgacgcccatcgaggag acgatggaagcgctcgataccctgatcaagcagggcaaagtccgctatatcggctgctcaaattattcg ggctggcatatcatgaaggcgctgcaggccgccgacaaaaaccacggccagcgcttcatcagccag cagatccactacacgctgcattcgcgcgaagccgaatatgaactgatcccgatcactggggatcaagg ccttggcgtcctcatctggtcgccgctggccggtggtttgctctcgggcaaataccgtcgcaacggtggcc ctgacagcgggcgccatgtcggcggcttccgcgagccgccggtttatgactgggagaagctctacgac atcgtcgatgtgatcgtggatattgctgatgaccgcggcgtgtcgggtgcacaggttgcgctggcatggtc gctggggcggccgggtgtaacctcggtgatcatcggtggccgcaatgatgcccagttcaaagacaatct ggcggcagccgagttgaagctgacggcggaagagcgcgcgcgacttgacgcggtcagccgtccac cgctgctctatccttattggcatcagacgtttacggcgcaggaccggttaggccctgtcgatcgaaacctg atcgaaccctatgcagaggaattgaaccgtggctga |
| | DNA sequenc e encoding SEQ ID NO. 4 | |
| 61 | SEQ ID NO. 61 | MRFEYLRQNVVGLALSTALIASLSGPAFAQHDANAAAEPSKAGQSAIENF QPVTADDLAGKNPANWPILRGNYQGWGYSPLDQINKDNVGDLQLVWSR TMEPGSNEGAAIAYNGVIFLGNTNDVIQAIDGKTGSLIWEYRRKLPSASKFI NSLGAAKRSIALFGDKVYFVSWDNFVVALDAKTGKLAWETNRGQGVEEG VANSSGPIVVDGVVIAGSTCQFSGFGCYVTGTDAESGEELWRNTFIPRPG EEGDDTWGGAPYENRWMTGAWGQITYDPELDLVYYGSTGAGPASEVQ RGTEGGTLAGTNTRFAVKPKTGEVVWKHQTLPRDNWDSECTFEMMVVS TSVNPDAKADGMMSVGANVPRGETRKVLTGVPCKTGVAWQFDAKTGDY FWSKATVEQNSIASIDDTGLVTVNEDMILKEPGKTYNYCPTFLGGRDWPS AGYLPKSNLYVIPLSNACYDVMARTTEATPADVYNTDATLVLAPGKTNMG RVDAIDLATGETKWSYETRAALYDPVLTTGGDLVFVGGIDRDFRALDAES GKEVWSTRLPGAVSGYTTSYSIDGRQYVAVVSGGSLGGPTFGPTTPDVD SASGANGIYVFALPEKK |
| | SEQ ID NO. 1 of WO2016 /154640 | |
| 62 | SEQ ID | MKKRTSILLASVAMLGMGSTAFAQVDINALPAVTDAILANPDAGDWPSYG |
| | NO. 62 | RDITNYRFSPLDQVNKDNVGQLTLAWARALEPGNLQSAPLEFGGVLFTAA PGDVVQAMDAATGQLIWEYRRQLPDRATLNSLGENKRGIALYEDKIYVAT WDNFIVALDAKTGQVAWESDRGGGADLISNTTGPIVANGVVVAGSTCQF SEFGCYVTGHDAATGEELWRNNFIPKKGEEGDDTWGDSTEDQRWMTG AWGQMTYDPELDLVYYGSTGAGPAAEFQRNTVGGTLFGSNTRFAVKPK TGEIVWRHQVLPRDNWDQECTYEMVPVDIDSAPAADMEGLLALGTAAPG KKRVLTGVPCKTGVMWQFDAQTGEFIYARDTVQQTLIESVDNTGLVTVNE AAIPTEVDVATPMCPTYLGGRDWSPTAFNPTSKVMFVPLTNMCADVTVL DQEPTGLDVYNTELTYKMPEGVTDAGRIDAINVETGKTLWSWTQQTPQY ASITATAGGLIFTGGADRRFKAIDQETGELVWSVTLGSRATGHPISYEVDG RQYIAIPAGGPGYATDLITASGSTVDVVSGSNMLYVFALPEQKK |
| | SEQ ID NO. 7 of WO2019 /046954 | |
| 63 | SEQ ID NO. 63 | MDYRYIGRSGLRVSVLTMGTMTFGGSDKVGHTAQADATRQIDLCLDHGI NLLDTANVYNAGVSEEMIGVALSEHGRRQKALIATKVRFRMGNGPNEVG LSKHHIIAQAEASLKRLKTDVIELYQVHEWDGMTPIEETMEALDTLVKQGK VRYVACSNYSGWHIMKALAAADKHHGQRFISQQIHYTLHSREADYELVPI SQDQGLGILIWSPLAGGLLSGKYRRDGGPDAGRHVGGFREPPVYDWDK LYEIIDVIVAIARERDVSGAQVALAWALGRPGVTSAIIGGRSEAQFRDNLAA AELRLTDDERARLDAASRPPLLYPYWHQSFTAQERLDQIDLDLIGPYAEEF KRG |
| 64 | SEQ ID NO. 64 | MDYKYLGRSGLRVSVLTMGTMTFAGSEKVGHTAQADATRQIDLCIDHGIN LLDTANVYNAGVSEEMIGVALSEHGRRQKALIATKVRFRMGNGPNEVGLS RHHLIAQAEASIKRLKTDVIDLYQVHEWDGMTPIEETMEALDTLVKQGKVR YVGCSNYSGWHIMKGLAAADKHHGQRFISQQIHYTLHSREAEYELVPISQ DQGLGILIWSPLAGGLLSGKYRRDGGPDAGRHVGGFRDPPVYDWDKLY EILDVIVAIAREKDVSGAQVALAWALGRPGVTSAIIGGRSEAQFRDNLAAA ELRLTDDERARLDAASRPPLLYPYWHQSFTGQDRLDQVDLDLIGPYAEEF KRG |
| 65 | SEQ ID NO. 65 | MDYKYLGRSGIRVSVLTMGTMTFAGSEKVGHTAQADATRQIDLCLDHGIN LLDTANVYNAGVSEEMIGVALSEHGRRQKALIATKVRFRMGNGPNEVGLS RHHLIAQAEASLKRIKTDVIDLYQVHEWDGMTPIEDTMEALDTLVKQAKVR YVGCSNYSGWHIMKALAGADKHHGQRFISQQIHYTLHSREAEYELVPISQ DQGLGILIWSPLAGGLLSGKYRRDGGPDAGRHVGGFRDPPVYDWDRLY DIIDVIVAIAREKDVSGAQVALAYALGRPGVTSAIIGGRSEAQFRDNLGAAE LKLTDDERARLDAASRPPLLYPYWHNSFTGQDRLDQVDLDLIGPYAEEFK RG |
| 66 | SEQ ID NO. 66 | MDYRFLGRSGIRVSVLTMGTMTFGGTEKVGHTAQADATRQLDICLDHGIN LLDTANVYNAGVSEEMIGVALSEHGKRQKALIATKVRFRMANGPNEVGLS RHHIIAQAEASLKRIKTDVIDLYQVHEWDGMTPIDETLEALDTLVKQGKVR YVGCSNYSGWHIMKGLAAADKHHGQRFISQQIHYTLHSREGEYELVPISQ DQGLAILIWSPLAGGLLSGKYKRDGGPDAGRHVGGFREPPLYDWDRLYD IIDVIVAIARERDVSGAQVALAFALGRPGVTSAIIGGRSEANFRDNLGAAEL RLSDDERARLDAASRPPLLYPYWHQSFTANDRLDQLDLDLIGPYADEFKR G |
| 67 | SEQ ID NO. 67 | MDYKYIGRSGIRVSVLTMGTMTFAGSERVGHTGQAEATRQLDICLDHGIN LLDTANVYNAGVSEEMLGVGLSEHGRRQKALIATKVRFRMANGPNEVGL SRHHIIAQAEASIKRLKTDVIELYQVHEWDGMTPIDETMEALDTLVKQAKV RYVACSNYSGWHIMKGLAGADKHHGQRFISQQIHYTLHSRDGEYELVPIS QDQGLAILIWSPLAGGLLSGKYRRDGGPDAGRHVGGFREPPVYDWDKLY EILDVIVAIARERDVSGAQVALAWALGRPGVTSAIIGGRSEAQFRDNIGAA DLKLSDEERARLDAASRPPLLYPYWHNSFTGNDRLDQLDLDLLGPYADEF KRG |
| 68 | SEQ ID NO. 68 | MDYKYLGRSGIRVSVLTMGTMTFAGSERVGHTGQAEATRQLDICLDHGIN LLDTANVYNAGVSEEMLGVGLSEHGRRQKALIATKVRFRMANGPNEVGL SRHHIIAQAEASLKRLKTDVIELYQVHEWDGMTPIEDTMEALDTLVKQAKV RYVGCSNYSGWHIMKGLAGADKHHGQRFISQQIHYTLHSRDAEYELVPIS |
| | | QDQGLGILIWSPLAGGLLSGKYRRDGGPDAGRHVGGFREPPVYDWDKL YELIDVIVAIARERDVSGAQVALAWALGRPGVTSAIIGGRSEAQFRDNIAG AELKLSDDERARLDAASRPPLLYPYWHQSFTGNDRLDQIDLDLLGPYADE FKRG |
| 69 | SEQ ID NO. 69 | MDYKYIGRSGIRVSVLTMGTMTFGGSEKVGHTGQADATRQIDICLDHGIN LLDTANVYNAGVSEEMLGVALSEHGRRQKALIATKVRFRMGNGPNEVGL SRHHIIAQAEASLKRLKTDVIELYQVHEWDGMTPIDETMEALDTLVKQAKV RYVACSNYSGWHIMKGLAAADKHHGQRFISQQIHYTLHSREGEYELVPIS QDQGLAILIWSPLAGGLLSGKYRRDGGPDAGRHVGGFREPPVYDWDKLY DILDVIVAIARERDVSGAQVALAWALGRPGVTSAIIGGRSEAQFRDNIGAA ELKLTDEERARLDAASRPPLLYPYWHNSFTGQDRLDQLDLDLLGPYADDF KRG |
| 70 | SEQ ID NO. 70 | MDYKYLGRSGLRVSVLTMGTMTFGGSERVGHTAQAEATRQIDLCLDHGI NLLDTANVYNAGVSEEMLGVALSEHGRRQKALIATKVRFRMGNGPNEVG LSRHHIIAQAEASLKRLKTDVIELYQVHEWDGMTPIEDTMEALDTLVKQGK VRYVACSNYSGWHIMKGLAAADKHHGQRFISQQIHYTLHSREAEYELVPI SQDQGLGILIWSPLAGGLLSGKYRRDGGPDAGRHVGGFREPPVYDWDK LYEILDVIVAIARERDVSGAQVALAWALGRPGVTSAIIGGRSEAQFRDNIGA ADLKLSDEERARLDAASRPPLLYPYWHQSFTANDRLDQLDLDLIGPYAED FKRG |
| 71 | SEQ ID NO. 71 | MDYRYLGRSGLRVSVLTMGTMTFAGSEKVGHTGQADATRQIDLCLDHGI NLLDTANVYNAGVSEEMIGVGLSEHGRRQKALIATKVRFRMANGPNEVG LSRHHIIAQAEASIKRLKTDVIDLYQVHEWDGMTPIDETMEALDTLVKQGK VRYVACSNYSGWHIMKGLAGADKHHGQRFISQQIHYTLHSREGEYELVPI SQDQGLAILIWSPLAGGLLSGKYRRDGGPDAGRHVGGFREPPVYDWDKL YDILDVIVAIARERDVSGAQVALAWALGRPGVTSAIIGGRSEAQFRDNIAAA ELKLSDDERARLDAASRPPLLYPYWHQSFTGQDRLDQIDLDLLGPYAEEF KRG |
| 72 | SEQ ID NO. 72 | MDYRYIGRSGLRVSVLTMGTMTFGGSEKVGHTGQADATRQIDICLDHGIN LLDTANVYNAGVSEEMLGVGLSEHGRRQKALIATKVRFRMANGPNEVGL SRHHIIAQAEASIKRLKTDVIDLYQVHEWDGMTPIEDTMEALDTLVKQGKV RYVACSNYSGWHIMKALAGADKHHGQRFISQQIHYTLHSREAEYELVPIS QDQGLGILIWSPLAGGLLSGKYRRDGGPDAGRHVGGFREPPVYDWDKL YDLIDVIVAIARERDVSGAQVALAWALGRPGVTSAIIGGRSEAQFRDNIGA ADLKLTDDERARLDAASRPPLLYPYWHNSFTANDRLDQIDLDLIGPYAEDF KRG |
| 73 | SEQ ID NO. 73 | MDYRYLGRSGLRVSVLTMGTMTFGGSEKVGHTGQADATRQLDLCLDHGI NLLDTANVYNAGVSEEMIGVGLSEHGRRQKALIATKVRFRMGNGPNEVG LSRHHIIAQAEASLKRLKTDVIELYQVHEWDGMTPIEDTMEALDTLVKQAK VRYVACSNYSGWHIMKALAGADKHHGQRFISQQIHYTLHSREAEYELVPI SQDQGLAILIWSPLAGGLLSGKYRRDGGPDAGRHVGGFREPPVYDWDKL YEIIDVIVAIARERDVSGAQVALAWALGRPGVTSAIIGGRSEAQFRDNLAAA ELKLTDEERARLDAASRPPLLYPYWHQSFTAQDRLDQLDLDLIGPYAEDF KRG |
| 74 | SEQ ID NO. 74 | MDYRYIGRSGIRVSVLTMGTMTFGGSERVGHTAQADATRQLDLCLDHGI NLLDTANVYNAGVSEEMIGVALSEHGRRQKALIATKVRFRMANGPNEVGL SRHHIIAQAEASIKRLKTDVIELYQVHEWDGMTPIEETMEALDTLVKQAKV RYVGCSNYSGWHIMKALAGADKHHGQRFISQQIHYTLHSREGEYELVPIS QDQGLGILIWSPLAGGLLSGKYRRDGGPDAGRHVGGFREPPVYDWDKL YDLIDVIVAIARERDVSGAQVALAWALGRPGVTSAIIGGRSEAQFRDNLGA AELKLTDEERARLDAASRPPLLYPYWHQSFTGNDRLDQIDLDLLGPYADE FKRG |
| 75 | SEQ ID NO. 75 | MDYRYIGRSGIRVSVLTMGTMTFAGSERVGHTAQADATRQLDICLDHGIN LLDTANVYNAGVSEEMLGVGLSEHGRRQKALIATKVRFRMANGPNEVGL SRHHIIAQAEASLKRLKTDVIELYQVHEWDGMTPIDETMEALDTLVKQAKV |
| | | RYVACSNYSGWHIMKGLAGADKHHGQRFISQQIHYTLHSRDGEYELVPIS QDQGLAILIWSPLAGGLLSGKYRRDGGPDAGRHVGGFREPPVYDWDKLY ELIDVIVAIARERDVSGAQVALAWALGRPGVTSAIIGGRSEAQFRDNIGAA DLKLSDDERARLDAASRPPLLYPYWHNSFTGNDRLDQLDLDLLGPYADE FKRG |
| 76 | SEQ ID NO. 76 | MDYRYLGRSGIRVSTLTMGTMTFAGSEKVGHTGQAEATRQLDICLDHGIN LLDTANVYNAGVSEEMLGVGLSENGRRQKALIATKVRFRMANGPNEVGL SRHHIIAQAEASIKRLKTDVIELYQVHEWDGMTPIDETMEALDTLVKQAKV RYIACSNYSGWHIMKGLAGADKSHGQRFISQQIHYTLHSREAEYELVPISQ DQGLAILIWSPLAGGLLSGKYRRDGGPDSGRHVGGSREPPVPDWDKLYE ILDVIVAIAEERGVSGAQVALAWALGRPGVTSAIIGGRSEAQFRDNLGAAD LKLSDEERQRLDAASRPPLLYPYWHNSFTGNDRLDPADLDLLGTYADEFK RG |
| 77 | SEQ ID NO. 77 | MDYRYLGRSGIRVSTLTMGTMTFAGSEKVGHTPQAEATRQLDLCLDHGI NLLDTANVYNAGVSEEMIGVALAENGRRQKALIATKVRFRMGNGPNEVG LSRHHIIAQAEASLKRLKTDVIDLYQVHEWDGMTPIEDTMEALDTLVKQAK VRYIGCSNYSGWHIMKALAAADKSHGQRFISQQIHYTLHSREAEYELVPIS QDQGLGILIWSPLAGGLLSGKYRRDGGPESGRHVGGSREPPVPDWDKL YDIIDVIVAIAEERGVSGAQVALAWALGRPGVTSVIIGGRSEAQFRDNLAAA DLKLSDEERQRLDAASRPPLLYPYWHQTFTGNDRLGPADQDLLSTYVED FKRG |
| 78 | SEQ ID NO. 78 | MDYRYLGRSGIRVSTLTMGTMTFGGSEKVGHTGQADATRQIDICLDHGIN LLDTANVYNAGVSEEMLGVALSENGRRQKALIATKVRFRMGNGPNEVGL SRHHIIAQAEASLKRLKTDVIELYQVHEWDGMTPIDETMEALDTLVKQAKV RYIACSNYSGWHIMKGLAAADKSHGQRFISQQIHYTLHSREAEYELVPISQ DQGLAILIWSPLAGGLLSGKYRRDGGPDSGRHVGGSREPPVPDWDKLYD ILDVIVAIAEERGVSGAQVALAWALGRPGVTSAIIGGRSEAQFRDNLGAAD LKLTDEERQRLDAASRPPLLYPYWHNSFTGQDRLGPADQDLLLSTYVED FKRG |
| 79 | SEQ ID NO. 79 | MDYRYLGRSGLRVSTLTMGTMTFGGSEKVGHTAQAEATRQIDLCLDHGI NLLDTANVYNAGVSEEMIGVALAENGRRQKALIATKVRFRMGNGPNEVG LSRHHIIAQAEASLKRLKTDVIELYQVHEWDGMTPIEDTMEALDTLVKQGK VRYIACSNYSGWHIMKGLAAADKSHGQRFISQQIHYTLHSREAEYELVPIS QDQGLGILIWSPLAGGLLSGKYRRDGGPDSGRHVGGSREPPVPDWDKL YEILDVIVAIAEERGVSGAQVALAWALGRPGVTSAIIGGRSEAQFRDNLGA ADLKLSDEERQRLDAASRPPLLYPYWHQSFTANDRLGPADQDLIGTYAE DFKRG |
| 80 | SEQ ID NO. 80 | MDYRYLGRSGLRVSTLTMGTMTFAGSEKVGHTGQADATRQIDLCLDHGI NLLDTANVYNAGVSEEMIGVGLSENGRRQKALIATKVRFRMANGPNEVG LSRHHIIAQAEASIKRLKTDVIDLYQVHEWDGMTPIDETMEALDTLVKQGK VRYIACSNYSGWHIMKGLAGADKSHGQRFISQQIHYTLHSREAEYELVPIS QDQGLAILIWSPLAGGLLSGKYRRDGGPDSGRHVGGSREPPVPDWDKL YDILDVIVAIAEERGVSGAQVALAWALGRPGVTSAIIGGRSEAQFRDNLAA ADLKLSDDERQRLDAASRPPLLYPYWHQTFTGQDRLDPADLDLLGTYAE EFKRG |
| 81 | SEQ ID NO. 81 | MDYRYLGRSGLRVSTLTMGTMTFGGSEKVGHTGQADATRQIDICLDHGI NLLDTANVYNAGVSEEMLGVGLSENGRRQKALIATKVRFRMANGPNEVG LSRHHIIAQAEASIKRLKTDVIDLYQVHEWDGMTPIEDTMEALDTLVKQGK VRYIACSNYSGWHIMKALAGADKSHGQRFISQQIHYTLHSREAEYELVPIS QDQGLGILIWSPLAGGLLSGKYRRDGGPDSGRHVGGSREPPVPDWDKL YDIIDVIVAIAEERGVSGAQVALAWALGRPGVTSAIIGGRSEAQFRDNLGA ADLKLTDEERQRLDAASRPPLLYPYWHQTFTANDRLDPADLDLLSTYVED FKRG |
| 82 | SEQ ID NO. 82 | MDYRYLGRSGLRVSTLTMGTMTFGGSEKVGHTGQADATRQLDLCLDHGI NLLDTANVYNAGVSEEMIGVGLSENGRRQKALIATKVRFRMGNGPNEVG |
| | | LSRHHIIAQAEASLKRLKTDVIELYQVHEWDGMTPIEDTMEALDTLVKQAK VRYIACSNYSGWHIMKALAGADKSHGQRFISQQIHYTLHSREAEYELVPIS QDQGLAILIWSPLAGGLLSGKYRRDGGPDSGRHVGGSREPPVPDWDKL YEIIDVIVAIAEERGVSGAQVALAWALGRPGVTSAIIGGRSEAQFRDNLAAA DLKLTDEERQRLDAASRPPLLYPYWHQTFTAQDRLGPADQDLIGTYAEDF KRG |
| 83 | SEQ ID NO. 83 | MDYRYLGRSGIRVSTLTMGTMTFGGSEKVGHTAQADATRQLDLCLDHGI NLLDTANVYNAGVSEEMIGVALSENGRRQKALIATKVRFRMANGPNEVGL SRHHIIAQAEASIKRLKTDVIELYQVHEWDGMTPIEETMEALDTLVKQAKV RYIGCSNYSGWHIMKALAGADKSHGQRFISQQIHYTLHSREAEYELVPISQ DQGLGILIWSPLAGGLLSGKYRRDGGPDSGRHVGGSREPPVPDWDKLY DIIDVIVAIAEERGVSGAQVALAWALGRPGVTSAIIGGRSEAQFRDNLGAA DLKLTDEERQRLDAASRPPLLYPYWHQSFTGNDRLDPADLDLLGTYADE FKRG |
| 84 | SEQ ID NO. 84 | MDYRYLGRSGIRVSTLTMGTMTFAGSEKVGHTAQADATRQLDICLDHGIN LLDTANVYNAGVSEEMLGVGLSENGRRQKALIATKVRFRMANGPNEVGL SRHHIIAQAEASLKRLKTDVIELYQVHEWDGMTPIDETMEALDTLVKQAKV RYIACSNYSGWHIMKGLAGADKSHGQRFISQQIHYTLHSREAEYELVPISQ DQGLGILIWSPLAGGLLSGKYRRDGGPDSGRHVGGSREPPVPDWDKLY EIIDVIVAIAEERGVSGAQVALAWALGRPGVTSAIIGGRSEAQFRDNLGAA DLKLSDEERQRLDAASRPPLLYPYWHNSFTGNDRLDPADLDLLGTYADE FKRG |
| 85 | SEQ ID NO. 85 | MDYRYLGRSGLRVSTLTMGTMTFGGSDKVGHTPQADATRQIDICLDHGI NLLDTANVYNAGVSEEMIGVALAEGRRQQKALIATKVRFRGGGGPNEVG LSKHHIIAQAEASLKRLKTDVIELYQVHEWDGMTPIEETMEALDTLVKQGK VRYIACSNYSGWHIMKALAAADKSHGQRFISQQIHYTLHSREADYELVPIS QDQGLGILIWSPLAGGLLSGKYRRDGGPDAGRHVGGSREPPVPDWDKL YEIIDVIVAIAEERGVSGAQVALAWALGRPGVTSAIIGGRSEAQFRDNLAAA ELKLTDDERQRLDAASRPPLLYPYWHQSFTAQERLGPADQDLLSTYVED FKRG |
| 86 | SEQ ID NO. 86 | MDYKYLGRSGLRVSTLTMGTMTFGGSEKVGHTPQADATRQIDLCLDHGI NLLDTANVYNAGVSEEMIGVALAEHGRRQKALIATKVRFRMGDGPNEVG LSRHHIIAQAEASLKRLKTDVIDLYQVHEWDGMTPIEETMEALDTLVKQGK VRYIGCSNYSGWHIMKALAAADKSHGQRFISQQIHYTLHSREAEYELVPIS QDQGLGILIWSPLAGGLLSGKYRRDGGPDAGRHVGGSRDPPVPDWDKL YEILDVIVAIAREKDVSGAQVALAWALGRPGVTSAIIGGRSEAQFRDNLAA AELKLTDDERQRLDAASRPPLLYPYWHQSFTGQDRLDQVDLDLIGPYAEE FKRG |
| 87 | SEQ ID NO. 87 | MDYKYLGRSGIRVSTLTMGTMTFGGSEKVGHTPQADATRQIDLCLDHGIN LLDTANVYNAGVSEEMIGVALAEHGRRQKALIATKVRFRMGDGPNEVGLS RHHIIAQAEASLKRIKTDVIDLYQVHEWDGMTPIEETMEALDTLVKQAKVR YIGCSNYSGWHIMKALAGADKSHGQRFISQQIHYTLHSREAEYELVPISQD QGLGILIWSPLAGGLLSGKYRRDGGPDAGRHVGGSRDPPVPDWDRLYDI IDVIVAIAEERGVSGAQVALAYALGRPGVTSAIIGGRSEAQFRDNLGAAEL KLTDDERQRLDAASRPPLLYPYWHNSFTGQDRLGPADQDLIGPYAEEFK RG |
| 88 | SEQ ID NO. 88 | MDYRYLGRSGIRVSTLTMGTMTFGGSEKVGHTPQADATRQIDICLDHGIN LLDTANVYNAGVSEEMIGVALAEHGRRQKALIATKVRFRMGDGPNEVGLS RHHIIAQAEASLKRIKTDVIDLYQVHEWDGMTPIEETMEALDTLVKQGKVR YIGCSNYSGWHIMKALAAADKSHGQRFISQQIHYTLHSREGEYELVPISQD QGLAILIWSPLAGGLLSGKYKRDGGPDAGRHVGGSREPPLPDWDRLYDII DVIVAIARERDVSGAQVALAFALGRPGVTSAIIGGRSEANFRDNLGAAELK LSDDERQRLDAASRPPLLYPYWHQSFTANDRLDQLDLDLIGPYADEFKR G |
| 89 | SEQ ID | MDYRYIGRSGLKVSAMTMGTMTFGGSERVGHTDLAGAARQVDLCLDAGI |
| | NO. 89 | NLYDTANVYNAGVSEDILGQVLAEHGRRQRALVATKVRFRMGNGPNEAG LSKHHIIAQAEASLKRLRTDAIELYQVHEWDGMTPIEETMEALDTLVRQGK VRYVGCSNYSGWHIMKALMAADKHHGQRFVSQQIHYSLHSRDAEYELVP ISQDQGLGILVWSPLAGGLLSGKYRRTGGPEAGRHVGGFREPPVYDWD KLYELIDVIVAIAAQRGVSGAQVALAWLLGRPGVTSLIIGGRSEAQFRDNLA AAHLRLTSEERARLDAASRPPLLYPYWHQSFTAQDRLSRIDLDLIGPYAEE FKRG |
| 90 | SEQ ID NO. 90 | MDYRYLGRSGLKVSAMTMGTMTFGGSERVGHTDLAGAARQIDLCIDAGI NLLDTANVYNAGVSEDIIGVALAEHGRRQRALVATKVRFKMGNGPNEVGL SRHHIIAQAEASLKRLRTDAIDLYQVHEWDGMTPIEETMEALDTLVKQGKV RYVGCSNYSGWHIMKALMAADKRQGQRFISQQIHYSLHSREAEYELVPIS QDQGLGILIWSPLAGGLLSGKYRRDGGPESGRHVGGFREPPVYDWDKL YEIIDVIVAIARQKGVSGAQVALSWLLERPGVTSLIIGGRSEAQFADNLAAIH LRLTSDERARLDAASRPPLLYPYWHQSFTAQDRLSQIDLDLIGPYAEEFKR G |
| 91 | SEQ ID NO. 91 | MDYRYLGRSGLKVSAMTMGTMTFGGSERVGHTDLAGAARQIDLCLDHGI NLYDTANVYNAGVSEDIIGQALAEHGRRQRALIATKVRFKMGNGPNEAGL SRHHIIAQAEASLKRLRTDAIELYQVHEWDGMTPIEETMEALDTLVKQAKV RYVGCSNYSGWHIMKALAAADKRQGQRFISQQIHYSLHSRDAEYELVPIS QDQGLGILIWSPLAGGLLSGKYRRDGGPESGRHVGGFREPPVYDWDRL YDLIDVIVDIAAQKGVSGAQVALSYALGRPGVTSLIIGGRNEAQFRDNLAAI HLKLTDEERARLDAASRPPLLYPYWHQSFTAQDRLSRIVDLDLEPYAEEF KRG |
| 92 | SEQ ID NO. 92 | MDYRFLGRSGLKVSAMTMGTMTFGGSERVGHTAQAGAARQVDLCLDAG INLLDTANVYNAGVSEDILGVVLAEHGRRQRALVATKVRFKMGNGPNEAG LSRHHIIAQAEASLKRLRTDAIDLYQVHEWDGMTPIDETMEALDTLVRQGK VRYVGCSNYSGWHIMKALMAADKRQGQRFVSQQIHYSLHSRDAEYELVP ISQDQGLGILVWSPLAGGLLSGKYKRDGGPESGRHVGGFREPPLYDWDK LYDLIDVIVAIARQRGVSGAQVALSFLLERPGVTSLIIGGRSEANFADNLAA AHLKLSSEERARLDAASRPPLLYPYWHQSFTAQDRLSQIDLDLIEPYAEEF KRG |
| 93 | SEQ ID NO. 93 | MDYKYIGRSGLKVSAMTMGTMTFGGSERVGHTDLAGAARQVDICLDAGI NLLDTANVYNAGVSEDILGVVLAEHGRRQRALIATKVRFRMGNGPNEGGL SRHHIIAQAEASLKRLRTDAIELYQVHEWDGMTPIDETMEALDTLVKQAKV RYVACSNYSGWHIMKALAGADKRQGQRFVSQQIHYSLHSRDGEYELVPI SQDQGLGILIWSPLAGGLLSGKYRRDGGPESGRHVGGFREPPVYDWDK LYEIIDVIVDIARQRGVSGAQVALAWALERPGVTSLIIGGRSEAQFADNIAAI HLKLSDEERARLDAASRPPLLYPYWHQSFTGQDRLSQIDLDLIEPYAEEFK RG |
| 94 | SEQ ID NO. 94 | MDYKYLGRSGLKVSAMTMGTMTFGGSERVGHTGQAGATRQVDICLDAG INLLDTANVYNAGVSEEMLGVVLAEHGRRQRALIATKVRFKMGNGPNEV GLSRHHIIAQAEASLKRLRTDVIELYQVHEWDGMTPIEDTMEALDTLVRQA KVRYVGCSNYSGWHIMKGLAGADKRQGQRFISQQIHYTLHSRDAEYELV PISQDQGLGILIWSPLAGGLLSGKYRRDGGPDAGRHVGGFREPPVYDWD KLYELIDVIVDIARQRGVSGAQVALSWALERPGVTSLIIGGRSEAQFADNIA GAHLKLSSEERARLDAASRPPLLYPYWHQSFTGQDRLSQIDLDLIGPYAE EFKRG |
| 95 | SEQ ID NO. 95 | MDYKYLGRSGLKVSAMTMGTMTFGGSERVGHTDLAGAARQVDLCLDAG INLYDTANVYNAGVSEDMLGVVLAENGRRQRALVATKVRFKMGNGPNEA GLSRHHIIAQAEASLKRLRTDAIDLYQVHEWDGMTPIDETMEALDTLVRQG KVRYVGCSNYSGWHIMKALMAADKRQGQRFVSQQIHYSLHSRDAEYELV PISQDQGLGILVWSPLAGGLLSGKYRRTGGPESGRHVGGFREPPVYDW DKLYDLIDVIVAIARQRGVSGAQVALAWLLERPGVTSLIIGGRSEAQFRDNI AAIHLKLSSEERARLDAASRPPLLYPYWHNSFTGQDRLDQIDLDLIEPYAE EFKRG |
| 96 | SEQ ID NOo. 96 | MDYKYLGRSGLKVSAMTMGTMTFGGSERVGHTAQAGAARQVDLCLDH GINLLDTANVYNAGVSEEMLGVVLAEHGRRQKALVATKVRFKMGNGPNE GGLSRHHIIAQAEASLKRLKTDAIELYQVHEWDGMTPIEDTMEALDTLVRQ GKVRYVACSNYSGWHIMKALAAADKRQGQRFVSQQIHYSLHSRDAEYEL VPISQDQGLGILVWSPLAGGLLSGKYRRDGGPESGRHVGGFREPPVYD WDKLYEILDVIVAIARQRGVSGAQVALAWLLERPGVTSLIIGGRSEAQFAD NIAAIHLKLSDEERARLDAASRPPLLYPYWHQSFTANDRLDQIDLDLIEPYA EDFKRG |
| 97 | SEQ ID NO. 97 | MDYRYLGRSGLKVSAMTMGTMTFGGSERVGHTDLAGATRQVDLCLDHG INLLDTANVYNAGVSEEMIGVVLSEHGRRQRALIATKVRFKMGNGPNEGG LSRHHIIAQAEASLKRLRTDAIDLYQVHEWDGMTPIDETMEALDTLVRQGK VRYVACSNYSGWHIMKALMGADKRQGQRFVSQQIHYTLHSRDGEYELV PISQDQGLGILVWSPLAGGLLSGKYRRDGGPDAGRHVGGFREPPVYDW DKLYDLLDVIVDIARQRGVSGAQVALSWLLGRPGVTSLIIGGRSEAQFRDN IAAAHLKLSSDERARLDAASRPPLLYPYWHQSFTAQDRLDQIDLDLLEPYA EEFKRG |
| 98 | SEQ ID NO. 98 | MDYRYIGRSGLKVSAMTMGTMTFGGSEKVGHTGQAGAARQVDLCLDHG INLLDTANVYNAGVSEDILGVVLAEHGRRQKALIATKVRFRMGNGPNEVG LSRHHIIAQAEASLKRLRTDAIDLYQVHEWDGMTPIEDTMEALDTLVRQGK VRYVACSNYSGWHIMKALAGADKHHGQRFISQQIHYTLHSREAEYELVPI SQDQGLGILIWSPLAGGLLSGKYRRDGGPESGRHVGGFREPPVYDWDK LYDLIDVIVDIARQRGVSGAQVALSWLLERPGVTSLIIGGRSEAQFRDNIAA IHLKLTSEERARLDAASRPPLLYPYWHNSFTAQDRLSQIDLDLIGPYAEDF KRG |
| 99 | SEQ ID NO. 99 | MDYRYLGRSGLKVSAMTMGTMTFGGSERVGHTGQAGAARQLDLCLDA GINLLDTANVYNAGVSEDILGVVLSEHGRRQRALVATKVRFKMGNGPNE GGLSRHHIIAQAEASLKRLRTDVIELYQVHEWDGMTPIEDTMEALDTLVKQ AKVRYVACSNYSGWHIMKALMGADKHHGQRFVSQQIHYSLHSRDAEYEL VPISQDQGLGILIWSPLAGGLLSGKYRRDGGPDAGRHVGGFREPPVYDW DKLYELIDVIVAIARQRGVSGAQVALAWLLERPGVTSLIIGGRSEAQFRDNL AAAHLKLTDEERARLDAASRPPLLYPYWHQSFTAQDRLSQIDLDLIEPYAE DFKRG |
| 10 0 | SEQ ID NO. 100 | MDYRYIGRSGLKVSAMTMGTMTFGGSERVGHTDLAGATRQVDLCLDAGI NLLDTANVYNAGVSEEMIGVVLAEHGRRQRALVATKVRFRMGNGPNEVG LSRHHIIAQAEASLKRLRTDAIELYQVHEWDGMTPIEETMEALDTLVRQAK VRYVGCSNYSGWHIMKALMGADKRQGQRFVSQQIHYSLHSRDGEYELV PISQDQGLGILVWSPLAGGLLSGKYRRDGGPDAGRHVGGFREPPVYDW DKLYDLIDVIVDIARQRGVSGAQVALAWALERPGVTSLIIGGRSEAQFADN LAAIHLKLTSEERARLDAASRPPLLYPYWHQSFTGNDRLDQIDLDLIGPYA DEFKRG |
| 10 1 | SEQ ID NO. 101 | MDYRYIGRSGLKVSAMTMGTMTFGGSERVGHTAQAGAARQVDLCLDAG INLYDTANVYNAGVSEDILGVVLSENGRRQKALVATKVRFKMGNGPNEAG LSRHHIIAQAEASLKRLKTDAIDLYQVHEWDGMTPIEETMEALDTLVRQGK VRYVGCSNYSGWHIMKALMAADKRQGQRFVSQQIHYSLHSRDAEYELVP ISQDQGLGILVWSPLAGGLLSGKYRRTGGPESGRHVGGFREPPVYDWD KLYELIDVIVDIAAQRGVSGAQVALAWLLGRPGVTSLIIGGRNEAQFRDNIA AIHLKLSSEERARLDAASRPPLLYPYWHQSFTGQDRLDQIDLDLIGPYADE FKRG |

| | | |
|---|---|---|
| Table 2: Depicting sequences as described herein. For some example sequences, positions of loop A and loop B are described directly in the table. Sequences 63-101 comprise amino acid substitutions in accordance with Table 1 depicted herein. | | |

### EXAMPLES OF THE INVENTION

### Example 1: Cloning, expression and enzyme purification

The inventors have discovered different candidate genes that could encode for enzymes having the capacity to reduce 3-oxo trichothecenes such as keto-DON into a trichothecene comprising a 3-hydroxy group such as epi-DON. For expression and protein purification of all candidate genes, nucleotide sequences encoding for the polypeptides of enzymes SEQ ID NO. 1-14 (namely SEQ ID NO. 47-60) as well as sequences encoding the prior art enzymes of SEQ ID NO. 15 and SEQ ID NO. 16, as well as sequences encoding variants comprising amino acid substitutions in accordance with Table 1 disclosed herein of the enzymes of either SEQ ID NO. 1 (SEQ ID NO. 63-75), SEQ ID NO. 3 (SEQ ID NO. 76-88) and SEQ ID NO. 6 (SEQ ID NO. 89-101) were integrated into plasmids for expression in a suitable host organism, such as *E. coli.* The recombinant vectors containing the respective coding sequences were assembled by TWIST bioscience.com (US) into *E. coli* expression vector pET28a with the nucleotide sequences containing the restriction site *Ncol* at the 5'end and the restriction site *Xhol* at the 3'end, as well as a 6xHis tag either on the C-terminal or the N-terminal end. The sequence identity of sequences SEQ ID NO. 1-16, 63-101 are shown in Figure 1A and Figure 1B as well as the sequence table herein.

For expression, recombinant pET28a-based vectors were transferred into E. *coli,* or any other suitable host organism. Protein expression was induced with addition of IPTG. Cells were harvested by centrifugation and then cells were broken open by ultrasound, French Press or any other suitable method to open up bacterial cells and produce crude cell lysates. The lysates were cleared and soluble recombinant protein was used to determine the catalytic properties of the produced reductases. To determine the specific properties of the reductases, the enzymes were selectively purified via nickel based affinity chromatography employing Nickel-sepharose columns. Unfortunately, the amounts of SEQ ID NO. 16 that were produced were too little to perform purity analysis and enzymatic assays. To determine the purity of the recombinant enzymes they were separated on an SDS PAGE protein gel to visualize the proteins with standard Coomassie Blue Staining. The concentrations of the different proteins were determined photometrically in a plated reader (Biotek, Synergy HT) with a wave length of 595 nm using the Bradford reagent (Sigma # B6916) and/or with fluorimetric measurements using the Qubit protein assay (Thermofischer Life technologies #Q33211). For the Bradford assay, a standard curve that measured Bovine Serum Albumin (BSA, Sigma #A4919) in concentrations from 0 µg ml-1 to maximum 1500 µg ml-1, built the base for protein concentration measurements. For the Qubit analysis, the standard curve was established by measuring the provided standards from 0 µg ml-1 to 400 µg ml -1 and subsequently used to determine protein concentration of the samples

### Example 2: In vitro transformation of 3-keto DON

The ability of the reductases produced in Example 1 to use 3-keto DON as a substrate was determined, and reductase enzymes with SEQ ID NO. 15 and 16 were used as examples from the prior art. All enzymes were produced according to Example 1 and were employed in the activity assay described below.

The following enzyme assay shall be defined as the reference method to determine activities, especially specific activities of enzymes claimed according to the present invention. The activity of 3-keto DON reducing enzymes was tested in a standard 3-keto DON degradation assay that is made up of: 100 mM Tris buffer pH 8.0 containing 0.5 mM NADPH, 0.5 mM NADH, 25 ppm 3-keto DON, and 100 nM of the respective enzyme. All components of the reaction except of NADH and NADPH were mixed on ice and a 0 minutes sample was removed (mix sample 1:1 (v/v) with 100% methanol to stop any reaction). Immediately thereafter, reaction mixtures were transferred to the heating block and the reaction was started by adding the cofactor mix (i.e. the NADH and NADPH) and allowed to proceed for 2 hours at 30°C. Samples were removed in regular intervals in order to monitor the progression of the reaction and immediately mixed 1:1 (v/v) with 100% methanol to stop the reaction. They were subsequently analyzed using the HPLC-MS/MS method described in below.

For HPLC analysis of DON, 3-keto DON and 3-epi DON a 150 mm x 4.6 mm Phenomenex Gemini-NX C18 separation column with a particle size of 5 µm was used. A mixture of acetonitrile and ultra-pure water was used a mobile phase and the UV-signal at 220 nm was recorded and evaluated in order to determine the concentrations of the analytes. For LC-MS/MS analysis, DON, 3-keto DON and 3-epi DON were separated on a 150 mm x 2.1 mm Phenomenex Kinetex Biphenyl column with a particle size of 2.6 µm. The mobile phase consisted of a mixture of methanol and ultrapure water with 0.1 % (v/v) acetic acid. Ions were generated using electro spray ionization (ESI) in negative ionization mode. The quantification is done using a QTrap and/or triple quadrupole mass detector. Samples need to be diluted to fall into the linear range of this method which ranges from analyte concentrations of 1 ppb to 500 ppb in the injected sample. The limit of quantification (LOQ) is the limit of reliable detection and concentrations below 1 ppb of compound detected in a sample are not considered as reliable, resulting in a LOQ of 1 ppb. In case the concentration of the analyte is below the LOQ, all further calculations (e.g. for determination of the specific activity or ratio of analytes) are made with 1 ppb.

Prior to HPLC-MS/MS analysis all samples were diluted with 40% methanol in water to a theoretical final concentration of 200 ppb 3-keto DON in the sample.

The results were used to calculate the specific activity (µmol_{product}/min/mg_{enzyme}) of the enzymes based on the 3-epi-DON (or DON) production in the linear range of the reaction in µmol_{product}/min/mg_{enzyme}. This was done by converting the absolute amount of 3-epi-DON (or DON) produced in the reaction up until the selected point in time (i.e. 10 minutes for 3-epi-DON and 120 minutes for DON) from ppm into µmol (i.e. the concentrations of 3-epi-DON in ppm (=µg/ml) were multiplied with the reaction volume in ml and divided by the molecular weight of 3-epi-DON in µg/µmol yielding µmol 3-epi-DON in the reaction at the selected time). The result was then divided by the selected time frame in min. This value was further divided by the total amount of enzyme in the reaction in mg to calculate the specific activity (µmol_{product}/min/mg_{enzyme}), which corresponds to Units per mg of enzyme (U/mg). The results of the activity assays are shown in Figure 2 for the conversion of 3-keto DON to 3-epi-DON and in Figure 3 for the conversion of 3-keto DON to DON.

### Example 3: Sequence analysis for conserved domains and unique structural features

Sequences SEQ ID NO. 1 - 14, and 63-101, and prior art-enzymes (SEQ ID NO. 15 and 16) belong to oxidoreductases and are often predicted as members as aldo-keto reductases, a superfamily of oxidoreductases that depends on NAD(P)(H) to produce primary and secondary alcohols via catalyzing the reduction of aldehydes and ketones, respectively. Aldo-keto reductase share a common (α/β)8 structure, a conserved catalytic mechanism with a catalytic tetrad made up of residues Tyrosine (Tyr, Y), Aspartate (Asp, D), Histidine (His, H) and Lysine (Lys, K) . In the SEQ ID NO. 15 and 16, both known from the prior art, the catalytic tetrade consists of Asp48, Tyr 53, Lys81, and His122. In reductase SEQ ID NO. 1 the catalytic tetrade locates to Asp53, Tyr58, Lys85, and His126. However, they differ significantly at least in the loops A and B that are displayed at the back of the barrel structure. These loops determine substrate specificity together with variations in the substrate binding pocket (Jez and Penning (1998).

The superfamily is characterized by the feature cd06660 in the reference database of the "National Center for Biotechnology Information" (NCBI; http://www.ncbi.nlm.nih.gov/). To further analyze domains of the sequences, a batch search was performed on the CDD conserved domain database" (NCBI; https://www.ncbi.nlm.nih.gov/cdd/) with the specific application for multiple sequences https://www.ncbi.nlm.nih.gov/Structure/bwrpsb/bwrpsb.cgi. The search was performed with standard parameters: automatic search mode against database CDD-52910 PSSMs, an expected threshold level of 0.01 and application of composition-corrected scoring. PSSM = A position-specific scoring matrix (PSSM) is calculated for the extent of the consensus sequence. The PSSM profiles describe how frequently certain residues are detected in a given position of the multiple sequence alignment for a domain model. All analyzed sequences belong to the superfamily cl30127 (Marchler-Bauer et al. (2011), Marchler-Bauer and Bryant (2004),, Marchler-Bauer et al. (2017).

Aldo-keto reductases differ in the loop structures that define substrate specificity. Loops A and B are thus defined as the substrate specificity loops A and B. The substrate specificity loops A and B in enzymes SEQ ID NO. 1-14 and 63-101 are significantly distinct from loops A and B of prior art enzymes, especially SEQ ID NO. 15 and 16. Figure 4. In all cases, loop A consists of 9 residues and contains the histidine of the catalytic tetrade, but instead of the nonpolar alanine (position 123 of prior art enzymes) located downstream to histidine 122 of prior art enzymes, in SEQ ID NO. 1-14 and 63-101, the respective residue directly downstream the catalytic histidine is not an alanine. Substrate specificity Loop A, encompasses the sequence downstream of the common motif I-D/E-LYQ and upstream of the motif I/M/L-E/D-E/D-T-L/M. Substrate specificity Loop B starts with the Serine in the common motif WSPL-X-G and ends upstream, of the common motif X-X-R/K/H-T/L-W/Y-X-I/V/L-I/V/L. Loop B significantly differs in length, with 35 or less residues

The variability in the loops A and B in terms of residues and lengths contributes to an almost 100% stereo selectivity towards 3-epi-DON and the increased specific activity of SEQ ID NO. 1-14 and 63-101, compared to SEQ ID NO. 15-16.

Figure 5. Substrate specificity loops A and B. Start residues, end residues and amino acid length. These are also summarized in the sequence table herein.

### Example 4: Determination of pH optimum

To determine the catalytic pH optimum of a polypeptide as referred to herein, reactions for the transformation of 3-keto-DON at different pH values were prepared. To this end, 16 reactions of different pH values were prepared to a final volume of 200 µL each. A polypeptide having the amino acid sequence of SEQ ID NO: 1 was used at a final concentration of 200 nM. As substrate, 3-keto-DON was used at a final concentration of 30 ppm, and NADPH was used at a final concentration of 1 mM. As buffer, Teorell Stenhagen (TS) buffer was used (Stenhagen & Teorell. 1938. Nature 141, 415) and set to a pH of either 2.5, 3.0, 3.5, 4.0, 4.5, 5.0, 5.5, 6.0, 6.5, 7.0, 7.5, 8.0, 8.5, 9.0, 9.5, 10.0. The transformation reactions were incubated at 30 °C for 120 min, and started by the addition of the NADPH. Throughout the incubation, 20 µL samples were drawn at 0 min, 10 min, 20 min, 30 min, 60 min and 120 min. The sample at 0 min was drawn prior to the addition of the NADPH. The 20 µL samples were immediately mixed with 20 µL of pure methanol to stop the transformation reaction, and then kept on ice until the end of the incubation.

Prior to analysis by LC-MS/MS, all samples were diluted with 40% methanol in water to a theoretical final concentration of 0.3 ppm 3-keto-DON. For LC-MS/MS analysis, DON, 3-keto-DON and 3-epi DON were separated on a 150 mm x 2.1 mm Phenomenex Kinetex Biphenyl column with a particle size of 2.6 µm. The mobile phase consisted of a mixture of methanol and ultrapure water with 0.1 % (v/v) acetic acid. Ions were generated using electro spray ionization (ESI) in negative ionization mode. The quantification was done using a QTrap and/or triple quadrupole mass detector. If necessary, samples were diluted to fall into the linear range of this method which ranges from analyte concentrations of 1 ppb to 500 ppb in the injected sample. The limit of quantification (LOQ) was the limit of reliable detection, and concentrations below 1 ppb of compound detected in a sample were not considered as reliable, resulting in a LOQ of 1 ppb. In case the concentration of the analyte was below the LOQ, all further calculations (e.g. for determination of the specific activity or ratio of analytes) were made with 1 ppb. Kinetic parameters were calculated based on the determined amounts of 3-epi-DON and/or DON from the individual sample time points. Specific activities were calculated from the max. linear product formation rate of each reaction. The specific activities for the formation of 3-epi-DON at different pH values are shown in Table 3 below.

**Table 3: Specific activities of a polypeptide having the amino acid sequence of SEQ ID NO: 1 at different pH values, in µmol of 3-epi-DON formed per min and per mg of polypeptide.**

| **pH** | **µmol/min/mg** |
|---|---|
| 2.5 | 0.000 |
| 3.0 | 0.000 |
| 3.5 | 0.000 |
| 4.0 | 0.007 |
| 4.5 | 0.020 |
| 5.0 | 0.082 |
| 5.5 | 0.133 |
| 6.0 | 0.201 |
| 6.5 | 0.243 |
| 7.0 | 0.199 |
| 7.5 | 0.181 |
| 8.0 | 0.154 |
| 8.5 | 0.197 |
| 9.0 | 0.137 |
| 9.5 | 0.127 |
| 10.0 | 0.114 |

The polypeptide was found active in a pH range from pH 4.5 to pH 10, with an optimum of 3-epi-DON formation at pH 6.5.

### Example 5: Determination of temperature optimum

To determine the catalytic temperature optimum of a polypeptide as referred to herein, reactions for the transformation of 3-keto-DON at different temperatures were prepared. To this end, 24 reactions were prepared to a final volume of 180 µL each. A polypeptide having the amino acid sequence of SEQ ID NO: 1 was used at a final concentration of 200 nM. As substrate, 3-keto-DON was used at a final concentration of 30 ppm, and NADPH was used at a final concentration of 1 mM. As buffer, TS buffer was used at pH 7.0. The transformation reactions were incubated in a laboratory PCR-thermocycler at different temperatures for 120 min, and started by the addition of the NADPH. Throughout the incubation, 20 µL samples were drawn at 0 min, 5 min, 10 min, 20 min, 30 min, 60 min and 120 min. The sample at 0 min was drawn from the reactions prior to the addition of the NADPH. The 20 µL samples were immediately mixed with 180 µL of pure methanol to stop the transformation reaction, and then kept on ice until the end of the incubation. Analyses were performed by LC-MS/MS as described in Example 4. The specific activities for the formation of 3-epi-DON at different temperatures are shown in Table 4 below.

**Table 4: Specific activities of a polypeptide having the amino acid sequence of SEQ ID NO: 1 at different temperatures, in µmol of 3-epi-DON formed per min and per mg of polypeptide.**

| **Temperature °C** | **µmol/min/mg** |
|---|---|
| 14.9 | 0.106 |
| 15.0 | 0.094 |
| 16.0 | 0.089 |
| 17.8 | 0.109 |
| 20.1 | 0.116 |
| 22.7 | 0.114 |
| 25.5 | 0.116 |
| 28.3 | 0.127 |
| 31.0 | 0.118 |
| 33.3 | 0.100 |
| 35.0 | 0.098 |
| 36.0 | 0.088 |
| 34.9 | 0.105 |
| 35.2 | 0.102 |
| 36.3 | 0.085 |
| 38.0 | 0.092 |
| 40.3 | 0.076 |
| 42.9 | 0.059 |
| 45.7 | 0.040 |
| 48.4 | 0.031 |
| 51.0 | 0.017 |
| 53.2 | 0.015 |
| 54.8 | 0.018 |
| 55.7 | 0.019 |

Most 3-epi-DON was formed by the polypeptide at temperatures from 14.9 °C to 36 °C, with an optimum between 25 °C and 31 °C.

### LIST OF REFERENCES

Altschul, J. Mol. Evol. 36 (1993), 290-300
Altschul, J. Mol. Biol. 215 (1990), 403-410
Altschul et al., 1997 (Nucleic Acids Res. (1997) 25:3389-3402
Carere et al. 2018 (Carere (2018) "The identification of DepB: An enzyme responsible for the final detroxification Step in the deoxynivalenol epimerization pathway in Devosia mutans 17-2-E-8." Frontiers in Microbiology. 9:1573
Cregg, Pichia Protocols, second Edition, ISBN-10: 1588294293, 2007
Ellis (2002) "Microbial aldo-keto reductases" FEMS Microbiol Lett. 5;216(2):123-31
Hassan et al. (2017) "The enzymatic epimerization of deoxynivalenol by Devosia mutans proceeds through the formation of 3-keto-DON intermediate." Scientific Reports 7, article number 6929
He et al. (2015) "Toxicology of 3-epi-deoxynivalenol, a deoxynivalenol-transformation product by Devosia mutans 17-2-E-8." Food and Chemical Toxicology 84: 250-259
Henikoff Proc. Natl. Acad. Sci., USA, 89, (1989), 10915
Henikoff and Henikoff (1992) 'Amino acid substitution matrices from protein blocks.' Proc Natl Acad Sci U S A. 1992 Nov 15;89(22):10915-9
Jez and Penning (1998) "Engineering steroid 5 beta-reductase activity into rat liver 3 alpha-hydroxysteroid dehydrogenase" Biochemistry.37(27):9695-703
Kundert K, Kortemme T. (2019) "Computational design of structured loops for new protein functions." Biol Chem. 2019 Feb 25;400(3):275-288
Kurtzman (2009) "Biotechnological strains of Komagataella (Pichia) pastoris are Komagataella phaffii as determined from multigene sequence analysis." J Ind Microbiol Biotechnol. 36(11):1435-8
Marchler-Bauer et al. (2011) "CDD: a Conserved Domain Database for the functional annotation of proteins." Nucleic Acids Res.39(D)225-9
Marchler-Bauer and Bryant (2004), "CD-Search: protein domain annotations on the fly.", Nucleic Acids Res.32(W)327-331
Marchler-Bauer et al. (2017), "CDD/SPARCLE: functional classification of proteins via subfamily domain architectures.", Nucleic Acids Res.45(D)200-3
Needleman and Wunsch, 1970 (Journal of Molecular Biology. 1970 Mar;48:443-453
Payros et al., (2016) "Toxicology of deoxynivalenol and its acetylated and modified forms." Archives of Toxicology 90(12): 2931-2957)
Penning (2015) "The aldo-keto reductases (AKRs): Overview" Chem Biol Interact. 234:236-46
Pierron et al., (2016) "Microbial biotransformation of DON: molecular basis for reduced toxicity." Scientific Reports 6(1))
POST-TRANSLATIONAL COVALENT MODIFICATION OF PROTEINS, B. C. Johnson, Ed., Academic Press, New York (1983), pgs. 1-12
PROTEINS - STRUCTURE AND MOLECULAR PROPERTIES, 2nd Ed., T. E. Creighton, W. H. Freeman and Company, New York (1993)
Rattan, Ann. NY Acad. Sci. 663 (1992); 48-62
Sambrook et al. 2012, Molecular Cloning, A Laboratory Manual 4th Edition, Cold Spring Harbor
Schatzmayr and Streit (2013) 'Global occurrence of mycotoxins in the food and feed chain: Facts and figures.' World Mycotoxin Journal 6(3):213-222
Seifter, Meth. Enzymol. 182 (1990); 626-646
Thompson Nucl. Acids Res. 2 (1994), 4673-4680) or FASTDB (Brutlag Comp. App. Biosci. 6 (1990), 237-245
WO2019/046954
Yamada et al. (1995) 'The Phylogenetic Relationships of Methanol-assimilating Yeasts Based on the Partial Sequences of 18S and 26S Ribosomal RNAs: The Proposal of Komagataella Gen. Nov. (Saccharomycetaceae)' Bioscience, Biotechnology and Biochemistry, Vol. 59, issue 3, pp. 439-444
Zdarta et al. (2018) "A general overview of support materials for enzyme immobilization: characteristics, properties, practical utility" Catalysts 8, 92, p. 1-27.
Zhu Y., Hassan Y. I., Lepp D., Shao S., Zhou T. (2017) "Strategies and Methodologies for Developing Microbial Detoxification Systems to Mitigate Mycotoxins." Toxins. 9 (4):130

## Claims

1. An agrarian composition comprising a plant or parts of a plant, said agrarian composition comprising:
a) one or more polypeptide(s) comprising or consisting of a sequence of SEQ ID NO. 1-14 or a sequence having a sequence identity of at least 75 % to any one of SEQ ID NO. 1-14,
wherein the polypeptide is capable of converting the substrate 3-keto-DON into the products 3-epi-DON and DON with a 3-epi-DON: DON ratio of at least 25:1; and
b) a carrier.

2. The composition of any one of the preceding claims, wherein the polypeptide is a reductase.

3. The composition of any one of the preceding claims, wherein the polypeptide, comprises a sequence comprising the sequence X1-H-X2-W-D-X3-X4-T-P, wherein
X1 is not M;
X2 is not A;
X3 is not A;
X4 is not V.

4. The composition of any one of the preceding claims, wherein the polypeptide comprises a sequence comprising the sequence:
(a) S-P-L-X5-G-G-X6-L-X7-G, wherein X5 is A, V, L, G or I, preferably A; X6 is L, I, V, M, A, Y, F, Norleucin or W, preferably L or W; X7 is S or T, preferably S; and/or
(b) X8-G-X9-X10-X11-X12-X13-X14-X15-X16, wherein X8 is G, R, K, Q, N, A, V, L, I, S, T, E, D; X9 is not M or G; X10 is not E; X11 is not S; X12 is not Y; X13 is not G or A; X14 is not P; X15 is R, K, Q, N, P, A, Y, W, F, T, S, H; X16 is not N.

5. The composition of any one of the proceedings claims, wherein one or more polypeptide(s) comprising a sequence having a sequence identity of at least 75 % to any one of SEQ ID NO. 1-14 comprises one or more amino acid substitutions compared to a sequence of any one of SEQ ID NO. 1-14, preferably the one or more amino acid substitutions are conservative amino acid substitutions.

6. The composition of any one of the proceedings claims, wherein the carrier is a liquid, preferably H₂O, or a solid, preferably a nutraceutical and/or a pharmaceutical.

7. Polypeptide comprising a sequence of SEQ ID NO. 1 or a sequence having a sequence identity of at least 92 % to SEQ ID NO. 1, wherein the polypeptide is capable of converting the substrate 3-keto-DON into the products 3-epi-DON and DON with a 3-epi-DON: DON ratio of at least 25:1.

8. Use of one or more polypeptide(s) comprising or consisting of a sequence of SEQ ID NO. 1-14 or a sequence having a sequence identity of at least 75 % to any one of SEQ ID NO. 1-14 for converting 3-keto-DON into 3-epi-DON.

9. Use of one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1, in the manufacture of: (a) a feed additive or feed composition; or (b) biogas, bioethanol, or sugar.

10. Feed additives or food additives or feed or food comprising one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1.

11. Additive for use in compositions, preferably agrarian compositions, comprising 3-keto-DON or DON, the additive comprising one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1.

12. Method of converting a trichothecene comprising a 3-oxo group into a trichothecene comprising a 3-hydroxy group, the method comprising
contacting one or more polypeptide(s) comprising or consisting of a sequence of SEQ ID NO. 1-14 or a sequence having a sequence identity of at least 75 % to any one of SEQ ID NO. 1-14 with one or more trichothecene(s) comprising a 3-oxo group.

13. Method of reducing the content of DON in a composition comprising DON or of reducing the toxicity of a composition comprising DON by converting DON into 3-epi-DON, the method comprising
a) contacting the composition with an enzyme capable of converting DON into 3-keto DON; and
b) subsequently or concurrently contacting the composition with one or more polypeptide(s) comprising or consisting of a sequence of SEQ ID NO. 1-14 or a sequence having a sequence identity of at least 75 % to any one of SEQ ID NO. 1-14.

14. A recombinant host cell comprising one or more polypeptide(s) comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON: DON ratio of at least 25:1, wherein said recombinant host cell is transferred with an expression vector capable of producing said one or more polypeptide(s).

15. A polypeptide comprising or consisting of a sequence of SEQ ID NO. 1-14 or a sequence having a sequence identity of at least 75 % to any one of SEQ ID NO. 1-14, wherein the polypeptide is capable of converting the substrate 3-keto-DON into the products 3-epi-DON and DON with a 3-epi-DON:DON ratio of at least 25:1 for use in the prevention and/or treatment of mycotoxicosis.

## Patentansprüche

1. Eine agrarische Zusammensetzung, die eine Pflanze oder Teile einer Pflanze umfasst, wobei die agrarische Zusammensetzung umfasst:
a) ein oder mehrere Polypeptide, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen,
wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln; und
b) einen Träger.

2. Die Zusammensetzung gemäß einem der vorstehenden Ansprüche, wobei das Polypeptid eine Reduktase ist.

3. Die Zusammensetzung gemäß einem der vorstehenden Ansprüche, wobei das Polypeptid eine Sequenz umfasst, die die Sequenz X1-H-X2-W-D-X3-X4-T-P umfasst, wobei
X1 nicht M ist;
X2 nicht A ist;
X3 nicht A ist;
X4 nicht V ist.

4. Die Zusammensetzung gemäß einem der vorstehenden Ansprüche, wobei das Polypeptid eine Sequenz umfasst, die die folgende Sequenz umfasst:
(a) S-P-L-X5-G-G-X6-L-X7-G, wobei X5 A, V, L, G oder I ist, vorzugsweise A; X6 L, I, V, M, A, Y, F, Norleucin oder W ist, vorzugsweise L oder W; X7 S oder T ist, vorzugsweise S;
und/oder
(b) X8-G-X9-X10-X11-X12-X13-X14-X15-X16, wobei X8 G, R, K, Q, N, A, V, L, I, S, T, E oder D ist; X9 ist nicht M oder G; X10 ist nicht E; X11 ist nicht S; X12 ist nicht Y; X13 ist nicht G oder A; X14 ist nicht P; X15 ist R, K, Q, N, P, A, Y, W, F, T, S oder H; X16 ist nicht N.

5. Die Zusammensetzung gemäß einem der vorstehenden Ansprüche, wobei ein oder mehrere Polypeptide, die eine Sequenz mit einer Sequenzidentität von mindestens 75 % zu einer der SEQ ID NO. 1-14 umfassen, eine oder mehrere Aminosäuresubstitutionen im Vergleich zu einer Sequenz einer der SEQ ID NO. 1-14 umfassen, wobei die eine oder die mehreren Aminosäuresubstitutionen vorzugsweise konservative Aminosäuresubstitutionen sind.

6. Die Zusammensetzung gemäß einem der vorstehenden Ansprüche, wobei der Träger eine Flüssigkeit ist, vorzugsweise H₂O, oder ein Feststoff ist, vorzugsweise ein Nutrazeutikum und/oder ein Pharmazeutikum.

7. Polypeptid, das eine Sequenz gemäß SEQ ID NO. 1 oder eine Sequenz mit einer Sequenzidentität von mindestens 92 % zu SEQ ID NO. 1 umfasst, wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln.

8. Verwendung eines oder mehrerer Polypeptide, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen, zur Umwandlung von 3-Keto-DON in 3-Epi-DON.

9. Verwendung eines oder mehrerer Polypeptide, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen, wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln, bei der Herstellung von: (a) einem Futtermittelzusatzstoff oder einer Futtermittelzusammensetzung; oder (b) Biogas, Bioethanol oder Zucker.

10. Futtermittelzusatzstoffe oder Lebensmittelzusatzstoffe oder Futtermittel oder Lebensmittel, die ein oder mehrere Polypeptide umfassen, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen, wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln.

11. Zusatzstoff zur Verwendung in Zusammensetzungen, vorzugsweise agrarischen Zusammensetzungen, die 3-Keto-DON oder DON umfassen, wobei der Zusatzstoff ein oder mehrere Polypeptide umfasst, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen, wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln.

12. Verfahren zur Umwandlung eines Trichothecens, das eine 3-Oxo-Gruppe umfasst, in ein Trichothecen, das eine 3-Hydroxy-Gruppe umfasst, wobei das Verfahren das Inkontaktbringen eines oder mehrerer Polypeptide umfasst, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen, mit einem oder mehreren Trichothecenen, die eine 3-Oxo-Gruppe umfassen.

13. Verfahren zur Verringerung des DON-Gehalts in einer DON-enthaltenden Zusammensetzung oder zur Verringerung der Toxizität einer DON-enthaltenden Zusammensetzung durch Umwandlung von DON in 3-Epi-DON, wobei das Verfahren umfasst:
a) das Inkontaktbringen der Zusammensetzung mit einem Enzym, das in der Lage ist, DON in 3-Keto-DON umzuwandeln; und
b) das anschließende oder gleichzeitige Inkontaktbringen der Zusammensetzung mit einem oder mehreren Polypeptiden, die eine Sequenz gemäß SEQ ID NO. 1-14 oder eine Sequenz, die eine Sequenzidentität von mindestens 75 % zu einer der SEQ ID NO. 1-14 aufweist, umfassen oder daraus bestehen.

14. Eine rekombinante Wirtszelle, die ein oder mehrere Polypeptide umfasst, die eine Sequenz von SEQ ID NO. 1-14 oder eine Sequenz, die eine Sequenzidentität von mindestens 75 % zu einer der SEQ ID NO. 1-14 aufweist, umfassen oder daraus bestehen, wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln, wobei die rekombinante Wirtszelle mit einem Expressionsvektor transformiert ist, der in der Lage ist, das eine oder die mehreren Polypeptide zu produzieren.

15. Ein Polypeptid, das eine Sequenz von SEQ ID NO. 1-14 oder eine Sequenz, die eine Sequenzidentität von mindestens 75 % zu einer der SEQ ID NO. 1-14 aufweist, umfasst oder daraus besteht, wobei das Polypeptid in der Lage ist, das Substrat 3-Keto-DON in die Produkte 3-Epi-DON und DON mit einem 3-Epi-DON:DON-Verhältnis von mindestens 25:1 umzuwandeln, zur Verwendung bei der Vorbeugung und/oder Behandlung von Mykotoxikose.

## Revendications

1. Composition agraire comprenant une plante ou des parties d'une plante, ladite composition agraire comprenant :
a) un ou plusieurs polypeptides comprenant ou consistant en une séquence de SEQ ID NO. 1 à 14 ou en une séquence présentant une identité de séquence d'au moins 75 % avec l'une quelconque des séquences de SEQ ID NO. 1 à 14,
dans laquelle le polypeptide est capable de convertir le substrat 3-céto-DON en produits 3-épi-DON et DON avec un rapport 3-épi-DON:DON d'au moins 25:1 ; et
b) un support.

2. Composition selon l'une quelconque des revendications précédentes, dans laquelle le polypeptide est une réductase.

3. Composition selon l'une quelconque des revendications précédentes, dans laquelle le polypeptide comprend une séquence comprenant la séquence X1-H-X2-W-D-X3-X4-T-P, dans laquelle
X1 n'est pas M ;
X2 n'est pas A ;
X3 n'est pas A ;
X4 n'est pas V.

4. Composition selon l'une quelconque des revendications précédentes, dans laquelle le polypeptide comprend une séquence comprenant la séquence :
(a) S-P-L-X5-G-G-X6-L-X7-G, dans laquelle X5 est A, V, L, G ou I, de préférence A ; X6 est L, I, V, M, A, Y, F, norleucine ou W, de préférence L ou W ; X7 est S ou T, de préférence S ;
et/ou
(b) X8-G-X9-X10-X11-X12-X13-X14-X15-X16, dans laquelle X8 est G, R, K, Q, N, A, V, L, I, S, T, E, D ; X9 n'est pas M ou G ; X10 n'est pas E ; X11 n'est pas S ; X12 n'est pas Y ; X13 n'est pas G ou A ; X14 n'est pas P ; X15 est R, K, Q, N, P, A, Y, W, F, T, S, H ; X16 n'est pas N.

5. Composition selon l'une quelconque des revendications précédentes, dans laquelle un ou plusieurs polypeptide(s) comprenant une séquence ayant une identité de séquence d'au moins 75 % avec l'une quelconque des SEQ ID NO. 1-14 comprennent une ou plusieurs substitutions d'acides aminés par rapport à une séquence de l'une quelconque des SEQ ID NO. 1-14, de préférence la ou les substitutions d'acides aminés sont des substitutions conservatrices d'acides aminés.

6. Composition selon l'une quelconque des revendications précédentes, dans laquelle le support est un liquide, de préférence H₂O, ou un solide, de préférence un nutraceutique et/ou un produit pharmaceutique.

7. Polypeptide comprenant une séquence de SEQ ID NO. 1 ou une séquence ayant une identité de séquence d'au moins 92 % avec SEQ ID NO. 1, dans laquelle le polypeptide est capable de convertir le substrat 3-céto-DON en produits 3-épi-DON et DON avec un rapport 3-épi-DON:DON d'au moins 25:1.

8. Utilisation d'un ou plusieurs polypeptides comprenant ou consistant en une séquence de SEQ ID NO. 1 à 14 ou en une séquence présentant une identité de séquence d'au moins 75 % avec l'une quelconque des séquences de SEQ ID NO. 1 à 14, pour la conversion du 3-céto-DON en 3-épi-DON.

9. Utilisation d'un ou de plusieurs polypeptide(s) comprenant ou consistant en une séquence de SEQ ID NO. 1-14 ou une séquence ayant une identité de séquence d'au moins 75 % avec l'une quelconque des SEQ ID NO. 1-14, dans laquelle le polypeptide est capable de convertir le substrat 3-céto-DON en produits 3-épi-DON et DON avec un rapport 3-épi-DON:DON d'au moins 25:1, dans la fabrication de : (a) un additif pour aliments pour animaux ou une composition d'aliments pour animaux ; ou (b) du biogaz, du bioéthanol ou du sucre.

10. Additifs pour aliments pour animaux ou additifs alimentaires ou aliments pour animaux ou aliments comprenant un ou plusieurs polypeptide(s) comprenant ou consistant en une séquence de SEQ ID NO. 1-14 ou une séquence ayant une identité de séquence d'au moins 75 % avec l'une quelconque des SEQ ID NO. 1-14, dans laquelle le polypeptide est capable de convertir le substrat 3-céto-DON en produits 3-épi-DON et DON avec un rapport 3-épi-DON:DON d'au moins 25:1.

11. Additif destiné à être utilisé dans des compositions, de préférence des compositions agraires, comprenant du 3-céto-DON ou du DON, ledit additif comprenant un ou plusieurs polypeptide(s) comprenant ou consistant en une séquence de SEQ ID NO. 1-14 ou une séquence ayant une identité de séquence d'au moins 75 % avec l'une quelconque des SEQ ID NO. 1-14, dans laquelle le polypeptide est capable de convertir le substrat 3-céto-DON en produits 3-épi-DON et DON avec un rapport 3-épi-DON:DON d'au moins 25:1.

12. Procédé de conversion d'un trichothécène comprenant un groupe 3-oxo en un trichothécène comprenant un groupe 3-hydroxy, le procédé comprenant la mise en contact d'un ou de plusieurs polypeptide(s) comprenant ou consistant en une séquence de SEQ ID NO. 1-14 ou une séquence ayant une identité de séquence d'au moins 75 % avec l'une quelconque des SEQ ID NO. 1-14, avec un ou plusieurs trichothécènes comprenant un groupe 3-oxo.

13. Procédé permettant de réduire la teneur en DON d'une composition contenant du DON ou de réduire la toxicité d'une composition contenant du DON en convertissant le DON en 3-épi-DON, ledit procédé comprenant:
a) la mise en contact de la composition avec une enzyme capable de convertir le DON en 3-céto-DON; et
b) la mise en contact ultérieure ou simultanée de la composition avec un ou plusieurs polypeptides comprenant ou consistant en une séquence de SEQ ID NO. 1 à 14 ou une séquence présentant une identité de séquence d'au moins 75 % avec l'une quelconque des séquences de SEQ ID NO. 1 à 14.

14. Cellule hôte recombinante comprenant un ou plusieurs polypeptide(s) comprenant ou consistant en une séquence de SEQ ID NO. 1-14 ou une séquence ayant une identité de séquence d'au moins 75 % avec l'une quelconque des SEQ ID NO. 1-14, dans laquelle le polypeptide est capable de convertir le substrat 3-céto-DON en produits 3-épi-DON et DON avec un rapport 3-épi-DON:DON d'au moins 25:1, dans laquelle ladite cellule hôte recombinante est transformée avec un vecteur d'expression capable de produire ledit ou lesdits polypeptide(s).

15. Polypeptide comprenant ou consistant en une séquence de SEQ ID NO. 1-14 ou une séquence ayant une identité de séquence d'au moins 75 % avec l'une quelconque des SEQ ID NO. 1-14, dans laquelle le polypeptide est capable de convertir le substrat 3-céto-DON en produits 3-épi-DON et DON avec un rapport 3-épi-DON:DON d'au moins 25:1 pour utilisation dans la prévention et/ou le traitement de la mycotoxicose.
